# EUROPEAN PATENT SPECIFICATION

(11) **EP 1 189 941 B1**
(45) Date of publication and mention of the grant of the patent: **09.01.2013**
(21) Application number: 00939563.3
(22) Date of filing: 05.06.2000
(51) Int. Cl.: C07K 14/655, C07K 14/47, A61K 38/31

(54) **NEUROMEDIN B AND SOMATOSTATIN RECEPTOR AGONISTS**
NEUROMEDIN B UND SOMATOSTATIN REZEPTOR ANTAGONISTEN
ANTAGONISTES DES RECEPTEURS DE LA SOMATOSTATINE ET DE LA NEUROMEDINE B

(30) Priority: 04.06.1999 US 137655 P
(43) Date of publication of application: 27.03.2002
(73) Proprietor: IPSEN PHARMA, 92100 Boulogne-Billancourt (FR)
(72) Inventor: SADAT-AALAEE, Dean, Shrewsbury, MA 01545 (US); MORGAN, Barry, A., Franklin, MA 02038 (US)
(74) Representative: Taylor, Kate Laura
(86) International application number: PCT/US2000/015396
(87) International publication number: WO 2000/075186

(56) References cited:
- EP-A- 0 127 899
- EP-A- 0 478 101
- WO-A-93/03056
- WO-A-94/05310
- WO-A-98/51332
- WO-A-99/25729
- US-A- 5 462 926
- KUBOTA AKIRA ET AL: "Effector coupling of somatostatin receptor subtypes on human endocrine tumors." METABOLISM CLINICAL AND EXPERIMENTAL, vol. 45, no. 8 SUPPL. 1, 1996, pages 42-45, XP002149617 ISSN: 0026-0495 & DATABASE BIOSIS BIOSCIENCES INFORMATION SERVICE, PHILADELPHIA, PA, US; Database accession no. PREV199699187744
- HORVATH, A. ET AL: "Somatostatin octa- and heptapeptides, structural and biological characteristics" PEPT. 1996, PROC. EUR. PEPT. SYMP., 24TH (1998), MEETING DATE 1996, 483-484. EDITOR(S): RAMAGE, ROBERT;EPTON, ROGER. PUBLISHER: MAYFLOWER SCIENTIFIC, KINGSWINFORD, UK. , XP000918406
- COY, DAVID H. ET AL: "Somatostatin receptor antagonists based on a mixed neuromedin B antagonist /somatostatin agonist" PEPT. PROC. AM. PEPT. SYMP., 15TH (1999), MEETING DATE 1997, 526-529. EDITOR(S): TAM, JAMES P.;KAUMAYA, PRAVIN T. P. PUBLISHER: KLUWER, DORDRECHT, NETH. , XP000917964
- RYAN, RICHARD R. ET AL: "Comparative pharmacology of the nonpeptide neuromedin B receptor antagonist PD 168368" J. PHARMACOL. EXP. THER. (1999), 290(3), 1202-1211 , XP000917708

## Description

### BACKGROUND OF THE INVENTION

The mammalian bombesin (Bn)-related peptides, gastrin-releasing peptide (GRP) and neuromedin B (NMB) have a wide range of biological and pharmacological effects. These include stimulation of the release of numerous gastrointestinal hormones and peptides, stimulation of exocrine gland secretion chemotaxis, contraction of smooth muscle, effects in the central nervous system such as thermoregulation, behavioral effects, maintenance of circadian rhythm, inhibition of TSH release and satiety. Bn-related peptides also function as a growth factor in numerous normal cells (e.g., bronchial cells, endometrial stomal cells and 3T3 cells) as well as neoplastic cells such as human small cell lung cancer cells, rat hepatocellular tumor cells, prostatic cells and breast adenocarcinoma cells.

Recent structure-function and cloning studies demonstrate that at least two classes of receptors mediate the actions of Bn-related peptides. One class, the GRP-preferring subtype (GRP receptor or GRP-R), has a high affinity for GRP and low affinity for NMB, whereas the other class, the NMB-preferring subtype (NMB receptor or NMB-R), has a high affinity for NMB and lower affinity for GRP. Both classes of receptors are widely present both in the central nervous system and in the gastrointestinal tract. Until recently, the physiological importance of Bn-related peptides in mediating various processes or which receptor subtype mediated the various reported biological effects of Bn-related peptides was unclear.

Five different classes of Bn-receptor antagonists have been described. Jensen, R. T. et al. Trends Pharmacol. Sci. 12:13 (1991). Members of a number of these classes have high potency, long duration of action and selectivity for the GRP receptor and thus are useful even *in vivo* for defining the role of GRP or GRP receptors in mediating various physiological events. However, at present few antagonists for the NMB receptor which are sufficiently selective or potent have been described. (See, e.g., Coy, D., and Taylor, J., U.S. Patent 5,462,926.) Further, NMB has been implicated in the inhibition of lung cancer and gliomas, Cancer Res 1991 Oct 1 51:19 5205-11; J Cell Biochem Suppl 1996 24: 237-46, Peptides 1995 16:6 1133-40; J Pharmacol Exp Ther 1992 Oct 263:1 311-7), stimulation of appetite, (Eur J Pharmacol 1994 Dec 12 271:1 R7-9; Am J Physiol 1997 Jan 272:1 Pt 2 R433-7; Pharmacol Biochem Behav 1996 Aug 54:4 705-11), stimulation of TSH secretion, (hypothyroidism), (Regul Pept 1996 Nov 14 67:1 47-53), and inhibition of aldosterone secretion, (hyperaldosteronism), (Histol Histopathol 1996 Oct 11:4 895-7). Thus, the compounds of the present invention are useful in the investigation of the physiological role played by NMB, and in the development of therapeutic compositions for treatment of NMB-related indications.

As is known in the art, agonists and antagonists of somatostatin are useful for treating a variety of medical conditions and diseases, such as inhibition of H. pylori proliferation, acromegaly, restenosis, Crohn's disease, systemic sclerosis, external and internal pancreatic pseudocysts and ascites, VIPoma, nesidoblastosis, hyperinsulinism, gastrinoma, Zollinger-Ellison Syndrome, diarrhea, AIDS related diarrhea, chemotherapy related diarrhea, scleroderma, Irritable Bowel Syndrome, pancreatitis, small bowel obstruction, gastroesophageal reflux, duodenogastric reflux and in treating endocrinological diseases and/or conditions, such as Cushing's Syndrome, gonadotropinoma, hyperparathyroidism, Graves' Disease, diabetic neuropathy, Paget's disease, and polycystic ovary disease; in treating various types of cancer such as thyroid cancer, hepatome, leukemia, meningioma and conditions associated with cancer such as cancer cachexia; in the treatment of such conditions as hypotension such as orthostatic hypotension and postprandial hypotension and panic attacks; GH secreting adenomas (Acromegaly) and TSH secreting adenomas. Activation of type 2 but not type 5 subtype receptor has been associated with treating prolactin secreting adenomas. Other indications associated with activation of the somatostatin subtypes are inhibition of insulin and/or glucagon and more particularly diabetes mellitus, hyperlipidemia, insulin insensitivity, Syndrome X, angiopathy, proliferative retinopathy, dawn phenomenon and Nephropathy; inhibition of gastric acid secretion and more particularly peptic ulcers, enterocutaneous and pancreaticocutaneous fistula, Dumping syndrome, watery diarrhea syndrome, acute or chronic pancreatitis and gastrointestinal hormone secreting tumors; inhibition of angiogenesis, treatment of inflammatory disorders such as arthritis; chronic allograft rejection; angioplasty; preventing graft vessel and gastrointestinal bleeding. Somatostatin agonists can also be used for decreasing body weight in a patient. Accordingly, the compounds of the instant invention are useful for the foregoing methods.

Recently, it was reported that a native somatostatin (SS), somatostatin-14 (SS-14), inhibited the cross-linking of ¹²⁵I-GRP to a 120 kD protein in triton extracts of 3T3 cells and human small cell lung cancer cells which are known to possess bombesin receptors. Recent studies have also demonstrated SS-14 could also weakly inhibit binding to opiate receptors, and subsequent structure-function led to the identification of various D-amino acid-substituted and constrained amino acid-substituted cyclo somatostatin analogs that functioned as potent mu opioid receptor antagonists.

WO94/05310 is directed to peptides which inhibit binding of neutrophils, monocytes, subsets of lymphocytes or other cells P-sectin, which inhibit leukocyte adhesion to endothelium that is mediated by E-selection or L-selection or that is mediated by ICAM-1. In particular, this reference discloses 10- or 9-membered peptides having an internal cyclic structure formed by a disulfide bridge between two Cys residues.

WO93/03056 is directed to a process producing a peptide having a lanthionine bridge. A lanthionine bridge is a monosulfide linkage between two small amino acid residues.

EP 0 478 101 discloses 5-membered cyclic analogues of thrombospondin capable of potentiating or inhibiting thrombospondin-like activity.

WO99/25729 is directed to pacitaxel derivatives that have been modified to include a highly specific compound having the sequence Phe-Cys-Tyr-Trp-A₁-Cys wherein A, is either a lysine, citruline, arginine or ornithine residue bound to the pacitaxel via a linker.

WO98/51332 is directed to a method of treating insulin resistance by administering somatostatin or a somatostatin agonist.

EP 0 127 899 discloses cyclic pentapeptides formed from a highly specific amide bond between Pro and Thr residues.

Kubota et al., "Effector coupling of somatostatin receptor subtypes of human endocrine tumors", Metabolism, 45:42-45, 1996, discloses experiments which investigated the effector coupling of somatostatin receptor subtypes SSTR1 and SSTR2 in CHO cells transformed to express the SST receptors. Kubota employed native somatostatin as well as two somatostatin analogs to determine that SSTR1 and SSTR2 are coupled to adenylyl cyclase via different G proteins and that different somatostatin molecules and analogs inhibited intracellular cAMP levels differently via the two receptors.

Horvath, et al., "Somatostatin octa- and heptapeptides, structural and biological characteristics", Peptides, p483-484, 1996 discloses variations of somatostatin analog MI-1761 which were prepared to study the role of exocyclic β-Asp(Ind) and Thr upon anti-tumor activity and the role of endocyclic Tyr and Lys in selectivity of the modified molecules. The research showed that growth hormone inhibitory activity and selective antitumor activity of the somatostatin analogs can be separated by different conformational requirements.

Coy, et al., "Somatostatin receptor antagonists based on a mixed neuromedin B antagonisUsomatostatin sgonist," Pept. Proc. Am. Pept. Symp., p526-529, 1999 discloses the effects of various somatostatin antagonists upon the inhibition of GH release. Of particular interest were substitutions of optimized aromatic amino acids structures at positions 1 and 8 of the peptides.

US 5462926 describes a method of selectively inhibiting the biochemical activity of cells induced by neuromedin B. The method includes the step of contacting the cells of interest expressing neuromedin B receptors with a cyclic octapeptide.

### SUMMARY OF THE INVENTION

The present invention relates to a series of analogues having unique structural features, and to a method of selectively modulating biochemical activity of cells induced by somatostatin and/or neuromedin B.

In one aspect the present invention is directed to a compound of the formula (I),

(R¹R²)-AA¹-AA²-AA³-AA^{3b}-AA⁴-AA⁵-AA⁶-AA⁷-AA^{7b}-AA⁸-R⁵ (I)

or a pharmaceutically acceptable salt thereof,
wherein
the α-nitrogen of AA¹, AA², AA³, AA^{3b}, AA⁴, AA⁵, AA⁶, AA⁷, AA^{7b}, and AA⁸ each is, independently, optionally substituted with (C₁₋₄)alkyl, (C₃₋₄)alkenyl, (C₃₋₄)alkynyl, or (C₁₋₆)alkyl-C(O)-:
AA¹ is absentor the D- or L-isomer of an amino acid selected from the group consisting of R¹¹, Aac, Aic, Arg, Asn, Asp, Dip, Gln, Glu, Hca, Hyp, Lys, Mac, Macab, Orn, Pro, Ser, Ser(Bzl), Thr, Thr(Bzl), Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, C4c, 5-Iqs, Htqa, 4-Mqc, Thn, α-Chpa, Cit, Nua, Pyp and an optionally substituted aromatic α-amino acid;
wherein said optionally substituted aromatic α-amino acid is optionally substituted with one or more substituents each independently selected from the group consisting of halogen, NO₂, OH, CN, (C₁₋₆)alkyl, (C₂₋₆)alkenyl, (C₂₋₆)alkynyl, (C₁-₆)alkoxy, Bzl, O-Bzl, and NR⁹R¹⁰;
AA² is absent or the D- or L-isomer of an amino acid selected from the group consisting of R¹¹, Aic, Arg, Hca, His, Hyp, Pal, F₅-Phe, Phe, Pro, Trp, and X⁰-Phe Pip, hArg, Bip, Bpa, Tic, Cmp, , Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, and Pyp;
AA³ is the D- or L-isomer of an amino acid selected from the group consisting of Cys, hCys, Pen, Tpa, Tmpa, Mac, Macab, and an optionally substituted aromatic α-amino acid;
wherein said optionally substituted aromatic α-amino acid is optionally substituted with one or more substituents selected from the group consisting of halogen, NO₂, OH, CN, (C₁₋₄)alkyl, (C₂₋₄)alkenyl, (C₂₋₄)alkynyl, (C₁₋₄)alkoxy, Bzl, O-Bzl, NR⁹R¹⁰, Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, and Pyp; AA^{3b} is absent or the D- or L-isomer of an amino acid selected from the group consisting of Pal, 4-Pal, His, Arg, Nal, Trp, Bpa, F₅-Phe, Phe, X⁰-Phe, R¹¹, hArg, Bip, Tic, , Htic, Dip, Sala, Aala, Thza, Thia, Bal, Fala, and Pala; AA⁴ is a D- or L-isomer of an optionally substituted amino acid or of an optionally substituted aromatic α-amino acid;
wherein said optionally substituted amino acid is selected from the group consisting of Trp, Lys, Orn, hLys, *cis*-4-Acha, *trans*-4-Acha, *trans*-4-Amcha, 4-Pip-Gly, N-Met-Trp, β-Met-Trp, His, hHis, hArg, Bip, Tic, Htic, Dip, Sala, Aala, Thza, Thia, Bal, Fala, Pala, and 4-Pip-Ala;
wherein the side chain amino group of said optionally substituted amino acid is optionally substituted with R³ and R⁴; and
wherein said optionally substituted aromatic α-amino acid is optionally substituted with one or more substituents each independently selected from the group consisting of halogen, NO₂, OH, CN, (C₁₋₄)alkyl, (C₂₋₄)alkenyl, (C₂₋₄)alkynyl, Bzl, O-Bzl, and NR⁹R¹⁰;
AA⁵ is absent, R¹¹, Aic, A3c, A4c, A5c, A6c, Abu, Aib, ß-Ala, Bpa, Cha, Deg, Gaba, Ile, Leu, Nal, Nle, Pro, Sar, Ser, Ser(Bzl), Thr, Thr(Bzl), Trp, Val, Pal, F₅-Phe, Phe, X⁰-Phe, or an optionally substituted D- or L- isomer of an amino acid selected from the group consisting of 4-Pip-Gly, 4-Pip-Ala, cis-4-Acha, trans-4-Acha, trans-4-Amcha, hLys, Lys, Orn, hArg, Bip, Tic, , Htic, Dip, Sala, Aala, Thza, Thia, Bal, Fala, and Pala;wherein the side-chain amino group of said optionally substituted amino acid is optionally mono- or di-substituted with R³ and R⁴;
AA⁶ is absent or the D- or L-isomer of an amino acid selected from the group consisting of R¹¹, an optionally substituted aromatic α-amino acid, Cys, hCys, Pen, Tpa, Tmpa, Thr, Thr(Bzl), Ser, Ser(Bzl), hArg, Bip, Tic, , Htic, Dip, Sala, Aala, Thza, Thia, Bal, Fala, and Pala;
AA⁷ is absent or the D- or L-isomer of an amino acid selected from the group consisting of R¹¹, an optionally substituted aromatic α-amino acid, A3c, A4c, A5c, A6c, Abu, Aib, Aic, ß-Ala, Arg, Cha, Deg, Gaba, Ile, Leu, Nle, Pip, Pro, Sar, Ser, Ser(Bzl), Thr, Thr(Bzl), Val, Tic, Htic, Sala, Aala, Thza, Thia, Bal, Fala, Pala, hArg, Bip, Bpa, Dip, Pal, Sala, and X⁰-Phe;
AA^{7b} is absent or a D- or L-isomer of an amino acid selected from the group consisting of R¹¹, Bpa, Phe, F₅-Phe, X⁰-Phe, Nal, Pro, Ser, Ser(Bzl), Thr, Thr(Bzl), Trp, hArg, Bip, Tic, Htic, Dip, Sala, Aala, Thza, Thia, Bal, Fala, and Pala;
AA⁸ is absent or the D- or L- isomer of an amino acid selected from the group consisting of R¹¹, Maa, Maaab, Thr, Thr(Bzl), Ser, Ser(Bzl), Tyr, Phe(4-O-Bzl), F₅-Phe, and X⁵-Phe, and an optionally substituted aromatic α-amino acid;
R¹ and R² each is, independently, H, E-, E(O)₂S-, E(O)C-, EOOC-, R¹³, or absent;
R³ and R⁴ each is, independently, (C₁₋₁₂)alkyl, (C₂₋₁₂)alkenyl, (C₂₋₁₂)alkynyl, phenyl, naphthyl, phenyl-(C₁₋₆)alkyl, phenyl-(C₂₋₆)alkenyl, phenyl-(C₂₋₆)alkynyl, naphthyl-(C₁₋₆)alkyl, naphthyl-(C₂₋₆)alkenyl, naphthyl-(C₂₋₆)alkynyl, (cyclo(C₃₋₇)alkyl)-(C₁₋₆)alkyl, (cyclo(C₃₋₇)alkyl)-(C₂₋₆)alkenyl, (cyclo(C₃₋₇)alkyl)-(C₂₋₆)alkynyl, heterocyclyl-(C₁₋₄)alkyl, heterocyclyl-(C₂₋₄)alkenyl, heterocyclyl-(C_{2- 4})alkynyl, 1-adamantyl, 2-adamantyl, 9-fluorenylmethyl, dicyclopropylmethyl, dimethylcyclopropylmethyl, or benzhydryl;
R⁵ is -OR⁶, -NR⁷R⁸, or absent,
wherein each R⁶, R⁷ and R⁸ is, independently, H, (C₁₋₁₂)alkyl, (C₂₋₁₂)alkenyl, (C₂₋₁₂)alkynyl, phenyl, naphthyl, phenyl-(C₁₋₆)alkyl, phenyl-(C₂₋₆)alkenyl, phenyl-(C₂₋₆)alkynyl, naphthyl-(C₁₋₆)alkyl, naphthyl-(C₂₋₆)alkenyl, naphthyl-(C₂₋₆)alkynyl, 1-adamantyl, 2-adamantyl, 9-fluorenylmethyl, dicyclopropylmethyl, dimethylcyclopropylmethyl, or benzhydryl;
R⁹ and R¹⁰ each is, independently, H, (C₁₋₆)alkyl, (C₃₋₄)alkenyl, (C₃₋₄)alkynyl, 1-adamantyl, or 2-adamantyl;
R¹¹ is, independently for each occurrence, a D- or L-amino acid of the formula: 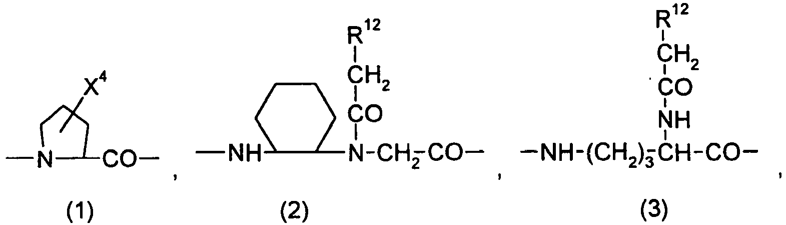 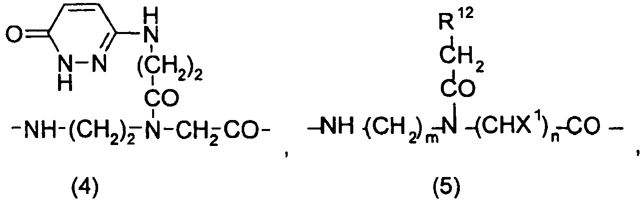 or 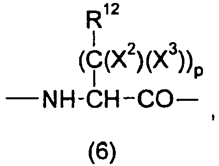 wherein m and n each is, independently, 1, 2, or 3, and p is 0, 1, or 2;
R¹² is, independently for each occurrence, an optionally substituted moiety of the formula: 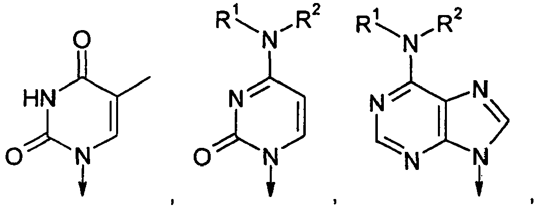 or 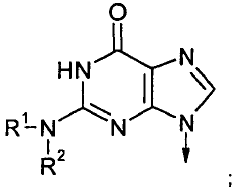 R¹³ is a moiety of the formula 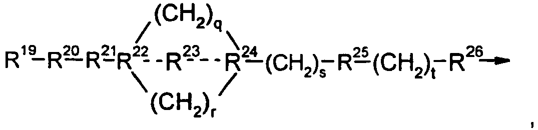 wherein q, r, s, and t each is, independently, 0, 1, 2, 3, 4 or 5;
R¹⁹ is absent, H, NH₂, OH, (C₁₋₆)hydroxyalkyl, N(R²⁷R²⁸), SO₃H, or an optionally substituted moiety selected from the group consisting of heterocyclyl, phenyl and naphthyl,
wherein the optionally substituted moiety defined for R¹⁹ is optionally substituted with one or more substituents selected, independently for each occurrence, from the group consisting of halogen, NO₂, OH, (C₁-₆)alkyl, (C₂₋₆)alkenyl, (C₂₋₆)alkynyl, (C₁-₆)alkoxy, NH₂, mono- or di-(C₁-₆)alkylamino, Bzl, and O-Bzl;
R²¹ is O or absent;
R²¹ is (C₁-₆)alkyl or absent;
R²² is N, O, C, or CH;
R²³ is (C₁-₆)alkyl or absent,
R²⁴ is N , CH , or C;
R²⁵ is NH, O, or absent;
R²⁶ is SO₂, CO, or CH;
R²⁷ and R²⁸ each is, independently, H or (C₁₋₆)alkyl;
E is, independently for each occurrence, an optionally substituted moiety selected from the group consisting of (C₁₋₁₂)alkyl, (C₂₋₁₂)alkenyl, (C₂₋₁₂)alkynyl, phenyl, naphthyl, phenyl-(C₁₋₆)alkyl, phenyl-(C₂₋₆)alkenyl, phenyl-(C₂₋₆)alkynyl, naphthyl-(C₁₋₆)alkyl, naphthyl-(C₂₋₆)alkenyl, naphthyl-(C₂₋₆)alkynyl, (cyclo(C₃₋₇)alkyl)-(C₁₋₆)alkyl, (cyclo(C₃₋₇)alkyl)-(C₂₋₆)alkenyl, (cyclo(C₃₋₇)alkyl)-(C₂₋₆)alkynyl, heterocyclyl-(C₁₋₄)alkyl, heterocyclyl-(C₂₋₄)alkenyl, heterocyclyl-(C₂₋₄)alkynyl, 1-adamantyl, 2-adamantyl, dicyclopropylmethyl, dimethylcyclopropylmethyl, 9-fluorenylmethyl, and benzhydryl;
wherein the optionally substituted moiety defined for E is optionally substituted with one or more substituents each independently selected from the group consisting of halogen, OH, Bzl, O-Bzl, NO₂, CN, COOH, and SH;
X⁰ is halogen, NO₂, OH, (C₁-₆)alkyl, (C₁-₆)alkoxy, mono- or di-(C₁₋₆)alkylamino, Bzl, O-Bzl, NR⁹R¹⁰, or CN;
X¹ is H, (C₁₋₆)alkyl, (C₂₋₆)alkenyl, (C₂₋₆)alkynyl, indolyl, imidazolyl, 1-naphthyl, 3-pyridyl, optionally ring-substituted benzyl, or a moiety which corresponds to the side-chain group of Arg, Leu, Gln, Lys, Tyr, His, Thr, Trp, Phe, Val, Ala, Lys, or His;
wherein said optionally ring-substituted benzyl is optionally substituted with one or more substituents selected from the group consisting of halogen, OH, (C₁₋₆)alkoxy, mono- or di-(C₁₋₆)alkylamino, (C₁₋₄) alkyl, (C₂₋₄) alkenyl, (C₂₋₄) alkynyl, and NR⁹R¹⁰;
X² and X³ each is, independently, H, halogen, OH, =O, =S, (C₁₋₁₂)alkyl, (C₂₋₁₂)alkenyl, (C₂₋₁₂)alkynyl, phenyl, naphthyl, phenyl-(C₁₋₆)alkyl, phenyl-(C₂₋₆)alkenyl, phenyl-(C₂₋₆)alkynyl, naphthyl-(C₁₋₆)alkyl, naphthyl-(C₂₋₆)alkenyl, naphthyl-(C₂₋₆)alkynyl, (cyclo(C₃₋₇)alkyl)-(C₁₋₆)alkyl, (cyclo(C₃₋₇)alkyl)-(C₂₋₆)alkenyl, (cyclo(C₃₋₇)alkyl)-(C₂₋₆)alkynyl, heterocyclyl-(C₁₋₄)alkyl, heterocyclyl-(C₂₋₄)alkenyl, heterocyclyl-(C₂₋₄)alkynyl, 1-adamantyl, 2-adamantyl, dicyclopropylmethyl, or dimethylcyclopropyl methyl;
X⁴ is H, OH, or NH₂; and
X⁵ is halogen, NO₂, CH₃, OH, Bzl or O-Bzl;
provided that:
at least six amino acid residues are present;
when AA³ is a D- or L-isomer of an amino acid selected from the group consisting of Cys, hCys, Pen, Tpa, or Tmpa, and AA⁶ is a D- or L-isomer of an amino acid selected from the group consisting of Cys, hCys, Pen, Tpa, or Tmpa, then AA³ and AA⁶ are connected by a disulfide bond; when AA¹ or AA³ is a D- or L-isomer of an amino acid selected from the group consisting of Mac or Macab, then AA⁸ is a D- or L-isomer of an amino acid selected from the group consisting of Maa and Maaab, and when AA⁸ is a D- or L-isomer of an amino acid selected from the group consisting of Maa and Maaab, then AA¹ or AA³ is a D- or L-isomer of Mac or of Macab, and AA¹ or AA³ is connected by a disulfide bond with AA⁸; AA² can be D- or L-Hca only when AA¹ is absent;
when one of R¹ or R² is E(O)₂S-, E(O)C-, EOOC-, or R¹³, the other is H; when R⁵ is absent, then one of R¹ or R² is also absent, and the N-terminal amino acid and C-terminal amino acid together form an amide bond; when one of X² or X³ is C=O or C=S, the other is absent; and
said compound of formula (I) is not of the formula:
D-Phe-Tyr-cyclo(D-Cys-D-Trp-Lys-Cys)-Abu-Thr-NH₂;
Ac-Phe-Tyr-cyclo(D-Cys-D-Trp-Lys-Cys)-Abu-Thr-NH₂;
L-4-NO₂-Phe-Tyr-cyclo(D-Cys-D-Trp-Lys-Cys)-Abu-Thr-NH₂:
Ac-L-4-NO₂-Phe-Tyr-cyclo(D-Cys-D-Trp-Lys-Cys)-Abu-Thr-NH₂;
Hca-Tyr-cyclo(D-Cys-D-Trp-Lys-Cys)-Abu-Thr-NH₂;
D-Dip-Tyr-cyclo(Cys-D-Trp-Lys-D-Cys)-Val-Nal-NH₂;
D-4-NO₂-Phe-Phe(4-O-Bzl)-cyclo(D-Cys-D-Trp-Lys-Cys)Cha-Nal-NH₂; or
D-4-NO₂-Phe-cyclo(D-Cys-Phe(4-O-Bzl)-D-Trp-Lys-Cys)-Val-Tyr-NH₂.

In another aspect, this invention is directed to a pharmaceutical composition comprising one or more of a compound of formula (I), as defined hereinabove, and a pharmaceutically acceptable carrier.

In yet another aspect, the present invention is directed to a method of eliciting an agonist effect from one or more of a somatostatin and/or neuromedin B subtype receptor in a subject in need thereof, which comprises administering a compound of formula (I), as described hereinabove, to said subject.

In still another aspect, the present invention is directed to a method of eliciting an antagonist effect from one or more of a somatostatin and/or neuromedin B subtype receptor in a subject in need thereof, which comprises administering a compound of formula (I), as described hereinabove, to said subject.

In a further aspect, the present invention is directed to a method of binding one or more somatostatin and/or neuromedin B subtype receptor in a subject in need thereof, which comprises administering a compound of formula (I), as described hereinabove, to said subject.

In a still further aspect, the present invention is directed to the use of one or more compounds according to formula I to bind to the neuromedin B receptor or to one or more of the somatostatin receptors, as when performing an in vitro or in vivo assay.

### DETAILED DESCRIPTION OF THE INVENTION

One of ordinary skill will recognize that certain substituents listed in this invention may have reduced chemical stability when combined with one another or with heteroatoms in the compounds. Such compounds with reduced chemical stability are not preferred.

In general, the compounds of formula (I) can be made by processes which include processes known in the chemical arts for the production of compounds. Certain processes for the manufacture of formula (I) compounds are provided as further features of the invention and are illustrated by the reaction schemes and examples included herein.

In the above structural formulas and throughout the instant application, the following terms have the indicated meanings unless expressly stated otherwise:

The term alkyl is intended to include those alkyl groups of the designated length in either a straight or branched configuration. Exemplary of such alkyl groups are methyl, ethyl, propyl, isopropyl, butyl, sec-butyl, tertiary butyl, pentyl, isopentyl, hexyl, isohexyl and the like. When the term C₀-alkyl is included in a definition it is intended to denote a single covalent bond.

The term alkoxy is intended to include those alkoxy groups of the designated length in either a straight or branched configuration. Exemplary of such alkoxy groups are methoxy, ethoxy, propoxy, isopropoxy, butoxy, isobutoxy, tertiary butoxy, pentoxy, isopentoxy, hexoxy, isohexoxy and the like.

The term halogen or halo is intended to include the halogen atoms fluorine, chlorine, bromine and iodine.

The term cycloalkyl is intended to include a mono-cycloalkyl group or a bi-cycloalkyl group of the indicated carbon number known to those of skill in the art.

The term dimethylcyclopropylmethyl refers to the structure

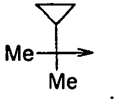

The term aryl is intended to include aromatic rings known in the art, which can be mono-cyclic, bi-cyclic or tri-cyclic, such as phenyl, naphthyl and anthracyl.

The term heterocycle includes mono-cyclic and bi-cyclic systems having one or more heteroatoms, such as oxygen, nitrogen and/or sulfur. The ring systems may be aromatic, for example pyridine, indole, quinoline, pyrimidine, thiophene (also known as thienyl), furan, benzothiophene, tetrazole, dihydroindole, indazole, N-formylindole, benzimidazole, thiazole, and thiadiazole. The ring systems also may be non-aromatic, for example pyrrolidine, piperidine, morpholine and the like.

The chemist of ordinary skill will recognize that certain combinations of heteroatom-containing substituents listed in this invention define compounds which will be less stable under physiological conditions. Accordingly, such compounds are less preferred.

As defined herein, certain residues or moieties are alternatively absent from certain peptides of the invention. Where the bond(s) to such a residue or moiety is indicated by a solid line it is understood that when the residue or moiety is absent a bond is formed between the remaining N-terminal residue or moiety(-ies) and the remaining C-terminal residue or moiety(-ies). Where the bond(s) to such a residue or moiety is indicated by dashed line(s) it is understood that when the residue or moiety is absent no bond is formed between the remaining N-terminal residue or moiety(-ies) and the remaining C-terminal residue or moiety(-ies). For example, in the following structure: 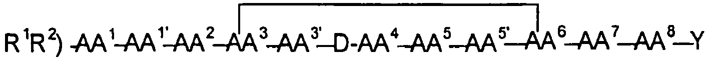 the absence of AA¹ results in 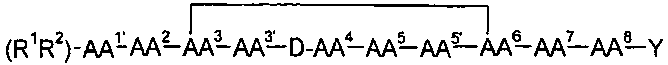 and the absence of AA^{1'} results in 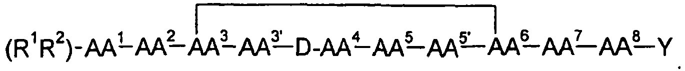 In the following structure: 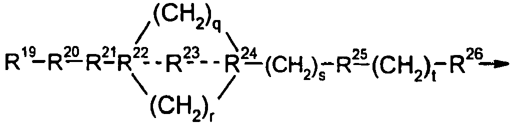 the absence of R²³ results in

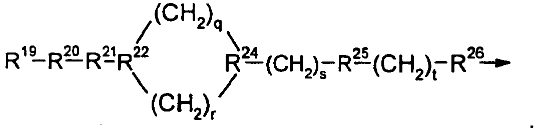

When a chemical structure as used herein has an arrow emanating from it, the arrow indicates the point of attachment. For example, the structure 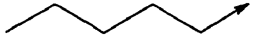 is a pentyl group. When a line is drawn through a cyclic moiety, the line indicates that the substituent can be attached to the cyclic moiety at any of the available bonding points. For example, 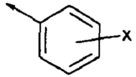 means that the substituent "X" can be bonded ortho, meta or para to the point of attachment. Similarly, when a line is drawn through a bi-cyclic or a tri-cyclic moiety, the line indicates that the substituent can be attached to the bi-cyclic or a tri-cyclic moiety at any of the available bonding points in any of the rings.

For all formulas depicted herein the N-terminus is at the left and the C-terminus at the right in accordance with the conventional representation of a polypeptide chain.

The symbol AA¹, AA², or the like in a peptide sequence stands for an amino acid residue, i.e., =N-CH(R)-CO- when it is at the N-terminus or -NH-CH(R)-CO- when it is not at the N-terminus, where R denotes the side-chain of that amino acid residue. Thus, R is -CH(CH₃)₂ for Val. Also, when the amino acid residue is optically active, it is the L-form configuration that is intended unless D-form is expressly designated.

Unless otherwise indicated, where an acetyl group appears at the N-terminus it is understood that the acetyl group is attached to the α-nitrogen rather than to the side chain of the N-terminal amino acid. For example, the structure of the amino acid sequence Ac-4-NO₂-Phe-AA²-AA³- ... is:

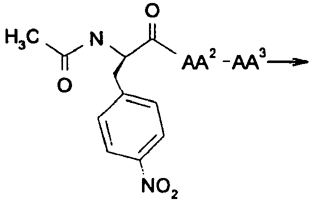

Where the substituent Y appears as, e.g., -OR⁵, at the C-terminus of the peptide, it is to be understood that -OR⁵ is attached directly to the carbonyl carbon in replacement of the -OH group.

E(O)C- stands for 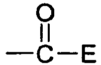 and EOOC- stands for

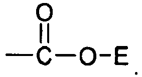

What is meant by "aromatic α-amino acid" is an amino acid residue of the formula 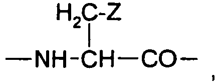 where Z is a moiety containing an aromatic ring. Examples of Z include, but are not limited to, a benzene or pyridine ring and the following structures with or without one or more substituent X on the aromatic ring (where X is, independently for each occurrence, halogen, NO₂, CH₃, OH, Bzl, or O-Bzl): 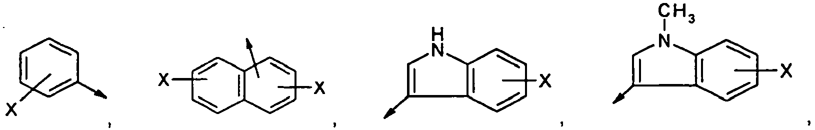 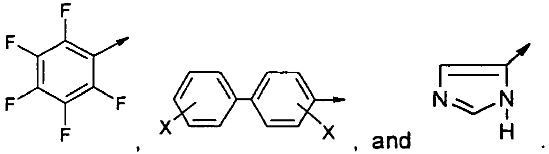 Other examples of an aromatic α-amino acid of the invention are substituted His, such as MeHis, His (τ-Me), or His (π-Me).

What is meant by nucleic acid base is an optionally substituted nucleic acid moiety of the formula: 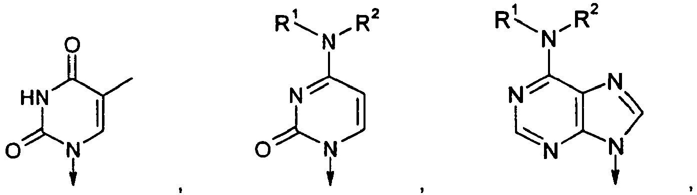 or 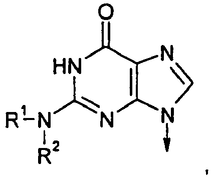 where R¹ and R² are as defined in the claims.

In certain embodiments of the invention the side chain amino group of one or more amino acids is optionally mono- or di-substituted with R³ and R⁴. For example, substituting R³ onto the side chain amino group of 4-Pip-Gly would result in the following structure:

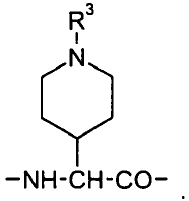

The compounds of the instant invention have at least one asymmetric center. Additional asymmetric centers may be present on the molecule depending upon the nature of the various substituents on the molecule. Each such asymmetric center will produce two optical isomers and it is intended that all such optical isomers, as separated, pure or partially purified optical isomers, racemic mixtures or diastereomeric mixtures thereof, be included within the scope of the instant invention.

The instant compounds can be generally isolated in the form of their pharmaceutically acceptable acid addition salts, such as the salts derived from using inorganic and organic acids. Examples of such acids are hydrochloric, nitric, sulfuric, phosphoric, formic, acetic, trifluoroacetic, propionic, maleic, succinic, D-tartaric, L-tartaric, malonic, methane sulfonic and the like. In addition, certain compounds containing an acidic function such as a carboxy can be isolated in the form of their inorganic salt in which the counter-ion can be selected from sodium, potassium, lithium, calcium, magnesium and the like, as well as from organic bases.

The pharmaceutically acceptable salts are formed by taking about 1 equivalent of a compound of formula (I) and contacting it with about 1 equivalent of the appropriate corresponding acid of the salt which is desired. Work-up and isolation of the resulting salt is well-known to those of ordinary skill in the art.

The compounds of this invention can be administered by oral, parenteral (e.g., intramuscular, intraperitoneal, intravenous or subcutaneous injection, or implant), nasal, vaginal, rectal, sublingual or topical routes of administration and can be formulated with pharmaceutically acceptable carriers to provide dosage forms appropriate for each route of administration. Accordingly, the present invention includes within its scope pharmaceutical compositions comprising, as an active ingredient, at least one of the compounds of formula (I) in association with a pharmaceutically acceptable carrier.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups, the elixirs containing inert diluents commonly used in the art, such as water. Besides such inert diluents, compositions can also include adjuvants, such as wetting agents, emulsifying and suspending agents, and sweetening, flavoring and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, excipients such as coca butter or a suppository wax.

Compositions for nasal or sublingual administration are also prepared with standard excipients well known in the art.

Further, a compound of this invention can be administered in a sustained release composition such as those described in the following patents. U.S. Patent No. 5,672,659 teaches sustained release compositions comprising a bioactive agent and a polyester. U.S. Patent No. 5,595,760 teaches sustained release compositions comprising a bioactive agent in a gelable form. U.S. Application No. 08/929,363 filed September 9, 1997, teaches polymeric sustained release compositions comprising a bioactive agent and chitosan. U.S. Application No. 08/740,778 filed November 1, 1996, teaches sustained release compositions comprising a bioactive agent and cyclodextrin. U.S. Application No. 09/015,394 filed January 29, 1998, teaches absorbable sustained release compositions of a bioactive agent.

In general, an effective dosage of active ingredient in the compositions of this invention may be varied; however, it is necessary that the amount of the active ingredient be such that a suitable dosage form is obtained. The selected dosage depends upon the desired therapeutic effect, on the route of administration, and on the duration of the treatment, all of which are within the realm of knowledge of one of ordinary skill in the art. Generally, dosage levels of between 0.0001 to 100 mg/kg of body weight daily are administered to humans and other animals, e.g., mammals.

A preferred dosage range is 0.01 to 10.0 mg/kg of body weight daily, which can be administered as a single dose or divided into multiple doses, or provided for continuous administration.

Compounds of the instant invention can be and were assessed for their ability to bind to a somatostatin subtype receptor according to the following assays.

The affinity of a compound for human somatostatin subtype receptors 1 to 5 (sst₁, sst₂, sst₃, sst₄ and sst₅, respectively) is determined by measuring the inhibition of [¹²⁵I-Tyr¹¹]SRIF-14 binding to CHO-K1 cells transfected with the sst receptor subtype.

The human sst₁ receptor gene was cloned as a genomic fragment. A 1.5 Kb *Pst*l*-Xmn*I segment containing 100 bp of the 5'-untranslated region, 1.17 Kb of the entire coding region, and 230 bp of the 3'-untranslated region was modified by the Bg1II linker addition. The resulting DNA fragment was subcloned into the *BamH*I site of a pCMV-81 to produce the mammalian expression plasmid (provided by Dr. Graeme Bell, University of Chicago, Chicago, IL.). A clonal cell line stably expressing the sst₁ receptor was obtained by transfection into CHO-K1 cells (American Type Culture Collection, Manassas, VA) ("ATCC") using the calcium phosphate co-precipitation method. The plasmid pRSV-neo (ATCC) was included as a selectable marker. Clonal cell lines were selected in RPMI 1640 media (Sigma Chemical Co., St. Louis, MO) containing 0.5 mg/ml of geneticin (Gibco BRL, Grand Island, NY) ring cloned, and expanded into culture.

The human sst₂ somatostatin receptor gene, isolated as a 1.7Kb *Bam*HI*-Hind*III genomic DNA fragment and subcloned into the plasmid vector pGEM3Z (Promega), was kindly provided by Dr. G. Bell (University of Chicago, Chicago, IL.). The mammalian cell expression vector is constructed by inserting the 1.7Kb *BamH*1*-Hind*II fragment into compatible restriction endonuclease sites in the plasmid pCMV5. A clonal cell line is obtained by transfection into CHO-K1 cells using the calcium phosphate co-precipitation method. The plasmid pRSV-neo is included as a selectable marker.

The human sst₃ was isolated at genomic fragment, and the complete coding sequence was contained within a 2.4 Kb *Bam*HI/*Hind*III fragment. The mammalian expression plasmid, pCMV-h3 was constructed by inserting the a 2.0 Kb Ncol-Hindlll fragment into the EcoR1 site of the pCMV vector after modification of the ends and addition of EcoR1 linkers. A clonal cell line stably expressing the sst₃ receptor was obtained by transfection into CHO-K1 cells (ATCC) using the calcium phosphate co-precipitation method. The plasmid pRSV-neo (ATCC) was included as a selectable marker. Clonal cell lines were selected in RPMI 1640 media containing 0.5 mg/ml of G418 (Gibco), ring cloned, and expanded into culture.

The human sst₄ receptor expression plasmid, pCMV-HX was provided by Dr. Graeme Bell (University of Chicago, Chicago, IL.). The vector contains the 1.4 Kb *Nhe*l-*Nhe*l genomic fragment encoding the human sst₄, 456 bp of the 5'-untranslated region and 200 bp of the 3'-untranslated region, cloned into the *Xba*I/*Eco*R1 sites of PCMV-HX. A clonal cell line stably expressing the sst₄ receptor was obtained by transfection into CHO-K1 cells (ATCC) using the calcium phosphate co-precipitation method. The plasmid pRSV-neo (ATCC) was included as a selectable marker. Clonal cell lines were selected in RPMI 1640 media containing 0.5 mg/ml of G418 (Gibco), ring cloned, and expanded into culture.

The human sst₅ gene was obtained by PCR using a λ genomic clone as a template, and kindly provided by Dr. Graeme Bell (University of Chicago, Chicago, IL). The resulting 1.2 Kb PCR fragment contained 21 base pairs of the 5'-untranslated region, the full coding region, and 55 bp of the 3'-untranslated region. The clone was inserted into EcoR1 site of the plasmid pBSSK(+). The insert was recovered as a 1.2 Kb *Hind*III*-Xba*I fragment for subcloning into pCVM5 mammalian expression vector. A clonal cell line stably expressing the SST₅ receptor was obtained by transfection into CHO-K1 cells (ATCC) using the calcium phosphate co-precipitation method. The plasmid pRSV-neo (ATCC) was included as a selectable marker. Clonal cell lines were selected in RPMI 1640 media containing 0.5 mg/ml of G418 (Gibco), ring cloned, and expanded into culture.

CHO-K1 cells stably expressing one of the human sst receptors are grown in RPMI 1640 containing 10% fetal calf serum and 0.4 mg/ml geneticin. Cells are collected with 0.5 mM EDTA, and centrifuged at 500g for about 5 minutes at about 4°C. The pellet is resuspended in 50 mM Tris[hydroxymethyl]aminomethane hydrochloride, pH = 7.4 at 25°C, ("Tris buffer"), and centrifuged twice at 500g for about 5 minutes at about 4°C. The cells are lysed by sonication and centrifuged at 39,000g for about 10 minutes at about 4°C. The pellet is resuspended in the same buffer and centrifuged at 50,000g for about 10 minutes at about 4°C and membranes in resulting pellet are stored at - 80°C.

Competitive inhibition experiments of [¹²⁵I-Tyr¹¹]SRIF-14 binding are run in duplicate in polypropylene 96 well plates. Cell membranes (10 µg protein/well) are incubated with [¹²⁵I-Tyr¹¹]SRIF-14 (Dr. Tom Davis, Univ. of Arizona, Tuscon, AZ) (0.05 nM) for about 60 minutes at about 37°C in 50 mM HEPES, 0.2% BSA, 2.5 mM MgCl₂.

Bound from free [¹²⁵I-Tyr¹¹]SRIF-14 is separated by immediate filtration through GF/C glass fiber filter plate (Unifilter, Packard, Meriden, CT) presoaked with 0.3% polyethylenimine (P.E.I.), using Filtermate 196 (Packard) cell harvester. Filters are washed with 50 mM Tris-HCl at about 0-4°C for about 4 seconds and assayed for radioactivity using Packard Top Count.

Specific binding is obtained by subtracting nonspecific binding (determined in the presence of 0.1 µM SRIF-14) from total binding. Binding data are analyzed by computer-assisted nonlinear regression analysis (Data Analysis Toolbox, v.1.0, Molecular Design Limited, San Leandro, CA) and inhibition constant (Ki) values are determined.

Whether a compound of the instant invention is an SST agonist or antagonist of somatostatin is determined by the following assay.

Functional Assay: Inhibition of cAMP Intracellular Production

CHO-K1 Cells expressing human somatostatin (SRIF-14) subtype receptors are seeded in 24-well tissue culture multidishes in RPMI 1640 media with 10% fetal calf serum (FCS). The medium is changed the day before the experiment.

Cells at 10⁵ cells/well are washed 2 times by 0.5 ml RPMI 1640 media. Fresh RPMI 1640 media with 0.2% BSA and supplemented with 0.5mM 3-isobutyl-1-methylxanthine ("IBMX") is added, and the cells are incubated for about 5 minutes at about 37°C. Cyclic AMP production is stimulated by the addition of 1mM forskolin ("FSK") (Sigma Chemical Co., St. Louis, MO) for about 15-30 minutes at about 37°C.

The agonist effect of a compound is measured by the simultaneous addition of FSK (1µM), SRIF-14 (Bachem, Torrence, CA), (10⁻¹² M to 10⁻⁶ M) and a test compound (10⁻¹⁰ M to 10⁻⁵ M). The antagonist effect of a compound is measured by the simultaneous addition of FSK (1µM) , SRIF-14 (1 to 10 nM) and a test compound (10⁻¹⁰ M to 10⁻⁵ M).

The reaction medium is removed and 200 ml 0.1 N HCl is added. cAMP is measured using radioimmunoassay method (Kit FlashPlate SMP001A, New England Nuclear, Boston).

Compounds of the instant invention can be and were assessed for its ability to bind to a neuromedin B receptor according to the following assay.

Cell Culture: Balb 3T3 cells, expressing the rat NMB receptor, were obtained from Dr. R.T. Jensen (National Institutes of Health, Bethesda, MD), and cultured in Dulbecco's modified Eagle's medium ("DMEM") containing 10% fetal calf serum, 0.5 mg/ml of G418 (Gibco). The cells were maintained at 37°C in a humidified atmosphere of 5% CO₂/95% air.

Radioligand Binding: Membranes were prepared for radioligand binding studies by homogenization of the cells in 20 ml of ice-cold 50 mM Tris-HCl with a Brinkman Polytron (Westbury, NY) (setting 6, 15 sec). The homogenates were washed twice by centrifugation (39,000g /10 minutes), and the final pellets were resuspended in 50 mM Tris-HCl containing 5.0 mM MgCl₂, and 0.1% BSA. For assay, aliquots (0.4 ml) were incubated with 0.05 nM [¹²⁵I-Tyr⁴]bombesin (2200 Ci/mmol, New England Nuclear, Boston, MA), with and without 0.05 ml of unlabeled competing test peptides. After incubation (30 minutes, 4°C), the bound [¹²⁵I-Tyr⁴]bombesin was separated from the free by rapid filtration through GF/C filters (Brandel, Gaithersburg, MD), which had been previously soaked in 0.3% polyethyleneimine. The filters were then washed three times with 5-ml aliquots of ice-cold 50 mM Tris-HCl, and the bound radioactivity trapped on the filters was counted by gamma spectrometry (Wallac LKB, Gaithersburg, MD). Specific binding was defined as the total [¹²⁵I-Tyr⁴]bombesin bound minus that bound in the presence of 1000 nM neuromedin B (Bachem, Torrence, CA).

One embodiment of the method includes the step of contacting the cells with a peptide of Formula (II): 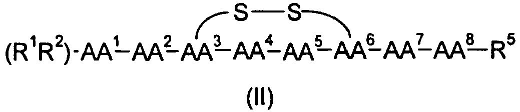 or a pharmaceutically acceptable salt thereof,
wherein
AA¹ is absent or the D- or L-isomer of an amino acid selected from the group consisting of R¹¹, Aac, Aic, Arg, Asn, Asp, Dip, Gln, Glu, Hyp, Lys, Mac, Macab, Orn, Pip, Pro, Ser, Ser(Bzl), Thr, Thr(Bzl), Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp and an optionally substituted aromatic α-amino acid, wherein said optionally substituted aromatic α-amino acid is optionally substituted with one or more substituents selected from the group consisting of halogen, NO₂, OH, CN, (C₁₋₆)alkyl, (C₂₋₆)alkenyl, (C₂₋₆)alkynyl, and NR⁹R¹⁰;
AA² is absent or the D- or L-isomer of an amino acid selected from the group consisting of R¹¹, Aic, Arg, Hca, His, Hyp, Pal, F₅-Phe, Phe, Pro, Trp, X⁰-Phe, Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, and Pyp;AA³ is the D- or L-isomer of an amino acid selected from the group consisting of Cys, hCys, Pen, Tpa and Tmpa;
AA⁴ is a D- or L-isomer of an amino acid selected from the group consisting of Trp, N-Met-Trp, β-Met-Trp, His, hHis, hArg, Bip, Tic, , Htic, Dip, Sala, Aala, Thza, Thia, Bal, Fala, Pala, and an optionally substituted aromatic α-amino acid,
wherein said optionally substituted aromatic α-amino acid is optionally substituted with one or more substituents each independently selected from the group consisting of halogen, NO₂, OH, (C₁₋₄)alkyl, (C₂₋₄)alkenyl, (C₂₋₄)alkynyl, Bzl, O-Bzl, and NR⁹R¹⁰;
AA⁵ is a D- or L-isomer of an amino acid selected from the group consisting of 4-Pip-Gly, 4-Pip-Ala, *cis*-4-Acha, *trans*-4-Acha, *trans*-4-Amcha, hLys, Lys, Orn, hArg, Bip, Tic, , Htic, Dip, Sala, Aala, Thza, Thia, Bal, Fala, and Pala,
wherein the side-chain amino group of said amino acid is optionally mono- or di-substituted with R³ and R⁴;
AA⁶ is a D- or L-isomer of an amino acid selected from the group consisting of Cys, hCys, Pen, Tpa, and Tmpa;
AA⁷ is absent or a D- or L-isomer of an amino acid selected from the group consisting of R¹¹, Aic, A3c, A4c, A5c, A6c, Abu, Aib, ß-Ala, Arg, Bpa, Cha, Deg, Gaba, His, Ile, Leu, Nal, Nle, Pal, Phe, F₅-Phe, Pro, Sar, Ser, Ser(Bzl), Thr, Thr(Bzl), Trp, N-Me-Trp, Val, N-Me-Val, hArg, Bip, Tic, , Htic, Dip, Sala, Aala, Thza, Thia, Bal, Fala, Pala, and X⁰-Phe ;
AA⁸ is absent or the D- or L-isomer of an amino acid selected from the group consisting of R¹¹, an optionally substituted aromatic α-amino acid, Maa, Maaab, Ser, Ser(Bzl), Thr, Thr(Bzl), Tyr, Phe(4-O-Bzl), F₅-Phe, and X⁵-Phe; R¹³ is a moiety according to the formula 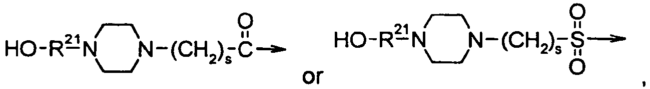 wherein R²¹ is (C₁₋₄)alkyl and s is 1, 2, 3, or 4; and
X⁰ is halogen, NO₂, CH₃, OH, Bzl, O-Bzl or CN;
provided that at least one of AA⁷ or AA⁸ is present.

Another embodiment of the method includes the step of contacting the cells with a peptide of Formula (III): 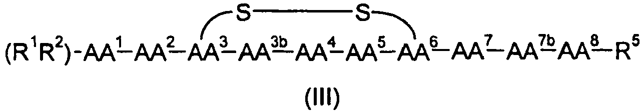 or a pharmaceutically acceptable salt thereof,
wherein
AA¹ is absent or the D- or L-isomer of an amino acid selected from the group consisting of R¹¹, Aac, Aic, Arg, Asn, Asp, Gln, Glu, Hca, His, Hyp, Lys, Mac, Macab, Orn, Pro, Ser, Ser(Bzl), Thr, Thr(Bzl), Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp and an optionally substituted aromatic α-amino acid,
wherein said optionally substituted aromatic α-amino acid is optionally substituted with one or more substituents selected from the group consisting of halogen, NO₂, OH, CN, (C₁₋₆)alkyl, (C₂₋₆)alkenyl, (C₂₋₆)alkynyl, and NR⁹R¹⁰;
AA³ is a D- or L-isomer of an amino acid selected from the group consisting of Cys, hCys, Pen, Tpa, and Tmpa;
AA^{3b} is the D- or L-isomer of an amino acid selected from the group consisting of R¹¹, Arg, Bpa, F₅-Phe, His, Nal, Pal, 4-Pal, Phe, Trp, hArg, Bip, Tic, , Htic, Dip, Sala, Aala, Thza, Thia, Bal, Fala, Pala, and X⁵-Phe;
AA⁴ is a D- or L-isomer of an amino acid selected from the group consisting of Trp, N-Met-Trp, β-Met-Trp, His, hHis, hArg, Bip, Tic, , Htic, Dip, Sala, Aala, Thza, Thia, Bal, Fala, Pala, and an optionally substituted aromatic α-amino acid;
wherein said optionally substituted aromatic α-amino acid is optionally substituted with one or more substituents each independently selected from the group consisting of halogen, NO₂, OH, CN, (C₁-₄)alkyl, (C₂₋₄)alkenyl, (C₂₋₄)alkynyl, Bzl, O-Bzl, and NR⁹R¹⁰;
AA⁵ is a D- or L-isomer of an amino acid selected from the group consisting of 4-Pip-Gly, 4-Pip-Ala, cis-4-Acha, trans-4-Acha, trans-4-Amcha, hLys, Lys and Orn, and, hArg, Bip, Tic, , Htic, Dip, Sala, Aala, Thza, Thia, Bal, Fala, Pala,
wherein the side-chain amino group of said amino acid is optionally mono-
or di-substituted with R³ and R⁴;
AA⁶ is a D- or L-isomer of an amino acid selected from the group consisting of Cys, hCys, Pen, Tpa, and Tmpa;
AA⁷ is absent or a D- or L-isomer of an amino acid selected from the group consisting of R¹¹, Aic, A3c, A4c, A5c, A6c, Abu, Aib, ß-Ala, Arg, Bpa, Cha, Deg, Gaba, His, Ile, Leu, Nal, Nle, Pal, Phe, F₅-Phe, Pro, Sar, Ser, Ser(Bzl), Thr, Thr(Bzl), Trp, N-Me-Trp, Val, N-Me-Val, hArg, Bip, Tic, , Htic, Dip, Sala, Aala, Thza, Thia, Bal, Fala, Pala, and X⁰-Phe;
X⁰ is halogen, NO₂, CH₃, OH, CN, Bzl or O-Bzl;
R¹ and R² each is, independently, H, E-, E(O)₂S-, E(O)C-, EOOC-, R¹³, or absent;
R⁵ is -OR⁶ or -NR⁷R⁸;
R¹³ is a moiety of the formula 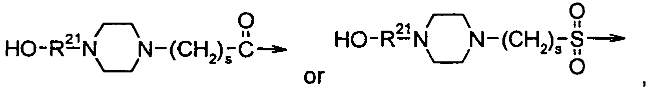 wherein R²¹ is (C₁₋₄)alkyl and s is 1, 2, 3, or 4;
provided that:
at least one of AA¹ or AA² is present;
when AA¹ is a D- or L-isomer of Pro, Hyp, Arg, Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp or His, AA² cannot be a D- or L-isomer of Pro, Hyp, Arg, Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp or His;
when AA⁷ is a D- or L-isomer of Thr or of Ser, AA⁸ cannot be a D- or L-isomer of Thr or of Ser;
at least one of AA¹, AA², AA^{3b}, AA⁷, AA^{7b}, or AA⁸ is the D- or L-isomer of R¹¹; and
when one of X² or X³ is =O or =S, the other is absent;
or a pharmaceutically acceptable salt thereof.

Yet another embodiment of the method includes the step of contacting the cells with a peptide of Formula (IV): 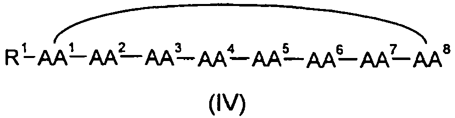 wherein
AA¹ is absent, the D- or L-isomer of an amino acid selected from the group consisting of R¹¹, Aic, Hyp, Pro, Ser, Ser(Bzl), Thr, Thr(Bzl), and an optionally substituted aromatic α-amino acid;
wherein said optionally substituted aromatic α-amino acid is optionally substituted with one or more substituents each independently selected from the group consisting of halogen, NO₂, OH, (C₁₋₆)alkyl, (C₂₋₆)alkenyl, (C₂₋₆)alkynyl, (C₁₋₆)alkoxy, Bzl, O-Bzl, and NR⁹R¹⁰;
AA² is absent or the D- or L-isomer of an amino acid selected from the group consisting of R¹¹, Arg, F₅-Phe, His, Pal, Phe, Trp, and X⁰-Phe;
AA³ is the D- or L-isomer of an optionally substituted aromatic α-amino acid, wherein said optionally substituted aromatic α-amino acid is optionally substituted with one or more substituents selected from the group consisting of halogen, NO₂, OH, (C₁₋₄)alkyl, (C₂₋₄)alkenyl, (C₂₋₄)alkynyl, Bzl, O-Bzl, and NR⁹R¹⁰;
AA⁴ is a D- or L-isomer of an optionally substituted amino acid selected from the group consisting of Lys, Orn, hLys, cis-4-Acha, trans-4-Acha, trans-4-Amcha, 4-Pip-Gly, and 4-Pip-Ala,
wherein the side chain amino group of said optionally substituted amino acid is optionally substituted with R³ and R⁴;
AA⁵ is absent or a D- or L-isomer of R¹¹, A3c, A4c, A5c, A6c, Abu, Aib, Aic, ß-Ala, Bpa, Cha, Deg, F₅-Phe, Gaba, Ile, Leu, Nal, Nle, Pal, Phe, Pro, Sar, Ser, Ser(Bzl), Thr, Thr(Bzl), Trp, N-Me-Trp, Val, N-Me-Val, or X⁰-Phe;
AA⁶ is absent, the D- or L-isomer of R¹¹, an aromatic α-amino acid, F₅-Phe, Phe, Thr, Thr(Bzl), Ser, Ser(Bzl), or X⁰-Phe;
AA⁷ is absent, the D- or L-isomer of R¹¹ or the D- or L-isomer of an aromatic α-amino acid;
AA⁸ is a D- or L- isomer of R¹¹;
R¹ is H, E-, E(O)₂S-, E(O)C-, EOOC-, or R¹³;
R¹³ is a moiety of the formula 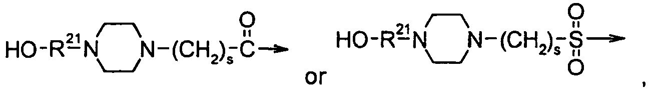 wherein R²¹ is (C₁₋₄)alkyl and s is 1, 2, 3, or 4;
X⁰ in the definition of AA² and AA⁵ is halogen, NO₂, OH, (C₁-₆)alkyl, (C₁₋₆)alkoxy, mono- or di-(C₁-₆)alkylamino, Bzl or O-Bzl;
X⁰ in the definition of AA⁶ is halogen, NO₂, OH, (C₁-₆)alkyl, (C₁-₆)a)koxy, mono- or di-(C₁-₆)alkylamino, Bzl, O-Bzl, or NR⁹R¹⁰;
provided that:
at least one of AA¹ or AA² is present;
when AA¹ is absent, AA² and AA⁸ together form a bond; and
at least two of AA⁵, AA⁶, and AA⁷ are present;
or a pharmaceutically acceptable salt thereof.

### ABBREVIATIONS:

- (A(z))aeg: (A)aeg where the amino group of the adenine moiety is protected with carbobenzyloxy, i.e., 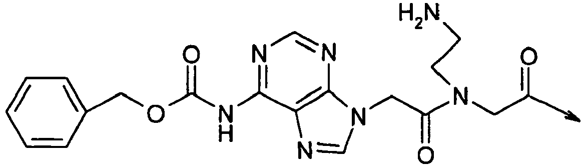
- (A)aeg: N-(2-aminoethyl)-N-(2-adeninyl-1-oxo-ethyl)-glycine
- (C(z))aeg: (C)aeg where the amino group of the cytosine moiety is protected with carbobenzyloxy,
i.e., 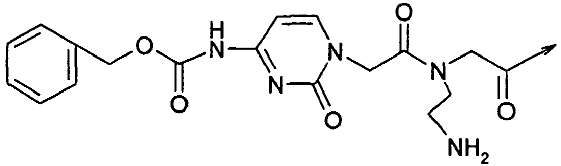
- (C)aeg: N-(2-aminoethyl)-N-(2-cytosinyl-1-oxo-ethyl)-glycine
- (G(z))aeg: (G)aeg where the amino group of the guanine moiety is protected with carbobenzyloxy, i.e., 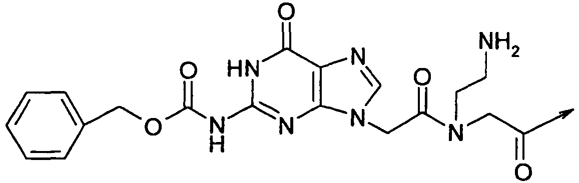
- (G)aeg: N-(2-aminoethyl)-N-(2-guaninyl-1-oxo-ethyl)-glycine
- (T)aeg: N-(2-aminoethyl)-N-(2-thyminyl-1-oxo-ethyl)-glycine
- A3c: 1-Amino-1-cyclopropane-1-carboxylic acid
- A4c: 1-Amino-1-cyclobutane-1-carboxylic acid
- A5c: 1-Amino-1-cyclopentane-1-carboxylic acid
- A6c: 1-Amino-1-cyclohexane-1-carboxylic acid
- Aaa: 2-Aminoanthraquinone
- Aac: an aminoalkyl carboxylic acid of the formula H₂N-(CH₂)ₙ-COOH, wherein n is 2 - 6
- Aala: Anthrylalanine
- Aba: N- (4-aminobenzoyl)-β-alanine
- Abp: 4-amino-1-benzylpiperidine
- Abu: 2-Aminobutyric Acid
- Ac: acetyl, i.e., CH3-C(O)-;
- Ach: trans-1, 4-Diaminocyclohexane
- 4-Acha: 3-(4-aminocyclohexyl) alanine
- Ads: 1-Amino-deoxy-D-sorbitol
- aeg: Aminoethylglycine
- Agly: Allylglycine
- Ahep: 1-Amino-4- (2-hydroxyethyl) Piperazine
- Aib: 2-Aminoisobutyric Acid
- Aic: 2-aminoindan-2-carboxylic acid
- 5Aiq: 5-Amino Isoquinoline
- Alla: Allantoic acid
- 4-Amcha: 3-((4-aminomethyl)cyclohexyl)alanine
- Amp: 1-Amino-4-methylpiperazine
- Apa: 2,3-Diaminopropionic acid
- Api: 1-(3-Aminopropyl) imidazole
- Bal: 3-Benzothienylalanine
- Bip: 4,4'-Biphenylalanine
- BOC: Tertiarybutyloxycarbonyl
- Bpa: 3-(4-biphenyl)-alanine
- Bzl: the benzyl radical
- Bzop: 4-Benzoylphenylalanine
- C4c: Cinnoline-4-Carboxylic acid
- Car: Carnosine
- Cbz: carbobenzyloxy radical
- Cha: 3-Cyclohexylalanine
- α-Chpa: Alpha-cyclohexylphenylacetic acid
- Cit: citrinin
- Cmp: 4-Carboxymethylpiperidine
- Cmpi: 4-Carboxymethylpiperazine
- Cpa: 2-, 3-, or 4-chloro phenylalanine, unless otherwise indicated
- Dap: 2, 3-Diaminopropionic acid
- Dapy: 2,6-Diaminopyridine
- DCM: dichloromethane
- Deg: Diethylglycine
- D-Ga: D-Glucosamine
- Dip: 3,3-Diphenylalanine
- DiPa: 3,5-Diiodo-4-Pyridone-1-acetic acid
- DIPEA: diisopropylethylamine
- DMF: dimethylformamide
- Edp: 4,4'-Ethylenedipiperidine
- Edt: 4,4'-Ethylenedi-m-toluidine
- **F₅-Phe**: 3-(Pentafluorophenyl)-alanine
- Fala: 2-Furylalanine
- FMOC: 9-Fluorenylmethoxycarbonyl
- Fpp: 1-(4-Fluorophenyl) piperazine
- Gaba: 4-Aminobutyric Acid
- Gba: 4-Guanidinobenzoic acid
- HATU: O-(7-azabenzotriazolyl)-1,1,3,3-tetramethyluronuim hexafluorophosphate
- HBTU: O-(benzotriazol-1-yl)-1,1,3,3-tetramethyluronuim hexafluorophosphate
- Hca: Hydrocinnamic acid (3-phenylpropionic acid)
- hCys: homocysteine
- Hep: 1-(2-Hydroxyethyl) piperazine
- hLys: homolysine
- HOAT: 1-hydroxy-7-azabenzotriazole
- Htic: 1,2,3,4-tetrahydroisoquinoline-7-hydroxy-3-carboxylic acid
- Htqa: 4-Hydroxy-7-Trifluoromethyl-3-quinoline carboxylic acid
- Hyd: Hydralazine
- Hyp: 4-Hydroxyproline
- laa: N- (3-Indolylacetyl)-L-Alanine
- lia: 2-Imino-1-imidazolidine acetic acid
- Ina: N- (3-Indolylacetyl)-L-Phenylalanine
- Inc: Indoline-2-Carboxylic Acid
- Inic: Isonicotinic acid
- Inip: Isonipecotic acid
- Ipa: 3-Indole Propionic Acid
- 1-Iqc: 1-Isoquinolinecarboxylic acid
- 3-Iqc: 3- Isoquinolinecarboxylic acid
- 5-Iqs: 5-Isoquinoline sulfonic Acid 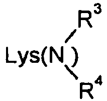 Lys with its ε amino group substituted with R³ and R⁴
- Lys(diEt): Lys with its ε amino group disubstituted by two ethyl groups
- Lys(iPr): Lys with its ε amino group monosubstituted by an isopropyl group
- Maa: a mercaptoalkyl amine of the formula HS-(CH₂)ₙ-NH₂, wherein n is 2-6;
- Maaab: a o-, m-, or p-(mercaptoalkyl)(aminoalkyl) benzene of the formula 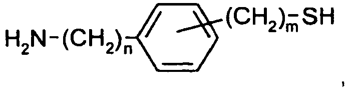 wherein m and n each is, independently, 0, 1, or 2.
- Mac: a mercaptoalkyl carboxylic acid of the formula HS-(CH₂)ₙ-COOH, wherein n is 2 - 6;
- Macab: a o-, m-, or **p-(mercaptoalkyl)(carboxyalkyl)** benzene of the formula 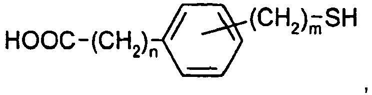 wherein m and n each is, independently, 0, 1, or 2.
- MBHA: 4-methylbenzhydrylamine
- Me-Trp: Trp with its indolyl nitrogen substituted with methyl
- Mim: Mimosine
- Mnf: 5-(4-methyl-2-nitrophenyl)-2-furoic acid
- Mpip: 1-Methylpiperazine
- 4-Mqc: 4-methoxy-2-quinolinecarboxylic acid
- Nal: 3-(2-naphthyl)-alanine, unless otherwise indicated
- Nip: Nipecotic acid
- Nle: norleucine
- Nua: Nicotinuric acid
- O-Bzl: the benzyloxy radical
- Orn: ornithine 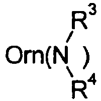 Orn with its amino group substituted with R³ and R⁴
- Pal: 3-(3-Pyridyl)-alanine, unless otherwise indicated
- 2-Pala: 2-Pyridylalanine
- 3-Pala: 3-Pyridylalanine
- 4-Pala: 4-Pyridylalanine
- Pap: 4'-Piperazinoacetophenone
- Pen: penicillamine
- Pgly: Propargylglycine
- Phg: phenylglycine
- Pip: pipecolinic acid
- 4-Pip-Ala: 3-(4-piperidyl)alanine
- 4-Pip-Gly: (4-piperidyl)glycine
- Pnf: para-Nitro-phenylalanine (i.e., 4-Nitro-phenylalanine)
- Ppc: 4-Phenylpiperidine-4-carboxylic Acid
- Pyp: 3-pyridine propionic acid
- Sala: Styrylalanine
- Sar: sarcosine (i.e., N-methylglycine)
- Thi: Thiaproline
- 2-Thia: 2-Thienylalanine
- 3-Thia: 3-Thienylalanine
- Thn: 1, 2, 3, 4-Tetrahydro-2-naphthoic acid
- Thnc: 1,2,3,4-Tetrahydronorbarman-3-carboxylic acid
- Thza: 4-Thiazolylalanine
- Tic: 1,2,3,4-tetrahydro-3-isoquinolinecarboxylic acid
- Tmpa: 3-(p-thiomethylphenyl)-alanine
- Tpa: 3-(p-thiophenyl)-alanine
- Tpr: Thioproline
- Tra: Tranexamic acid
- TrPa: Tryptamine
- X-Phe: phenylalanine with p-, o- or m-substituents X on its benzene ring, e.g., 3-(4-chlorophenyl)-alanine
- z: carbobenzyloxy

Administration of a pharmaceutically acceptable salt of a compound covered by formula (I) into a patient whose disorder arises from biochemical activity induced by NMB or somatostatin is also within the present invention. In other words, the peptides can be provided in the form of pharmaceutically acceptable salts, e.g., acid addition salts, or metal complexes, e.g., with zinc, iron or the like. Illustrative examples of acid addition salts are those with organic acids such as acetic, lactic, pamoic, maleic, citric, malic, ascorbic, succinic, benzoic, palmitic, suberic, salicylic, tartric, methanesulfonic or toluenesulfonic acid, those with polymeric acids such as tannic acid or carboxymethyl cellulose, and those with inorganic acids such as hydrochloric acid, hydrobromic acid, sulfuric acid or phosphoric acid.

Other features and advantages of the present invention will be apparent from the following description of the preferred embodiments, and also from the claims. It is believed that one skilled in the art can, based on the description herein, utilize the present invention to its fullest extent. The following specific embodiments are therefore to be construed as merely illustrative and not limitative of the remainder of the disclosure in any way whatsoever.

### DESCRIPTION OF THE PREFERRED EMBODIMENTS

In a preferred embodiment the invention features a compound according to Formula II, wherein
AA¹ is absent, Ac-D-Phe, or the D- or L- isomer of R¹¹, Pip, Pro, or Ser, or of an aromatic α-amino acid selected from the group consisting of Cpa, Dip, Nal, Pal, and Phe;
AA² is Aic, Pal, Phe, F₅-Phe, 4-NO₂-Phe, Trp, Tyr, Phe(4-O-Bzl), or absent;
AA³ is the D- or L- isomer of an amino acid selected from the group consisting of Pen, Cys, hCys and Tmpa;
AA⁴ is the D- or L-isomer of Trp or of His;
AA⁵ is Lys, hLys, N-Me-Lys, Orn, cis-4-Acha or 4-Pip-Ala;
AA⁶ is the D- or L-isomer of an amino acid selected from the group consisting of Cys, hCys, Pen and Tmpa;
AA⁷ is A3c, A4c, A5c, A6c, Abu, Aic, β-alma, Gaba, Nle, F₅-Phe, Phe, Pro, Sar, Ser, Thr, Thr(Bzl), Tyr, Val or absent; and
AA⁸ is R¹¹, Nal, Thr, Thr(Bzl), Tyr, Phe(4-O-Bzl), or absent;
or a pharmaceutically acceptable salt thereof.

In a more preferred embodiment the invention features a compound according to the immediately foregoing, wherein
AA¹ is absent or the D- or L- isomer of R¹¹, Pip or Pro, or of an aromatic α-amino acid selected from the group consisting of Cpa, Dip, Nal, Pal, Phe, and Ac-Phe;
AA² is Tyr, Pal, Phe, 4-NO₂-Phe, Trp, or absent;
AA³ is a D- or L-isomer of Cys or Pen;
AA⁴ is D-Trp;
AA⁵ is Lys, Orn, or cis-4-Acha;
AA⁶ is a D- or L-isomer of Cys or Pen;
AA⁷ is A3c, A4c, A5c, A6c, Abu, Aic, β-Ala, Gaba, Nle, Phe, Pro, Sar, Thr, Thr(Bzl), Tyr, Val, or absent; and
AA⁸ is R¹¹, Thr, Tyr, Nal, or absent;
or a pharmaceutically acceptable salt thereof.

In another preferred embodiment the invention features a compound according to Formula III, wherein
AA¹ is R¹¹, Aic, Hca, Pro, Ser, Ser(Bzl), Trp, Tyr, or a D- or L-isomer of an aromatic α-amino acid selected from the group consisting of Cpa, Nal, Ac-Nal, Phe, Ac-Phe, 4-NO₂-Phe, and Ac-4-NO₂-Phe;
AA² is Pal, Phe, F₅-Phe, Tyr, or absent;
AA³ is a D- or L-isomer of Cys, hCys, Pen or Tmpa;
AA^{3b} is Pal, 4-Pal, His, Trp, Tyr, Phe(4-O-Bzl), Phe, or R¹¹;
AA⁴ is a D- or L-isomer of Trp or His;
AA⁵ is Lys, N-Me-Lys, Orn, hLys, cis-4-Acha, or 4-Pip-Ala;
AA⁶ is a D- or L-isomer of Cys, hCys, Pen or Tmpa;
AA⁷ is R¹¹, A4c, A5c, Abu, ß-Ala, Gaba, Phe, F₅-Phe, Ser(Bzl), Thr, Thr(Bzl), Phe(4-O-Bzl), or absent;
AA^{7b} is R¹¹, Nal, F₅-Phe, X⁰-Phe or absent, wherein X⁰ is halogen, NO₂, CH₃, OH, Bzl or O-Bzl; and
AA⁸ is R¹¹, Nal, Tyr, Phe(4-O-Bzl), or absent;
or a pharmaceutically acceptable salt thereof.

In a more preferred embodiment the invention features a compound according to the immediately foregoing, wherein
AA' is R¹¹, Aic, Hca, Pro, Ser(Bzl), or a D- or L-isomer of an aromatic α-amino acid selected from the group consisting of Cpa, Nal, Ac-Nal, Phe, Ac-Phe, 4-NO₂-Phe, and Ac-4-NO₂-Phe;
AA² is Pal, Tyr, or absent;
AA³ is a D- or L-isomer of Cys or Pen;
AA^{3b} is R¹¹, Pal, 4-Pal, Trp, Tyr, Phe(4-O-Bzl), or Phe, wherein R" is (T)aeg; AA⁴ is D-Trp;
AA⁵ is Lys, N-Me-Lys, Orn, or cis-4-Acha;
AA⁶ is a D- or L-isomer of Cys or Pen;
AA⁷ is R¹¹, A5c, Abu, Ser(Bzl), Thr, Thr(Bzl), Phe(4-O-Bzl), Gaba, or absent;
AA^{7b} is Nal, X⁰-Phe or absent; and
AA⁸ is Tyr or absent;
or a pharmaceutically acceptable salt thereof.

In yet another preferred embodiment the invention features a compound according to Formula IV, wherein
AA¹ is Aic, Hyp, Cpa, D-Cpa, Nal, Pal, Phe, Pro, R¹¹, Tyr or absent;
AA² is Phe, Trp, F₅-Phe, His, Tyr, Phe(4-O-Bzl), or R¹¹;
AA³ is a D-isomer of Trp, His, or Pal;
AA⁴ is Lys, N-Me-Lys, Orn, hLys, cis-4-Acha, or 4-Pip-Ala;
AA⁵ is Pal, Phe(4-O-Bzl), Thr(Bzl), Thr, Sar, Gaba, ß-Ala, A4c, A5c, A6c, Abu, Aic or absent;
AA⁶ is Thr, Tyr, Ser, F₅-Phe, Cpa, Nal, or D- or L-Phe;
AA⁷ is Nal, Pal, or absent; and
AA⁸ is R¹¹;
or a pharmaceutically acceptable salt thereof.

In yet another more preferred embodiment the invention features a compound according to the immediately foregoing, wherein
AA¹ is Cpa, Nal, Pal, Phe, Tyr or absent;
AA² is Phe, Tyr, Trp, or R¹¹;
AA³ is D-Trp;
AA⁴ is Lys, N-Me-Lys, or cis-4-Acha;
AA⁵ is Pal, Phe(4-O-Bzl), Aic, Gaba, A5c or absent;
AA⁶ is Thr, Nal, or D- or L-Phe;
AA⁷ is absent; and
AA⁸ is R¹¹;
or a pharmaceutically acceptable salt thereof.

In still yet another preferred embodiment the invention features a compound according to Formula II, wherein R¹ and R⁵ are absent and the N-terminal amino acid and the C-terminal amino acid together form an amide bond; or a pharmaceutically acceptable salt thereof.

In still yet another preferred embodiment the invention features a compound according to Formula III, wherein R¹ and R⁵ are absent and the N-terminal amino acid and the C-terminal amino acid together form an amide bond; or a pharmaceutically acceptable salt thereof.

In a most preferred embodiment the invention features a compound according to Formula II, wherein said compound is of the formula:
Ac-D-Phe-Tyr-cyclo(D-Cys-D-Trp-Lys-Cys)-Abu-Thr-NH₂;
Nal-Tyr-cyclo(Cys-D-Trp-Lys-D-Cys)-Val-Nal-NH₂;
Nal-Tyr-cyclo(Cys-D-Trp-Lys-D-Cys)-Abu-Nal-NH2;
D-Dip-Tyr-cyclo(Cys-D-Trp-Lys-D-Cys)-Abu-Nal-NH₂;
Dip-Tyr-cyclo(D-Cys-D-Trp-Lys-D-Cys)-Abu-Nal-NH2;
Nal-Tyr-cyclo(D-Cys-D-Trp-Lys-D-Cys)-Abu-Nal-NH₂;
Dip-Tyr-cyclo(D-Cys-D-Trp-Lys-D-Cys)-Val-Nal-NH₂;
Nal-Tyr-cyclo(D-Cys-D-Trp-Lys-D-Cys)-Val-Nal-NH₂;
cyclo(D-Phe-Tyr-cyclo(D-Cys-D-Trp-Lys-Cys)-Abu-Thr);
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo (D-Cys-D-Trp-Lys-D-Cys)-A6c-Nal-NH₂;
(G(z))aeg-cyclo(D-Cys-D-Trp-Lys-D-Cys)-A5c-Nal-NH₂;
Pal-cyclo(D-Cys-D-Trp-Lys-D-Cys)-A5c-Nal-NH₂;
Cpa-Pal-cyclo(D-Cys-D-Trp-Lys-D-Cys)-B-Ala-Nal-NH₂;
Cpa-Pal-cyclo(D-Cys-D-Trp-Lys-D-Cys)-Sar-Nal-NH₂;
Cpa-Pal-cyclo(D-Cys-D-Trp-Lys-D-Cys)-Gaba-Nal-NH₂; or
Cpa-Pal-cyclo(D-Cys-D-Trp-Lys-D-Cys)-Pro-Nal-NH₂;
or a pharmaceutically acceptable salt thereof.

In another most preferred embodiment the invention features a compound according to Formula II, wherein said compound is of the formula:
Phe-cyclo(Cys-D-Trp-Lys-Cys)-Thr-NH₂;
Phe-Tyr-cyclo(D-Cys-D-Trp-Lys-Cys)-Abu-Thr-NH₂;
Ac-D-Phe-Tyr-cyclo(D-Cys-D-Trp-Lys-Cys)-Abu-Thr-NH₂;
Nal-Tyr-cyclo(Cys-D-Trp-Lys-D-Cys)-Val-Nal-NH₂;
Nal-Tyr-cyclo(Cys-D-Trp-Lys-D-Cys)-Abu-Nal-NH₂;
Dip-Tyr-cyclo(D-Cys-D-Trp-Lys-D-Cys)-Abu-Nal-NH₂;
Nal-Tyr-cyclo(D-Cys-D-Trp-Lys-D-Cys)-Abu-Nal-NH₂;
Dip-Tyr-cyclo(D-Cys-D-Trp-Lys-D-Cys)-Val-Nal-NH₂;
Nal-Tyr-cyclo(D-Cys-D-Trp-Lys-D-Cys)-Val-Nal-NH₂;
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp-Lys-D-Cys)-A6c-Nal-NH₂;
(G(z))aeg-cyclo(D-Cys-D-Trp-Lys-D-Cys)-A5c-Nal-NH₂;
D-Cpa-cyclo(Cys-D-Trp-Lys-D-Cys)-A5c-Nal-NH₂;
Pal-cyclo(D-Cys-D-Trp-Lys-D-Cys)-A5c-Nal-NH₂;
Cpa-cyclo(D-Cys-D-Trp-Lys-D-Cys)-A5c-Nal-NH₂;
Cpa-Pal-cydo(D-Cys-D-Trp-Lys-D-Cys)-ß-Ala-Nal-NH₂;
Cpa-Pal-cyclo(D-Cys-D-Trp-Lys-D-Cys)-Sar-Nal-NH₂;
Cpa-Pal-cyclo(D-Cys-D-Trp-Lys-D-Cys)-Aic-Nal-NH₂;
Cpa-Pal-cyclo(D-Cys-D-Trp-Lys-D-Cys)-Gaba-Nal-NH₂;
Cpa-Pal-cyclo(D-Cys-D-Trp-Lys-D-Cys)-Pro-Nal-NH₂;
(T)aeg-cyclo(D-Cys-D-Trp-Lys-D-Cys)-(A)aeg-NH₂;
Cpa-Pal-cyclo(D-Cys-D-Trp-Lys-D-Cys)-A4c-Nal-NH₂;
Cpa-Pal-cyclo(D-Cys-D-Trp-Lys-D-Cys)-Nal-NH₂;
Pal-cyclo(D-Cys-D-Trp-Lys-D-Cys)-Nal-NH₂;
Pro-Phe-cyclo(Cys-D-Trp-Lys-D-Cys)-Val-NH₂;
Pro-Phe-cyclo(D-Cys-D-Trp-Lys-Cys)-Val-NH₂;
Pip-4-NO2-Phe-cyclo(D-Cys-D-Trp-Lys-D-Cys)-Nle-NH₂;
(G)aeg-Pal-cyclo(D-Cys-D-Trp-Lys-D-Cys)-Thr(Bzl)-(C)aeg-NH₂;
(C)aeg-Pal-cyclo(D-Cys-D-Trp-Lys-D-Cys)-Thr(Bzl)-(G)aeg-N H₂;
Pro-Phe-c(D-Cys-D-Trp-Lys-D-Cys)-Nle-Phe-NH₂;
Pro-Phe-c(D-Cys-D-Trp-Lys-D-Cys)-Thr-Nle-NH₂;
Pro-Phe-c(D-Cys-D-Trp-Lys-D-Cys)-Thr-Phe-NH₂;
Cpa-Phe-c(D-Cys-D-Trp-Lys-D-Cys)-Gaba-NH₂;
Cpa-Phe-c(D-Cys-D-Trp-Lys-D-Cys)-Gaba-Tyr-NH₂;
Pip-Phe-c(D-Cys-D-Trp-Lys-D-Cys)-NH₂;
Pip-Phe-c(Cys-D-Trp-Lys-Cys)-Gaba-NH₂; or
Pro-Phe-c(D-Cys-D-Trp-Lys-D-Cys)-Thr-NH₂;
or a pharmaceutically acceptable salt thereof.

In yet another most preferred embodiment the invention features a compound according to Formula III, wherein said compound is of the formula:
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂;
D-Phe-cyclo(Cys-Tyr-D-Trp-Lys-Cys)-Thr-NH₂;
D-4-NO₂-Phe-cyclo(D-Cys-Tyr-D-Trp-Lys-Cys)-Nal-NH₂;
Ac-D-4-NO₂-Phe-cyclo(D-Cys-Tyr-D-Trp-Lys-Cys)-Nal-NH₂;
D-4-NO₂-Phe-Pal-cyclo(D-Cys-Phe(4-O-Bzl)-D-Trp-Lys-Cys)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys)-Thr-Tyr-NH₂;
D-4-NO₂-Phe-cyclo(D-Cys-Pal-D-Trp-Lys-Cys)-Thr(Bzl)-NH₂;
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Cys-Pal-D-Trp-Lys-Cys)-Thr(Bzl)-Tyr-NH₂;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NH₂;
D-Cpa-cyclo(D-Cys-(T)aeg-D-Trp-Lys-Cys)-Thr(Bzl)-Tyr-NH₂;
(C)aeg-c(D-Cys-Pal-D-Trp-Lys-D-Cys)-Thr(Bzl)-Tyr-NH₂;
D-Cpa-c(D-Cys-Pal-D-Trp-Lys-D-Cys)Thr(Bzl)-Tyr-NH₂;
(T)aeg-c(Pen-Pal-D-Trp-Lys-D-Cys)Thr(Bzl)-Tyr-NH₂;
(T)aeg-c(D-Cys-Trp-D-Trp-Lys-D-Cys)Thr(Bzl)-Tyr-NH₂;
(T)aeg-c(D-Cys-Phe-D-Trp-Lys-D-Cys)Thr(Bzl)-Tyr-NH₂;
(T)aeg-c(D-Cys-Pal-D-Trp-Orn-D-Cys)Thr(Bzl)-Tyr-NH₂;
(T)aeg-c(D-Cys-Pal-D-Trp-hLys-D-Cys)Thr(Bzl)-Tyr-NH₂;
(T)aeg-c(D-Cys-Pal-D-Trp-lamp-D-Cys)Thr(Bzl)-Tyr-NH₂;
(T)aeg-c(D-Cys-Pal-D-Trp-Cha(4-am)-D-Cys)Thr(Bzl)-Tyr-NH₂;
(T)aeg-c(D-Cys-Pal-D-Trp-Lys-D-Cys)-Ser(Bzl)-Tyr-NH₂;
(T)aeg-c(D-Cys-Pal-D-Trp-Lys-D-Cys)Thr(Bzl)-D-Tyr-NH₂;
(T)aeg-c(D-Cys-Pal-D-Trp-Lys-D-Cys)Thr(Bzl)-Trp-NH₂;
(T)aeg-c(D-Cys-Pal-D-Trp-Lys-D-Pen)Thr(Bzl)-Tyr-NH₂;
(C)aeg-c(D-Cys-Phe-D-Trp-Lys-D-Cys)Thr(Bzl)-Tyr-NH₂;
Ina-c(D-Cys-Phe-D-Trp-Lys-D-Cys)-Thr(Bzl)-Tyr-NH₂;
Mnf-c(D-Cys-Phe-D-Trp-Lys-D-Cys)-Thr(Bzl)-Tyr-NH₂;
Inp-c(D-Cys-Phe-D-Trp-Lys-D-Cys)-Thr(Bzl)-Tyr-NH₂:
Nua-c(D-Cys-Phe-D-Trp-Lys-D-Cys)-Thr(Bzl)-Tyr-NH₂;
Pyp-c(D-Cys-Phe-D-Trp-Lys-D-Cys)-Thr(Bzl)-Tyr-NH₂;
c(D-Cys-Phe-D-Trp-Lys-D-Cys)-Thr(Bzl)-Tyr-NH₂;
(T)aeg-Pal-c(D-Cys-D-Trp-Lys-D-Cys)Thr(Bzl)-Tyr-NH₂;
(T)aeg-Pal-c(D-Cys-D-Trp-Lys-D-Cys)Tyr(Bzl)-Thr-NH₂;
(C)aeg-Phe-c(D-Cys-D-Trp-Lys-D-Cys)Thr(Bzl)-Tyr-NH₂;
(T)aeg-D-Trp-c(D-Cys-Pal-Lys-D-Cys)Thr(Bzl)-Leu-NH₂;
or a pharmaceutically acceptable salt thereof.

In still yet another most preferred embodiment the invention features a compound according to Formula III, wherein said compound is of the formula:
Hca-cyclo(D-Cys-Tyr-D-Trp-Lys-Cys)-Nal-NH₂;
Ac-Nal-cyclo(D-Cys-Tyr-D-Trp-Lys-Cys)-Nal-NH₂;
Ac-D-Phe-cyclo(D-Cys-Tyr-D-Trp-Lys-Cys)-Nal-NH₂;
Ac-D-Nal-cyclo(D-Cys-Tyr-D-Trp-Lys-Cys)-Nal-NH₂;
D-Phe-cyclo(D-Cys-Tyr-D-Trp-Lys-Cys)-Nal-NH₂;
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂;
D-Phe-cyclo(Cys-Tyr-D-Trp-Lys-Cys)-Thr-NH₂;
D-4-NO₂-Phe-cyclo(D-Cys-Tyr-D-Trp-Lys-Cys)-Nal-NH₂;
Ac-D-4-NO₂-Phe-cyclo(D-Cys-Tyr-D-Trp-Lys-Cys)-Nal-NH₂;
D-4-NO₂-Phe-Pal-cyclo(D-Cys-Phe(4-O-Bzl)-D-Trp-Lys-Cys)-Tyr-NH₂;
D-4-NO₂-Phe-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys)-Thr(Bzl)-NH₂;
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Cys-Pal-D-Trp-Lys-Cys)-Thr(Bzl)-Tyr-NH₂;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-Cys)-Thr(Bzl)-Tyr-NH₂;
(C)aeg-cyclo(D-Cys-Pal-D-Trp-Lys-Cys)-Thr(Bzl)-Tyr-NH2;
Aic-cyclo(D-Cys-Pal-D-Trp-Lys-Cys)-Thr(Bzl)-Tyr-NH₂;
(C(z))aeg-cyclo(D-Cys-Pal-D-Trp-Lys-Cys)-Thr(Bzl)-Tyr-NH₂;
(A(z))aeg-cyclo(D-Cys-Pal-D-Trp-Lys-Cys)-Thr(Bzl)-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pai-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NH₂;
D-Cpa-cyclo(D-Cys-(T)aeg-D-Trp-Lys-Cys)-Thr(Bzl)-Tyr-NH₂;
(T)aeg-cyclo(D-Cys-Pal-D-Trp-Lys-D-Cys)-Thr(Bzl)-p-Me-Phe-NH₂;
Ac-(T)aeg-cyclo(D-Cys-Pal-D-Trp-Lys-D-Cys)-Thr(Bzl)-Tyr-NH₂;
(T)aeg-cyclo(D-Cys-Pal-D-Trp-Lys-D-Cys)-Nal-NH₂;
D-Cpa-cyclo(D-Cys-Pal-D-Trp-Lys-D-Cys)-Nal-NH₂;
(A)aeg-cyclo(D-Cys-Pal-D-Trp-Lys-D-Cys)-Thr(Bzl)-Tyr-NH₂; or
(C)aeg-cyclo(D-Cys-Pal-D-Trp-Lys-D-Cys)-Thr(Bzl)-Tyr-NH₂;
or a pharmaceutically acceptable salt thereof.

In still another most preferred embodiment the invention features a compound according to Formula IV, wherein said compound is of the formula:
cyclo(Trp-D-Trp-Lys-Phe(4-O-Bzl)-Phe-(T)aeg);
cyclo(Trp-D-Trp-Lys-Pal-Phe -(T)aeg); or
cyclo(Phe-Phe-D-Trp-Lys-Thr-(T)aeg);
or a pharmaceutically acceptable salt thereof.

### PREPARATION OF PEPTIDES

Peptides were synthesized on Rink Amide MBHA resin, (4-(2', 4'-dimethoxyphenyl-Fmoc-aminomethyl)-phenoxyacetamido-norleucyl-MBHA resin), using a standard solid phase protocol of FMOC chemistry and cleaved with a TFA/Phenol/H₂O/triisoproylsilane (83ml/5g/10ml/2ml) mixture. Peptides were cyclized in CH₃CN/H₂O (5ml/5ml) using EKATHIOX™ resin (EKAGEN Corporation, San Carlos, CA) and purified on C₁₈ silica (Rainin Instruments Co., Woburn, MA, now Varian Analytical, Walnut Creek, CA), using acetonitrile/0.1% trifluoroacetic acid buffers. Homogeneity was assessed by analytical HPLC and mass spectrometry and was determined to be >95% for each peptide.

Peptides having general structure 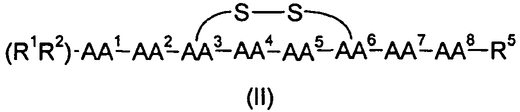 or 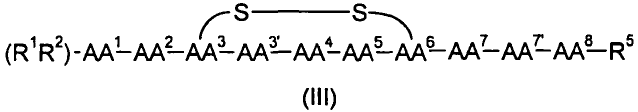 that is, having a cyclic tetra- or pentapeptyl backbone, were synthesized on Rink Amide MBHA resin, (4-(2',4'-dimethoxyphenyl-Fmoc-aminomethyl)-phenoxyacetamidonor-leucyl-MBHA resin), following a standard solid phase protocol of Fmoc-chemistry until the desired peptide was assembled. Final cleavage/deprotection was achieved by the treatment of the peptide-resin with a cocktail of TFA/Phenole/H₂O/Triisopropylsilane (83:5:10:2 mUg/mUmL).

Cyclization (S-S bond formation) was achieved by dissolving the linear peptide in a 50% mixture of CH₃CN/H₂O, except where otherwise indicated, followed by the addition of 2.5 eq. of EKATHIOX resin then stirring overnight.

Peptides were purified on C₁₈ silica column using acetonitrile/0.1%TFA buffer. Homogeneity was assessed by analytical HPLC and MAS spectrometry and was determined to be >95% for each peptide except where otherwise indicated.

Peptides having a carboxylic function at their C-Terminal were synthesized on Wang resin (p-Benzyloxybenzyl Alcohol resin), cleaved from resin and deprotected by cocktail B (TFA:Phenole:H₂O:Triisopropylsilane in the ratio 88:5:5:2).

Head-To-Tail cyclic peptides having the general structure of 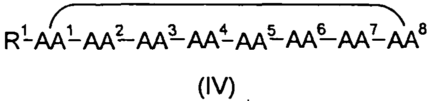 were synthesized first as a totally protected linear peptide on 2-chlorotrityl chloride resin.

The first Fmoc deprotection was carried out using 5% piperidine in DMF/DCM (1:1) for about 10 minutes followed by 25% piperidine in DMF for about 15 minutes. All subsequent deprotections were performed using a standard solid phase protocol of FMOC chemistry.

Protected linear peptides were obtained by treating the resin with acetic acid/TFE/DCM (1:1:8 by vol.) for about 60 minutes at room temperature.

Head-to-tail cyclization was achieved using HATU/HOAT/DIPEA as the coupling/cyclization reagent. The all-protected cyclic peptide was treated with a cocktail of TFA/Phenole/H₂O/Triisopropylsilane (83:5:10:2 mL/g/mL/mL) for about 2 1/2 hours to achieve final deprotection.

Peptides were purified on C₁₈ silica column using acetonitrile/0.1%TFA buffer as eluant. Homogeneity was assessed by analytical HPLC and MAS spectrometry and was determined to be >97% pure for each peptide.

As noted above, certain compounds of the invention incorporate one or more of the amino acid moiety R¹¹, having the structure 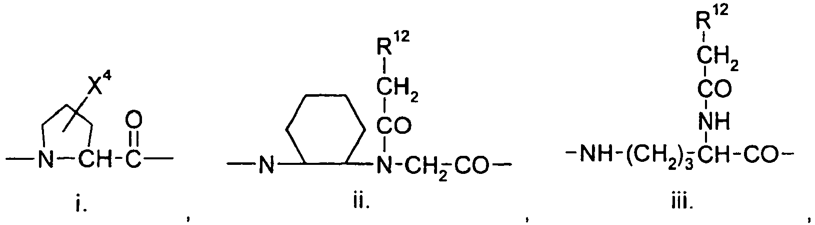 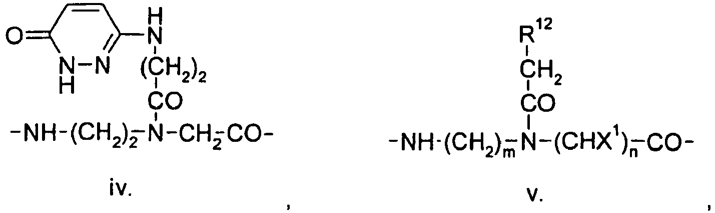 or 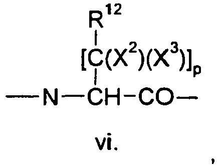 wherein R¹², X¹, X², X³, X⁴, m, n, and p each is as defined in the claims. It will be apparent to one skilled in the art of chemical synthesis that the various R¹¹ amino acids may be readily synthesized using appropriate starting materials and known synthesis procedures. Examples of pertinent procedures may be found in the following publications, hereby incorporated by reference: aminoethylglycine: Tetrahedron, vol. 51, pp. 6179 (1995); Bioorganic & Medicinal Chemistry Letters, vol 5, No. 11, p.1159 (1995); Tetrahedron, vol. 53, no. 43, p. 14671 (1997); Nucleosides, Nucleotides, vol. 16 (10 & 11), p. 1893 (1997); α,α-dialkylated amino acid with nucleobase side chain, Proc. Natl. Acad. Sci. USA, vo. 92, p. 12013 (1995); aminocyclohexylglycine, Chem. Eur. J. vol. 3. No. 6, p. 912 (1997); σ-N-Boc-α-N-(thymin-1-ylacetyl)ornithine, Bioorganic & Medicinal Chemistry Letters, vol. 6, no. 7, p. 793 (1996); substituted proline, J. Chem. Soc. Perkin. Trans., vol 1, pp. 539, 547, 555 (1997); N-(aminomethyl)-β-alanine, Tetrahedron Lett., vol. 36, No. 38, p. 6941 (1995); substituted ornithine, Nucleosides & Nucleotides, vol 17 (1-3), pp. 219, 339 (1998); structure vi., Tetrahedron Lett., Vol. 36, no 10, p. 1713 (1995); Tetrahedron Lett, Vol. 38, no 48, p. 8363 (1997); structure v., Tetrahedron Lett., Vol 39, p. 4707 (1998); compound iv., J. Amer. Chem. Soc., vol. 119, p. 11116 (1997); aminoproline, Bioorganic & Medicinal Chemistry Lett., vol. 7, no. 6, p. 681 (1997); chiral polynucleic acid, Tetrahedron Lett., vol. 35, no. 29, p. 5173 (1994); Bioorganic & Medicinal Chemistry Lett., vol. 4, no. 8, p. 1077 (1994).

Below is a detailed description regarding the synthesis of Analog #1. Other peptides of the invention can be prepared by making appropriate modifications, within the ability of someone of ordinary skill in the art of peptide synthesis.

### Step 1: Preparation of Fmoc-Nal-O-tert-Butyl-Tyr-S-trityl-D-Cys-N-in-t-Boc-D-Trp-N-ε-t-Boc-Lys-S-trityl-D-Cys-Abu-Nal-4-(2',4'-Dimethoxphenylamino methyl) phenoxyacetamido-norluacyl-4-methylbenzhydrylamine resin.

Rink amide MBHA resin (Novabiochem, Inc., San Diego, CA), 1 g, (0.53 mmole), was placed in reaction vessel #1 (RV-1) of a Model 90 peptide synthesizer, (Advanced ChemTech, Louisville, KY). The peptide synthesizer was programmed to perform the following reaction cycle:
a. Dimethylformamide;
b. 25% piperidine in dimethylformamide (2 times for 15 minutes each, with 1 time wash with DMF in between);
c. DMF washes (3x10 mL, 1 minute each);

The resin was stirred with FMOC-Nal (2.12 mmol), 2-(1H-benzotriazol-1-yl)-1, 1, 3, 3-tetramethyluronium hexafluorophosphate (HBUT) (2.01 mmole), and diisopropylethyl amino (4.24 mmole) in dimethylformamide for about 1½ hours and the resulting amino acid resin was then cycled through steps (a) to (c) in the above wash program. The Nal-resin was coupled with Fmoc-Abu, then cycled as described above. It was dried under vacuum.

The following amino acids (1.4 mmole) were coupled successively to the peptide resin (0.35 mmole), by the same procedure: Fmoc-S-Trityl-D-Cys, Fmoc-N-ε-t-Boc-Lys, Fmoc-N-in-t-Boc-D-Trp. The peptide resin, after drying under vacuum, was split and one portion coupled with Fmoc-S-Trityl-D-Cys, Fmoc-O-t-butyl-Tyr. The coupled portion was split again and one portion coupled with Fmoc-Nal. After washing with DMF (3 x 10 mL, 1 minute each) and drying under vacuum, the completed resin weighed 0.242 g.

### Step 2: Preparation of H-Nal-Tyr-D-Cys-D-Trp-Lys-D-Cys-Abu-Nal-NH₂

The peptide resin obtained from Step 1 (0.24 g, 0.087 mmole) was mixed with a freshly prepared solution of TFA (8.8 mL), phenol (0.5g), H₂O (0.5 mL) and triisopropylsilane (0.2mL) at room temperature and stirred for about 2½ hours. Excess TFA was evaporated under reduced pressure to an oily residue. Ether was then added to the oily residue and the free linear peptide was precipitated, filtered, then washed with dry ether. The crude peptide was then dissolved in 11mL of CH₃CN/H₂O/0.1N HOAc (5mL/5mL/1mL), followed by the addition of 200 mg EKATHIOX^{®} resin. The mixture was stirred overnight and then filtered. The filtrate was evaporated to a small volume then applied to a column (22-250 mm) of microsorb octadecylsilane silica (5µm), followed by elution with a linear gradient (30% to 80%, 30 minutes) of acetonitrile in water, in which both solvents have 0.1% trifluoroacetic acid. Fractions were examined by analytical high performance liquid chromatography ("HPLC") and pooled to give maximum purity. Lyophilization of the solutions from water gave 10 mg of the product as a white, fluffy powder. The product was found to be homogeneous by HPLC C₁₈ silica using the same eluant as immediately above, (tR = 16.646 minutes). Infusion mass spectrometry confirmed the composition of the cyclic octapeptide; (MW 1178.45).

## Claims

1. A compound of formula (I):
(R¹R²)-AA¹-AA²-AA³-AA^{3b}-AA⁴-AA⁵-AA⁶-AA⁷-AA^{7b}-AA⁸-R⁵ (I)
or a pharmaceutically acceptable salt thereof,
wherein
the α-nitrogen of AA¹, AA², AA³, AA^{3b}, AA⁴, AA⁵, AA⁶, AA⁷, AA^{7b}, and AA⁸ each is, independently, optionally substituted with (C₁₋₄)alkyl, (C₃₋₄)alkenyl, (C₃₋₄)alkynyl, or (C₁₋₆)alkyl=C(O)-;
AA¹ is absentor the D- or L-isomer of an amino acid selected from the group consisting of R¹¹, Aac, Aic, Arg, Asn, Asp, Dip, Gln, Glu, Hca, Hyp, Lys, Mac, Macab, Orn, Pro, Ser, Ser(Bzl), Thr, Thr(Bzl), Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, C4c, 5-Iqs, Htqa, 4-Mqc, Thn, α-Chpa, Cit, Nua, Pyp and an optionally substituted aromatic α-amino acid;
wherein said optionally substituted aromatic α-amino acid is optionally substituted with one or more substituents each independently selected from the group consisting of halogen, NO₂, OH, CN, (C₁₋₆)alkyl, (C₂₋₆)alkenyl, (C₂₋₆)alkynyl, (C₁₋₆)alkoxy, Bzl, O-Bzl, and NR⁹R¹⁰;
AA² is absent or the D- or L-isomer of an amino acid selected from the group consisting of R¹¹, Aic, Arg, Hca, His, Hyp, Pal, F₅-Phe. Phe, Pro, Trp, and X⁰-Phe Pip, hArg, Bip, Bpa, Tic, Cmp, , Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, and Pyp;
AA³ is the D- or L-isomer of an amino acid selected from the group consisting of Cys, hCys, Pen, Tpa, Tmpa, Mac, Macab, and an optionally substituted aromatic α-amino acid;
wherein said optionally substituted aromatic α-amino acid is optionally substituted with one or more substituents selected from the group consisting of halogen, NO₂, OH, CN, (C₁₋₄)alkyl, (C₂₋₄)alkenyl, (C₂₋₄)alkynyl, (C₁₋₄)alkoxy, Bzl, O-Bzl, NR⁹R¹⁰, Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, and Pyp; AA^{3b} is absent or the D- or L-isomer of an amino acid selected from the group consisting of Pal, 4-Pal, His, Arg, Nal, Trp, Bpa, F₅-Phe. Phe, X⁰-Phe, R¹¹, hArg, Bip, Tic, , Htic, Dip, Sala, Aala, Thza, Thia, Bal, Fala, and Pala;
AA⁴ is a D- or L-isomer of an optionally substituted amino acid or of an optionally substituted aromatic α-amino acid;
wherein said optionally substituted amino acid is selected from the group consisting of Trp, Lys, Orn, hLys, *cis*-4-Acha, *trans*-4-Acha, *trans*-4-Amcha, 4-Pip-Gly, N-Met-Trp, β-Met-Trp, His, hHis, hArg, Bip, Tic, Htic, Dip, Sala, Aala, Thza, Thia, Bal, Fala, Pala, and 4-Pip-Ala;
wherein the side chain amino group of said optionally substituted amino acid is optionally substituted with R³ and R⁴; and
wherein said optionally substituted aromatic α-amino acid is optionally substituted with one or more substituents each independently selected from the group consisting of halogen, NO₂, OH, CN, (C₁₋₄)alkyl, (C₂₋₄)alkenyl, (C₂₋₄)alkynyl, Bzl, O-Bzl, and NR⁹R¹⁰;
AA⁵ is absent, R¹¹, Aic, A3c, A4c, A5c, A6c, Abu, Aib, ß-Ala, Bpa, Cha, Deg, Gaba, Ile, Leu, Nal, Nle, Pro, Sar, Ser, Ser(Bzl), Thr, Thr(Bzl), Trp, Val, Pal, F₅-Phe, Phe, X⁰-Phe, or an optionally substituted D- or L- isomer of an amino acid selected from the group consisting of 4-Pip-Gly, 4-Pip-Ala, cis-4-Acha, *trans*-4-Acha, *trans*-4-Amcha, hLys, Lys, Orn, hArg, Bip, Tic, , Htic, Dip, Sala, Aala, Thza, Thia, Bal, Fala, and Pala;wherein the side-chain amino group of said optionally substituted amino acid is optionally mono- or di- substituted with R³ and R⁴;
AA⁶ is absent or the D- or L-isomer of an amino acid selected from the group consisting of R¹¹, an optionally substituted aromatic α-amino acid, Cys, hCys, Pen, Tpa, Tmpa, Thr, Thr(Bzl), Ser, Ser(Bzl), hArg, Bip, Tic, , Htic, Dip, Sala, Aala, Thza, Thia, Bal, Fala, and Pala;
AA⁷ is absent or the D- or L-isomer of an amino acid selected from the group consisting of R¹¹, an optionally substituted aromatic α-amino acid, A3c, A4c, A5c, A6c, Abu, Aib, Aic, ß-Ala, Arg, Cha, Deg, Gaba, Ile, Leu, Nle, Pip, Pro, Sar, Ser, Ser(Bzl), Thr, Thr(Bzl), Val, Tic, Htic, Sala, Aala, Thza, Thia, Bal, Fala, Pala, hArg, Bip, Bpa, Dip, Pal, Sala, and X⁰-Phe;
AA^{7b} is absent or a D- or L-isomer of an amino acid selected from the group consisting of R¹¹, Bpa, Phe, F₅-Phe. X⁰-Phe, Nal, Pro, Ser, Ser(Bzl), Thr, Thr(Bzl), Trp, hArg, Bip, Tic, Htic, Dip, Sala, Aala, Thza, Thia, Bal, Fala, and Pala;
AA⁸ is absent or the D- or L- isomer of an amino acid selected from the group consisting of R¹¹, Maa, Maaab, Thr, Thr(Bzl), Ser, Ser(Bzl), Tyr, Phe(4-O-Bzl), F₅-Phe, and X⁵-Phe, and an optionally substituted aromatic α-amino acid;
R¹ and R² each is, independently, H, E-, E(O)₂S-, E(O)C-, EOOC-, R¹³, or absent;
R³ and R⁴ each is, independently, (C₁₋₁₂)alkyl, (C₂₋₁₂)alkenyl, (C₂₋₁₂)alkynyl, phenyl, naphthyl, phenyl-(C₁₋₆)alkyl, phenyl-(C₂₋₆)alkenyl, phenyl-(C₂₋₆)alkynyl, naphthyl-(C₁₋₆)alkyl, naphthyl-(C₂₋₆)alkenyl, naphthyl-(C₂₋₆)alkynyl, (cyclo(C₃₋₇)alkyl)-(C₁₋₆)alkyl, (cyclo(C₃₋₇)alkyl)-(C₂₋₆)alkenyl, (cyclo(C₃₋₇)alkyl)-(C₂₋₆)alkynyl, heterocyclyl-(C₁₋₄)alkyl, heterocyclyl-(C₂₋₄)alkenyl, heterocyclyl-(C_{2- 4})alkynyl, 1-adamantyl, 2-adamantyl, 9-fluorenylmethyl, dicyclopropylmethyl, dimethylcyclopropylmethyl, or benzhydryl;
R⁵ is -OR⁶, -NR⁷R⁸, or absent,
wherein each R⁶, R⁷ and R⁸ is, independently, H, (C₁₋₁₂)alkyl, C₂₋₁₂)alkenyl, (C₂₋₁₂)alkynyl, phenyl, naphthyl, phenyl-(C₁₋₆)alkyl, phenyl-(C₂₋₆)alkenyl, phenyl-(C₂₋₆)alkynyl, naphthyl-(C₁₋₆)alkyl, naphthyl-(C₂₋₆)alkenyl, naphthyl-(C₂₋₆)alkynyl, 1-adamantyl, 2-adamantyl, 9-fluorenylmethyl, dicyclopropylmethyl, dimethylcyclopropylmethyl, or benzhydryl;
R⁹ and R¹⁰ each is, independently, H, (C₁₋₆)alkyl, (C₃₋₄)alkenyl, (C₃₋₄)alkynyl, 1-adamantyl, or 2-adamantyl;
R¹¹ is, independently for each occurrence, a D- or L-amino acid of the formula: 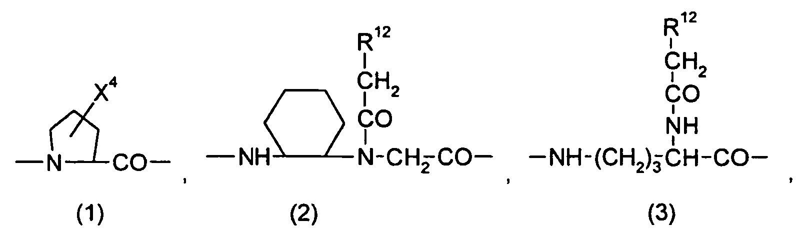 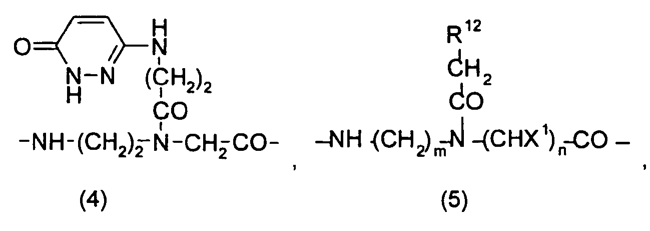 or 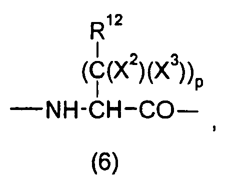 wherein m and n each is, independently, 1, 2, or 3, and p is 0, 1, or 2;
R¹² is, independently for each occurrence, an optionally substituted moiety of the formula: 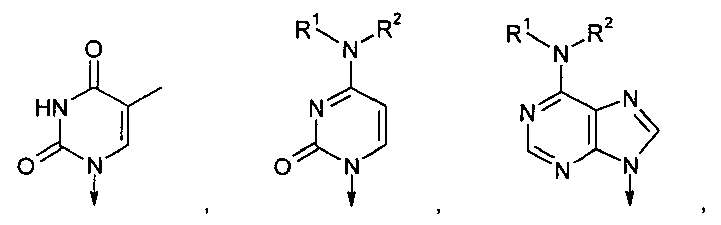 or 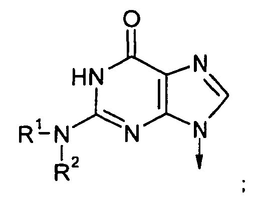 R¹³ is a moiety of the formula 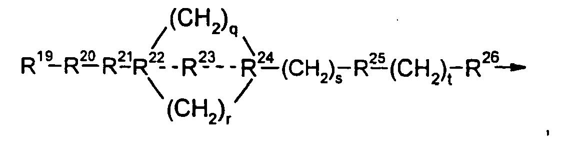 wherein q, r, s, and t each is, independently, 0, 1, 2, 3, 4 or 5;
R¹⁹ is absent, H, NH₂, OH, (C₁₋₆)hydroxyalkyl, N(R²⁷R²⁸), SO₃H, or an optionally substituted moiety selected from the group consisting of heterocyclyl, phenyl and naphthyl,
wherein the optionally substituted moiety defined for R¹⁹ is optionally substituted with one or more substituents selected, independently for each occurrence, from the group consisting of halogen, NO₂, OH, (C₁₋₆)alkyl, (C₂₋₆)alkenyl, (C₂₋₆)alkynyl, (C₁₋₆)alkoxy, NH₂, mono- or di-(C₁₋₆)alkylamino, Bzl, and O-Bzl;
R²⁰ is O or absent;
R²¹ is (C₁₋₆)alkyl or absent;
R²² is N, O, C, or CH;
R²³ is (C₁₋₆)alkyl or absent,
R²⁴ is N, CH, or C;
R²⁵ is NH, O, or absent;
R²⁶ is SO₂, CO, or CH;
R²⁷ and R²⁸ each is, independently, H or (C₁₋₆)alkyl;
E is, independently for each occurrence, an optionally substituted moiety selected from the group consisting of (C₁₋₁₂)alkyl, (C₂₋₁₂)alkenyl, (C₂₋₁₂)alkynyl, phenyl, naphthyl, phenyl-(C₁₋₆)alkyl, phenyl-(C₂₋₆)alkenyl, phenyl-(C₂₋₆)alkynyl, naphthyl-(C₁₋₆)alkyl, naphthyl-(C₂₋₆)alkenyl, naphthyl-(C₂₋₆)alkynyl, (cyclo(C₃₋₇)alkyl)-(C₁₋₆)alkyl, (cyclo(C₃₋₇)alkyl)-(C₂₋₆)alkenyl, (cyclo(C₃₋₇)alkyl)-(C₂₋₆)alkynyl, heterocyclyl-(C₁₋₄)alkyl, heterocyclyl-(C₂₋₄)alkenyl, heterocyclyl-(C₂₋₄)alkynyl, 1-adamantyl, 2-adamantyl, dicyclopropylmethyl, dimethylcyclopropylmethyl, 9-fluorenylmethyl, and benzhydryl;
wherein the optionally substituted moiety defined for E is optionally substituted with one or more substituents each independently selected from the group consisting of halogen, OH, Bzl, O-Bzl, NO₂, CN, COOH, and SH;
X⁰ is halogen, NO₂, OH, (C₁₋₆)alkyl, (C₁₋₆)alkoxy, mono- or di-(C₁₋₆)alkylamino, Bzl, O-Bzl, NR⁹R¹⁰, or CN;
X¹ is H, (C₁₋₆)alkyl, (C₂₋₆)alkenyl, (C₂₋₆)alkynyl, indolyl, imidazolyl, 1-naphthyl, 3-pyridyl, optionally ring-substituted benzyl, or a moiety which corresponds to the side-chain group of Arg, Leu, Gln, Lys, Tyr, His, Thr, Trp, Phe, Val, Ala, Lys, or His;
wherein said optionally ring-substituted benzyl is optionally substituted with one or more substituents selected from the group consisting of halogen, OH, (C₁₋₆)alkoxy, mono- or di-(C₁₋₆)alkylamino, (C₁₋₄) alkyl, (C₂₋₄) alkenyl, (C₂₋₄) alkynyl, and NR⁹R¹⁰;
X² and X³ each is, independently, H, halogen, OH, =O, =S, (C₁₋₁₂)alkyl, (C₂₋₁₂)alkenyl, (C₂₋₁₂)alkynyl, phenyl, naphthyl, phenyl-(C₁₋₆)alkyl, phenyl-(C₂₋₆)alkenyl, phenyl-(C₂₋₆)alkynyl, naphthyl-(C₁₋₆)alkyl, naphthyl-(C₂₋₆)alkenyl, naphthyl-(C₂₋₆)alkynyl, (cyclo(C₃₋₇)alkyl)-(C₁₋₆)alkyl, (cyclo(C₃₋₇)alkyl)-(C₂₋₆)alkenyl, (cyclo(C₃₋₇)alkyl)-(C₂₋₆)alkynyl, heterocyclyl-(C₁₋₄)alkyl, heterocyclyl-(C₂₋₄)alkenyl, heterocyclyl-(C₂₋₄)alkynyl, 1-adamantyl, 2-adamantyl, dicyclopropylmethyl, or dimethylcyclopropyl methyl;
X⁴ is H, OH, or NH₂; and
X⁵ is halogen, NO₂, CH₃, OH, Bzl or O-Bzl;
provided that:
at least six amino acid residues are present;
when AA³ is a D- or L-isomer of an amino acid selected from the group consisting of Cys, hCys, Pen, Tpa, or Tmpa, and AA⁶ is a D- or L-isomer of an amino acid selected from the group consisting of Cys, hCys, Pen, Tpa, or Tmpa, then AA³ and AA⁶ are connected by a disulfide bond;
when AA¹ or AA³ is a D- or L-isomer of an amino acid selected from the group consisting of Mac or Macab, then AA⁸ is a D- or L-isomer of an amino acid selected from the group consisting of Maa and Maaab, and
when AA⁸ is a D- or L-isomer of an amino acid selected from the group consisting of Maa and Maaab, then AA¹ or AA³ is a D- or L-isomer of Mac or of Macab, and AA¹ or AA³ is connected by a disulfide bond with AA⁸;
AA² can be D- or L-Hca only when AA¹ is absent;
when one of R¹ or R² is E(O)₂S-, E(O)C-, EOOC-, or R¹³, the other is H;
when R⁵ is absent, then one of R¹ or R² is also absent, and the N-terminal amino acid and C-terminal amino acid together form an amide bond;
when one of X² or X³ is C=O or C=S, the other is absent; and
said compound of formula (I) is not of the formula:
D-Phe-Tyr-cyclo(D-Cys-D-Trp-Lys-Cys)-Abu-Thr-NH₂;
Ac-Phe-Tyr-cyclo(D-Cys-D-Trp-Lys-Cys)-Abu-Thr-NH₂;
L-4-NO₂-Phe-Tyr-cyclo(D-Cys-D-Trp-Lys-Cys)-Abu-Thr-NH₂;
Ac-L-4-NO₂-Phe-Tyr-cyclo(D-Cys-D-Trp-Lys-Cys)-Abu-Thr-NH₂;
Hca-Tyr-cyclo(D-Cys-D-Trp-Lys-Cys)-Abu-Thr-NH₂;
D-Dip-Tyr-cyclo(Cys-D-Trp-Lys-D-Cys)-Val-Nal-NH₂;
D-4-NO₂-Phe-Phe(4-O-Bzl)-cyclo(D-Cys-D-Trp-Lys-Cys)Cha-Nal-NH₂;
or
D-4-NO₂-Phe-cyclo(D-Cys-Phe(4-O-Bzl)-D-Trp-Lys-Cys)-Val-Tyr-NH₂.

2. A compound according to claim 1, wherein said compound is of formula (II): 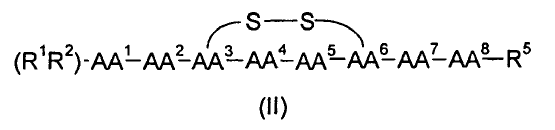 or a pharmaceutically acceptable salt thereof,
wherein
AA¹ is absent or the D- or L-isomer of an amino acid selected from the group consisting of R¹¹, Aac, Aic, Arg, Asn, Asp, Dip, Gln, Glu, Hyp, Lys, Mac, Macab, Orn, Pip, Pro, Ser, Ser(Bzl), Thr, Thr(Bzl), Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp and an optionally substituted aromatic α-amino acid,
wherein said optionally substituted aromatic α-amino acid is optionally substituted with one or more substituents selected from the group consisting of halogen, NO₂, OH, CN, (C₁₋₆)alkyl, (C₂₋₆)alkenyl, (C₂₋₆)alkynyl, and NR⁹R¹⁰;
AA² is absent or the D- or L-isomer of an amino acid selected from the group consisting of R¹¹, Aic, Arg, Hca, His, Hyp, Pal, F₅-Phe, Phe, Pro, Trp, X⁰-Phe, Pip, hArg, Bip, Bpa, Tic, Cmp, , Inc, Inp, Nip, Ppc, Htic, Thi, Tra, Cmpi, Tpr, ,
lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, and Pyp;AA³ is the D- or L-isomer of an amino acid selected from the group consisting of Cys, hCys, Pen, Tpa and Tmpa;
AA⁴ is a D- or L-isomer of an amino acid selected from the group consisting of Trp, N-Met-Trp, β-Met-Trp, His, hHis, hArg, Bip, Tic, , Htic, Dip, Sala, Aala, Thza, Thia, Bal, Fala, Pala, and an optionally substituted aromatic α-amino acid,
wherein said optionally substituted aromatic α-amino acid is optionally substituted with one or more substituents each independently selected from the group consisting of halogen, NO₂, OH, (C₁₋₄)alkyl, (C₂₋₄)alkenyl, (C₂₋₄)alkynyl, Bzl, O-Bzl, and NR⁹R¹⁰;
AA⁵ is a D- or L-isomer of an amino acid selected from the group consisting of 4-Pip-Gly, 4-Pip-Ala, *cis*-4-Acha, *trans*-4-Acha, *trans*-4-Amcha, hLys, Lys, Orn, hArg, Bip, Tic, , Htic, Dip, Sala, Aala, Thza, Thia, Bal, Fala, and Pala,
wherein the side-chain amino group of said amino acid is optionally mono- or di-substituted with R³ and R⁴;
AA⁶ is a D- or L-isomer of an amino acid selected from the group consisting of Cys, hCys, Pen, Tpa, and Tmpa;
AA⁷ is absent or a D- or L-isomer of an amino acid selected from the group consisting of R¹¹, Aic, A3c, A4c, A5c, A6c, Abu, Aib, ß-Ala, Arg, Bpa, Cha, Deg, Gaba, His, Ile, Leu, Nal, Nle, Pal, Phe, F₅-Phe, Pro, Sar, Ser, Ser(Bzl), Thr, Thr(Bzl), Trp, N-Me-Trp, Val, N-Me-Val, hArg, Bip, Tic, , Htic, Dip, Sala, Aala, Thza, Thia, Bal, Fala, Pala, and X⁰-Phe ;
AA⁸ is absent or the D- or L-isomer of an amino acid selected from the group consisting of R¹¹, an optionally substituted aromatic α-amino acid, Maa, Maaab, Ser, Ser(Bzl), Thr, Thr(Bzl), Tyr, Phe(4-O-Bzl), F₅-Phe, and X⁵-Phe; R¹³ is a moiety according to the formula 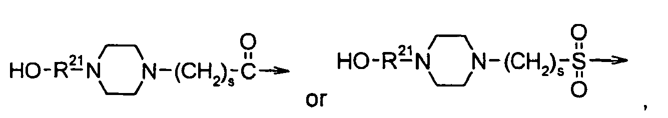 wherein R²¹ is (C₁₋₄)alkyl and s is 1, 2, 3, or 4; and
X⁰ is halogen, NO₂, CH₃, OH, Bzl, O-Bzl or CN;
provided that at least one of AA⁷ or AA⁸ is present.

3. A compound according to claim 1, wherein said compound is of formula (III): 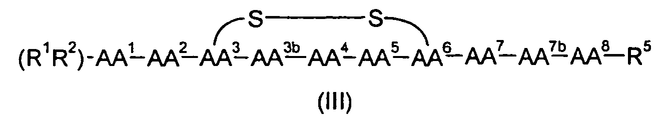 or a pharmaceutically acceptable salt thereof,
wherein
AA¹ is absent or the D- or L-isomer of an amino acid selected from the group consisting of R¹¹, Aac, Aic, Arg, Asn, Asp, Gln, Glu, Hca, His, Hyp, Lys, Mac, Macab, Orn, Pro, Ser, Ser(Bzl), Thr, Thr(Bzl), Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp and an optionally substituted aromatic α-amino acid,
wherein said optionally substituted aromatic α-amino acid is optionally substituted with one or more substituents selected from the group consisting of halogen, NO₂, OH, CN, (C₁₋₆)alkyl, (C₂₋₆)alkenyl, (C₂₋₆)alkynyl, and NR⁹R¹⁰;
AA³ is a D- or L-isomer of an amino acid selected from the group consisting of Cys, hCys, Pen, Tpa, and Tmpa;
AA^{3b} is the D- or L-isomer of an amino acid selected from the group consisting of R¹¹, Arg, Bpa, F₅-Phe, His, Nal, Pal, 4-Pal, Phe, Trp, hArg, Bip, Tic, , Htic, Dip, Sala, Aala, Thza, Thia, Bal, Fala, Pala, and X⁵-Phe;
AA⁴ is a D- or L-isomer of an amino acid selected from the group consisting of Trp, N-Met-Trp, β-Met-Trp, His, hHis, hArg, Bip, Tic, , Htic, Dip, Sala, Aala, Thza, Thia, Bal, Fala, Pala, and an optionally substituted aromatic α-amino acid;
wherein said optionally substituted aromatic α-amino acid is optionally substituted with one or more substituents each independently selected from the group consisting of halogen, NO₂, OH, CN, (C₁₋₄)alkyl, (C₂₋₄)alkenyl, (C₂₋₄)alkynyl, Bzl, O-Bzl, and NR⁹R¹⁰;
AA⁵ is a D- or L-isomer of an amino acid selected from the group consisting of 4-Pip-Gly, 4-Pip-Ala, *cis*-4-Acha, *trans*-4-Acha, *trans*-4-Amcha, hLys, Lys and Orn, and, hArg, Bip, Tic, , Htic, Dip, Sala, Aala, Thza, Thia, Bal, Fala, Pala,
wherein the side-chain amino group of said amino acid is optionally mono- or di-substituted with R³ and R⁴;
AA⁶ is a D- or L-isomer of an amino acid selected from the group consisting of Cys, hCys, Pen, Tpa, and Tmpa;
AA⁷ is absent or a D- or L-isomer of an amino acid selected from the group consisting of R¹¹, Aic, A3c, A4c, A5c, A6c, Abu, Aib, ß-Ala, Arg, Bpa, Cha, Deg, Gaba, His, Ile, Leu, Nal, Nle, Pal, Phe, F₅-Phe, Pro, Sar, Ser, Ser(Bzl), Thr, Thr(Bzl), Trp, N-Me-Trp, Val, N-Me-Val, hArg, Bip, Tic, , Htic, Dip, Sala, Aala, Thza, Thia, Bal, Fala, Pala, and X⁰-Phe;
X⁰ is halogen, NO₂, CH₃, OH, CN, Bzl or O-Bzl;
R¹ and R² each is, independently, H, E-, E(O)₂S-, E(O)C-, EOOC-, R¹³, or absent;
R⁵ is -OR⁶ or -NR⁷R⁸;
R¹³ is a moiety of the formula 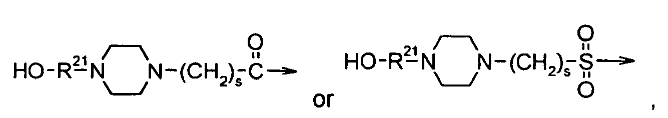 wherein R²¹ is (C₁₋₄)alkyl and s is 1, 2, 3, or 4;
provided that:
at least one of AA¹ or AA² is present;
when AA¹ is a D- or L-isomer of Pro, Hyp, Arg, Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp or His, AA² cannot be a D- or L-isomer of Pro, Hyp, Arg, Pip, hArg, Bip, Bpa, Tic, Cmp, , Inc, Inp, Nip, Ppc, Htic, Thi, Tra, Cmpi, Tpr, , lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp or His;
when AA⁷ is a D- or L-isomer of Thr or of Ser, AA⁸ cannot be a D- or L-isomer of Thr or of Ser;
at least one of AA¹, AA², AA^{3b}, AA⁷, AA^{7b}, or AA⁸ is the D- or L-isomer of R¹¹; and
when one of X² or X³ is =O or =S, the other is absent;
or a pharmaceutically acceptable salt thereof.

4. A compound according to claim 1, wherein said compound is of formula (IV): 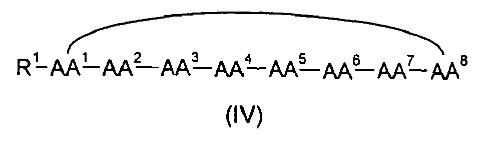 wherein
AA¹ is absent or the D- or L-isomer of an amino acid selected from the group consisting of R¹¹, Aic, Hyp, Pro, Ser, Ser(Bzl), Thr, Thr(Bzl), Tic, Htic, Fala and an optionally substituted aromatic α-amino acid;
wherein said optionally substituted aromatic α-amino acid is optionally substituted with one or more substituents each independently selected from the group consisting of halogen, NO₂, OH, CN, (C₁₋₆)alkyl, (C₂₋₆)alkenyl, (C₂₋₆)alkynyl, (C₁₋₆)alkoxy, Bzl, O-Bzl, and NR⁹R¹⁰;
AA² is absent or the D- or L-isomer of an amino acid selected from the group consisting of R¹¹, Arg, F₅-Phe, His, Pal, Phe, Trp, hArg, Pala, Bal, Fala, , Sala and X⁰-Phe;
AA³ is the D- or L-isomer of an optionally substituted aromatic α-amino acid, wherein said optionally substituted aromatic α-amino acid is optionally substituted with one or more substituents selected from the group consisting of halogen, NO₂, OH, CN, (C₁₋₄)alkyl, (C₂₋₄)alkenyl, (C₂₋₄)alkynyl, Bzl, O-Bzl, and NR⁹R¹⁰;
AA⁴ is a D- or L-isomer of an optionally substituted amino acid selected from the group consisting of Trp, N-Met-Trp, β-Me-Trp, Lys, Orn, hLys, cis-4-Acha, *trans*-4-Acha, *trans*-4-Amcha, 4-Pip-Gly, 4-Pip-Ala, hArg, Bip, Tic, Htic, Dip, Sala, Aala, Thza, Thia, Bal, Fala, and Pala;
wherein the side chain amino group of said optionally substituted amino acid is optionally substituted with R³ and R⁴;
AA⁵ is absent or a D- or L-isomer of R¹¹, A3c, A4c, A5c, A6c, Abu, Aib, Aic, ß-Ala, Bpa, Cha, Deg, F₅-Phe, Gaba, Ile, Leu, Nal, Nle, Pal, Phe, Pro, Sar, Ser, Ser(Bzl), Thr, Thr(Bzl), Trp, N-Me-Trp, Val, N-Me-Val, hArg, Bip, Tic, , Htic, Dip, Sala, Aala, Thza, Thia, Bal, Fala, Pala, or X⁰-Phe;
AA⁶ is absent, the D- or L-isomer of R¹¹, an aromatic α-amino acid, F₅-Phe, Phe, Thr, Thr(Bzl), Ser, Ser(Bzl), or X⁰-Phe;
AA⁷ is absent, the D- or L-isomer of R¹¹ or the D- or L-isomer of an aromatic α-amino acid;
AA⁸ is a D- or L- isomer of R¹¹;
R¹ is H, E-, E(O)₂S-, E(O)C-, EOOC-, or R¹³; R¹³ is a moiety of the formula 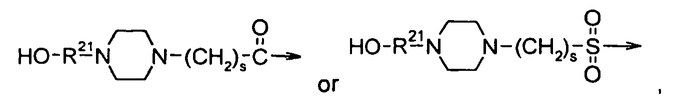 wherein R²¹ is (C₁₋₄)alkyl and s is 1, 2, 3, or 4;
X⁰ in the definition of AA² and AA⁵ is halogen, NO₂, OH, (C₁₋₆)alkyl, (C₁₋₆)alkoxy, mono- or di-(C₁₋₆)alkylamino, Bzl or O-Bzl;
X⁰ in the definition of AA⁶ is halogen, NO₂, OH, (C₁₋₆)alkyl, (C₁₋₆)alkoxy, mono- or di-(C₁₋₆)alkylamino, Bzl, O-Bzl, or NR⁹R¹⁰;
provided that:
at least one of AA¹ or AA² is present;
when AA¹ is absent, AA² and AA⁸ together form a bond; and
at least two of AA⁵, AA⁶, and AA⁷ are present;
or a pharmaceutically acceptable salt thereof.

5. A compound according to claim 2, wherein
AA¹ is absent, Ac-D-Phe, or the D- or L- isomer of R¹¹, Pip, Pro, or Ser, or of an aromatic α-amino acid selected from the group consisting of Cpa, Dip, Nal, Pal, and Phe; 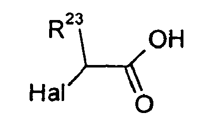 AA² is absent, Aic, Pal, Phe, F₅-Phe, 4-NO₂-Phe, Trp, Tyr, Phe(4-O-Bzl) 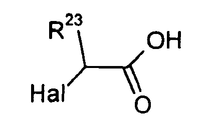 AA³ is the D- or L- isomer of an amino acid selected from the group consisting of Pen, Cys, hCys and Tmpa;
AA⁴ is the D- or L-isomer of Trp, His, N-Me-Trp, β-Me-Trp, hTrp, or hHis; AA⁵ is Lys, hLys, N-Me-Lys, Orn, cis-4-Acha or 4-Pip-Ala;
AA⁶ is the D- or L-isomer of an amino acid selected from the group consisting of Cys, hCys, Pen and Tmpa;
AA⁷ is A3c, A4c, A5c, A6c, Abu, Aic, β-Ala, Gaba, Nle, F₅-Phe, Phe, Pro, Sar, Ser, Thr, Thr(Bzl), Tyr, Val or absent; and
AA⁸ is R¹¹, Nal, Thr, Thr(Bzl), Tyr, Phe(4-O-Bzl), or absent;
or a pharmaceutically acceptable salt thereof.

6. A compound according to claim 5, wherein
AA' is absent or the D- or L- isomer of R¹¹, Pip or Pro, or of an aromatic α-amino acid selected from the group consisting of Cpa, Dip, Nal, Pal, Phe, and Ac-Phe;
AA² is Tyr, Pal, Phe, 4-NO₂-Phe, Trp, or absent;
AA³ is a D- or L-isomer of Cys or Pen;
AA⁴ is D-Trp;
AA⁵ is Lys, Orn, or cis-4-Acha;
AA⁶ is a D- or L-isomer of Cys or Pen;
AA⁷ is A3c, A4c, A5c, A6c, Abu, Aic, β-Ala, Gaba, Nle, Phe, Pro, Sar, Thr, Thr(Bzl), Tyr, Val, or absent; and
AA⁸ is R¹¹, Thr, Tyr, Nal, or absent;
or a pharmaceutically acceptable salt thereof.

7. A compound according to claim 3, wherein
AA¹ is R¹¹, Aic, Hca, Pro, Ser, Ser(Bzl), Trp, Tyr, or a D- or L-isomer of an aromatic α-amino acid selected from the group consisting of Cpa, Nal, Ac-Nal, Phe, Ac-Phe, 4-NO₂-Phe, and Ac-4-NO₂-Phe;
AA² is Pal, Phe, F₅-Phe, Tyr, or absent;
AA³ is a D- or L-isomer of Cys, hCys, Pen or Tmpa;
AA^{3b} is Pal, 4-Pal, His, Trp, Tyr, Phe(4-O-Bzl), Phe, or R¹¹;
AA⁴ is a D- or L-isomer of Trp or His;
AA⁵ is Lys, N-Me-Lys, Orn, hLys, cis-4-Acha, or 4-Pip-Ala;
AA⁶ is a D- or L-isomer of Cys, hCys, Pen or Tmpa;
AA⁷ is R¹¹, A4c, A5c, Abu, β-Ala, Gaba, Phe, F₅-Phe, Ser(Bzl), Thr, Thr(Bzl), Phe(4-O-Bzl), or absent;
AA^{7b} is R¹¹, Nal, F₅-Phe, X⁰-Phe or absent, wherein X⁰ is halogen, NO₂, CH₃, OH, Bzl or O-Bzl; and
AA⁸ is R¹¹, Nal, Tyr, Phe(4-O-Bzl), or absent;
or a pharmaceutically acceptable salt thereof.

8. A compound according to claim 7, wherein
AA¹ is R¹¹, Aic, Hca, Pro, Ser(Bzl), or a D- or L-isomer of an aromatic α-amino acid selected from the group consisting of Cpa, Nal, Ac-Nal, Phe, Ac-Phe, 4-NO₂-Phe, and Ac-4-NO₂-Phe;
AA² is Pal, Tyr, or absent;
AA³ is a D- or L-isomer of Cys or Pen;
AA^{3b} is R¹¹, Pal, 4-Pal, Trp, Tyr, Phe(4-O-Bzl), or Phe, wherein R¹¹ is (T)aeg; AA⁴ is D-Trp;
AA⁵ is Lys, N-Me-Lys, Orn, or cis-4-Acha;
AA⁶ is a D- or L-isomer of Cys or Pen;
AA⁷ is R¹¹, A5c, Abu, Ser(Bzl), Thr, Thr(Bzl), Phe(4-O-Bzl), Gaba, or absent; AA^{7b} is Nal, X⁰-Phe or absent; and
AA⁸ is Tyr or absent;
or a pharmaceutically acceptable salt thereof.

9. A compound according to claim 4, wherein
AA¹ is Aic, Hyp, Cpa, D-Cpa, Nal, Pal, Phe, Pro, R¹¹, Tyr or absent;
AA² is Phe, Trp, F₅-Phe, His, Tyr, Phe(4-O-Bzl), or R¹¹;
AA³ is a D-isomer of Trp, His, or Pal;
AA⁴ is Lys, N-Me-Lys, Orn, hLys, cis-4-Acha, or 4-Pip-Ala;
AA⁵ is Pal, Phe(4-O-Bzl), Thr(Bzl), Thr, Sar, Gaba, β-Ala, A4c, A5c, A6c, Abu, Aic or absent;
AA⁶ is Thr, Tyr, Ser, F₅-Phe, Cpa, Nal, or D- or L-Phe;
AA⁷ is Nal, Pal, or absent; and
AA⁸ is R¹¹;
or a pharmaceutically acceptable salt thereof.

10. A compound according to claim 9, wherein
AA¹ is Cpa, Nal, Pal, Phe, Tyr or absent;
AA² is Phe, Tyr, Trp, or R¹¹;
AA³ is D-Trp;
AA⁴ is Lys, N-Me-Lys, or cis-4-Acha;
AA⁵ is Pal, Phe(4-O-Bzl), Aic, Gaba, A5c or absent;
AA⁶ is Thr, Nal, or D- or L-Phe;
AA⁷ is absent; and
AA⁸ is R¹¹;
or a pharmaceutically acceptable salt thereof.

11. A compound according to claim 2, wherein R¹ and R⁵ are absent and the N-terminal amino acid and the C-terminal amino acid together form an amide bond; or a pharmaceutically acceptable salt thereof.

12. A compound according to claim 3, wherein R¹ and R⁵ are absent and the N-terminal amino acid and the C-terminal amino acid together form an amide bond; or a pharmaceutically acceptable salt thereof.

13. A compound according to claim 6, wherein said compound is of the formula:
Ac-D-Phe-Tyr-cyclo(D-Cys-D-Trp-Lys-Cys)-Abu-Thr-NH₂;
Nal-Tyr-cyclo (Cys-D-Trp-Lys-D-Cys)-Val-Nal-NH₂;
Nal-Tyr-cyclo(Cys-D-Trp-Lys-D-Cys)-Abu-Nal-NH₂;
D-Dip-Tyr-cyclo(Cys-D-Trp-Lys-D-Cys)-Abu-Nal-NH₂;
Dip-Tyr-cyclo(D-Cys-D-Trp-Lys-D-Cys)-Abu-Nal-NH₂;
Nal-Tyr-cyclo(D-Cys-D-Trp-Lys-D-Cys)-Abu-Nal-NH₂;
Dip-Tyr-cyclo(D-Cys-D-Trp-Lys-D-Cys)-Val-Nal-NH₂;
Nal-Tyr-cyclo(D-Cys-D- Trp-Lys-D-Cys)-Val-Nal-NH₂;
cyclo(D-Phe-Tyr-cyclo(D-Cys-D-Trp-Lys-Cys)-Abu-Thr);
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp-Lys-D-Cys)-A6c-Nal-NH₂;
(G(z))aeg-cyclo(D-Cys-D-Trp-Lys-D-Cys)-A5c-Nal-NH₂;
Pal-cyclo(D-Cys-D-Trp-Lys-D-Cys)-A5c-Nal-NH₂;
Cpa-Pal-cyclo(D-Cys-D-Trp-Lys-D-Cys)-β-Ala-Nal-NH₂;
Cpa-Pal-cyclo(D-Cys-D-Trp-Lys-D-Cys)-Sar-Nal-NH₂;
Cpa-Pal-cyclo(D-Cys-D-Trp-Lys-D-Cys)-Gaba-Nal-NH₂;
Cpa-Pal-cyclo(D-Cys-D-Trp-Lys-D-Cys)-Pro-Nal-NH₂;
Pro-Phe-c(D-Cys-D-Trp-Lys-D-Cys)-Nle-Phe-NH₂;
Pro-Phe-c(D-Cys-D-Trp-Lys-D-Cys)-Thr-Nle-NH₂;
Pro-Phe-c(D-Cys-D-Trp-Lys-D-Cys)-Thr-Phe-NH₂;
Cpa-Phe-c(D-Cys-D-Trp-Lys-D-Cys)-Gaba-NH₂.
Cpa-Phe-c(D-Cys-D-Trp-Lys-D-Cys)-Gaba-Tyr-NH₂;
Pip-Phe-c(D-Cys-D-Trp-Lys-D-Cys)-NH₂;
Pip-Phe-c(Cys-D-Trp-Lys-Cys)-Gaba-NH₂; or
Pro-Phe-c(D-Cys-D-Trp-Lys-D-Cys)-Thr-NH₂;
or a pharmaceutically acceptable salt thereof.

14. A compound according to claim 6, wherein said compound is according to the formula:
Phe-cyclo(Cys-D-Trp-Lys-Cys)-Thr-NH₂;
Phe-Tyr-cyclo(D-Cys-D-Trp-Lys-Cys)-Abu-Thr-NH₂;
Ac-D-Phe-Tyr-cyclo(D-Cys-D-Trp-Lys-Cys)-Abu-Thr-NH₂;
Nal-Tyr-cyclo(Cys-D-Trp-Lys-D-Cys)-Val-Nal-NH₂;
Nal-Tyr-cyclo(Cys-D-Trp-Lys-D-Cys)-Abu-Nal-NH₂;
Dip-Tyr-cyclo(D-Cys-D-Trp-Lys-D-Cys)-Abu-Nal-NH₂;
Nal-Tyr-cyclo(D-Cys-D-Trp-Lys-D-Cys)-Abu-Nal-NH₂ Dip-Tyr-cyclo(D-Cys-D-Trp-Lys-D-Cys)-Val-Nal-NH₂;
Nal-Tyr-cyclo(D-Cys-D-Trp-Lys-D-Cys)-Val-Nal-NH₂;
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp-Lys-D-Cys)-A6c-Nal-NH₂;
(G(z))aeg-cyclo(D-Cys-D-Trp-Lys-D-Cys)-A5c-Nal-NH₂;
D-Cpa-cyclo(Cys-D-Trp-Lys-D-Cys)-A5c-Nal-NH₂;
Pal-cyclo(D-Cys-D-Trp-Lys-D-Cys)-A5c-Nal-NH₂;
Cpa-cyclo(D-Cys-D-Trp-Lys-D-Cys)-A5c-Nal-NH₂;
Cpa-Pal-cyclo(D-Cys-D-Trp-Lys-D-Cys)-β-Ala-Nal-NH₂;
Cpa-Pal-cyclo(D-Cys-D-Trp-Lys-D-Cys)-Sar-Nal-N H₂;
Cpa-Pal-cyclo(D-Cys-D-Trp-Lys-D-Cys)-Alc-Nal-NH₂;
Cpa-Pal-cyclo(D-Cys-D-Trp-Lys-D-Cys)-Gaba-Nal-NH₂;
Cpa-Pal-cyclo(D-Cys-D-Trp-Lys-D-Cys)-Pro-Nal-NH₂;
(T)aeg-cyclo(D-Cys-D-Trp-Lys-D-Cys)-(A)aeg-NH₂;
Cpa-Pal-cyclo(D-Cys-D-Trp-Lys-D-Cys)-A4c-Nal-NH₂;
Cpa-Pal-cyclo(D-Cys-D-Trp-Lys-D-Cys)-Nal-NH₂;
Pal-cyclo(D-Cys-D-Trp-Lys-D-Cys)-Nal-NH₂;
Pro-Phe-cyclo(Cys-D-Trp-Lys-D-Cys)-Val-NH₂;
Pro-Phe-cyclo(D-Cys-D-Trp-Lys-Cys)-Val-NH₂;
Pip-4-NO2-Phe-cyclo(D-Cys-D-Trp-Lys-D-Cys)-Nle-N H₂;
(G)aeg-Pal-cyclo(D-Cys-D-Trp-Lys-D-Cys)-Thr(Bzl)-(C)aeg-NH₂; or
(C)aeg-Pal-cyclo(D-Cys-D-Trp-Lys-D-Cys)-Thr(Bzl)-(G)aeg-NH₂;
or a pharmaceutically acceptable salt thereof.

15. A compound according to claim 8, wherein said compound is according to the formula
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂;
D-Phe-cyclo(Cys-Tyr-D-Trp-Lys-Cys)-Thr-NH₂;
D-4-NO₂-Phe-cyclo(D-Cys-Tyr-D-Trp-Lys-Cys)-Nal-NH₂;
Ac-D-4-NO₂-Phe-cyclo(D-Cys-Tyr-D-Trp-Lys-Cys)-Nal-NH₂;
D-4-NO₂-Phe-Pal-cyclo(D-Cys-Phe(4-O-Bzl)-D-Trp-Lys-Cys)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys)-Thr-Tyr-NH₂;
D-4-NO₂-Phe-cyclo(D-Cys-Pal-D-Trp-Lys-Cys)-Thr(Bzl)-NH₂;
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Cys-Pal-D-Trp-Lys-Cys)-Thr(Bzl)-Tyr-NH₂,
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NH₂;
D-Cpa-cyclo(D-Cys-(T)aeg-D-Trp-Lys-Cys)-Thr(Bzl)-Tyr-NH₂;
(C)aeg-c(D-Cys-Pal-D-Trp-Lys-D-Cys)-Thr(Bzl)-Tyr-NH₂;
D-Cpa-c(D-Cys-Pal-D-Trp-Lys-D-Cys)Thr(Bzl)-Tyr-NH₂;
(T)aeg-c(Pen-Pal-D-Trp-Lys-D-Cys)Thr(Bzl)-Tyr-NH₂;
(T)aeg-c(D-Cys-Trp-D-Trp-Lys-D-Cys)Thr(Bzl)-Tyr-NH₂;
(T)aeg-c(D-Cys-Phe-D-Trp-Lys-D-Cys)Thr(Bzl)-Tyr-NH₂;
(T)aeg-c(D-Cys-Pa)-D-Trp-Orn-D-Cys)Thr(Bz))-Tyr-NH₂.
(T)aeg-c(D-Cys-Pa)-D-Trp-hLys-D-Cys)Thr(Bz))-Tyr-NH₂;
(T)aeg-c(D-Cys-Pa)-D-Trp-!amp-D-Cys)Thr(Bz))-Tyr-NH₂;
(T)aeg-c(D-Cys-Pal-D-Trp-Cha(4-am)-D-Cys)Thr(Bzl)-Tyr-NH₂;
(T)aeg-c(D-Cys-Pal-D-Trp-Lys-D-Cys)-Ser(Bzl)-Tyr-NH₂;
(T)aeg-c(D-Cys-Pa)-D-Trp-Lys-D-Cys)Thr(Bzl)-D-Tyr-NH₂;
(T)aeg-c(D-Cys-Pal-D-Trp-Lys-D-Cys)Thr(Bzl)-Trp-NH₂;
(T)aeg-c(D-Cys-Pal-D-Trp-Lys-D-Pen)Thr(Bzl)-Tyr-NH₂;
(C)aeg-c(D-Cys-Phe-D-Trp-Lys-D-Cys)Thr(Bzt)-Tyr-NH₂:
Ina-c(D-Cys-Phe-D-Trp-Lys-D-Cys)-Thr(Bzl)-Tyr-NH₂;
Mnf-c(D-Cys-Phe-D-Trp-Lys-D-Cys)-Thr(Bzl)-Tyr-NH₂;
Inp-c(D-Cys-Phe-D-Trp-Lys-D-Cys)-Thr(Bzl)-Tyr-NH₂;
Nua-c(D-Cys-Phe-D-Trp-Lys-D-Cys)-Thr(Bzl)-Tyr-NH₂;
(T)aeg-Pal-c(D-Cys-D-Trp-Lys-D-Cys)Thr(Bzl)-Tyr-NH₂;
(T)aeg-Pal-c(D-Cys-D-Trp-Lys-D-Cys)Tyr(Bzl)-Thr-NH₂;
(C)aeg-Phe-c(D-Cys-D-Trp-Lys-D-Cys)Thr(Bzl)-Tyr-NH₂; or
(T)aeg-D-Trp-c(D-Cys-Pal-Lys-D-Cys)Thr(Bzl)-Leu-NH₂;
or a pharmaceutically acceptable salt thereof.

16. A compound according to claim 8, wherein said compound is according to the formula
Hca-cyclo(D-Cys-Tyr-D-Trp-Lys-Cys)-Nal-NH₂;
Ac-Nal-cyclo(D-Cys-Tyr-D-Trp-Lys-Cys)-Nal-NH₂;
Ac-D-Phe-cyclo(D-Cys-Tyr-D-Trp-Lys-Cys)-Nal-NH₂;
Ac-D-Nal-cyclo(D-Cys-Tyr-D-Trp-Lys-Cys)-Nal-NH₂;
D-Phe-cyclo(D-Cys-Tyr-D-Trp-Lys-Cys)-Nal-NH₂;
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂;
D-Phe-cyclo(Cys-Tyr-D-Trp-Lys-Cys)-Thr-NH₂;
D-4-NO₂-Phe-cyclo(D-Cys-Tyr-D-Trp-Lys-Cys)-Nal-NH₂;
Ac-D-4-NO₂-Phe-cyclo(D-Cys-Tyr-D-Trp-Lys-Cys)-Nal-NH₂;
D-4-NO₂-Phe-Pal-cyclo(D-Cys-Phe(4-O-Bzl)-D-Trp-Lys-Cys)-Tyr-NH₂;
D-4-NO₂₋Phe-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Tyr-NH₂;
D-4-NO₂₋Phe-cyclo(D-Cys-Pal-D-Trp-Lys-Cys)-Thr(Bzl)-NH₂;
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Cys-Pal-D-Trp-Lys-Cys)-Thr(Bzl)-Tyr-NH₂;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-Cys)-Thr(Bzl)-Tyr-NH₂;
(C)aeg-cyclo(D-Cys-Pal-D-Trp-Lys-Cys)-Thr(Bzl)-Tyr-NH₂;
Aic-cyclo(D-Cys-Pal-D-Trp-Lys-Cys)-Thr(Bzl)-Tyr-NH₂;
(C(z))aeg-cyclo(D-Cys-Pal-D-Trp-Lys-Cys)-Thr(Bzl)-Tyr-NH₂;
(A(z))aeg-cyclo(D-Cys-Pal-D-Trp-Lys-Cys)-Thr(Bzl)-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NHz;
D-Cpa-cyclo(D-Cys-(T)aeg-D-Trp-Lys-Cys)-Thr(Bzl)-Tyr-NH₂;
(T)aeg-cyclo(D-Cys-Pal-D-Trp-Lys-D-Cys)-Thr(Bzl)-p-Me-Phe-NH₂;
Ac-(T)aeg-cyclo(D-Cys-Pal-D-Trp-Lys-D-Cys)-Thr(Bzl)-Tyr-NH₂;
(T)aeg-cyclo(D-Cys-Pal-D-Trp-Lys-D-Cys)-Nal-NH₂;
D-Cpa-cyclo(D-Cys-Pal-D-Trp-Lys-D-Cys)-Nal-NH₂;
(A)aeg-cyclo(D-Cys-Pal-D-Trp-Lys-D-Cys)-Thr(Bzl)-Tyr-NH₂; (C)aeg-cyclo(D-Cys-Pal-D-Trp-Lys-D-Cys)-Thr(Bzl)-Tyr-NH₂;
(C)aeg-c(D-Cys-Pal-D-Trp-Lys-D-Cys)-Thr(Bzl)-Tyr-NH₂:
D-Cpa-c(D-Cys-Pal-D-Trp-Lys-D-Cys)Thr(Bzl)-Tyr-NH₂;
(T)aeg-c(Pen-Pal-D-Trp-Lys-D-Cys)Thr(Bzl)-Tyr-NH₂;
(T)aeg-c(D-Cys-Trp-D-Trp-Lys-D-Cys)Thr(Bzl)-Tyr-NH₂,
(T)aeg-c(D-Cys-Phe-D-Trp-Lys-D-Cys)Thr(Bzl)-Tyr-NH₂;
(T)aeg-c(D-Cys-Pal-D-Trp-Orn-D-Cys)Thr(Bzl)-Tyr-NH₂;
(T)aeg-c(D-Cys-Pal-D-Trp-hLys-D-Cys)Thr(Bzl)-Tyr-NH₂;
(T)aeg-c(D-Cys-Pal-D-Trp-lamp-D-Cys)Thr(Bzl)-Tyr-NH₂;
(T)aeg-c(D-Cys-Pal-D-Trp-Cha(4-am)-D-Cys)Thr(Bzl)-Tyr-NH₂;
(T)aeg-c(D-Cys-Pa)-D-Trp-Lys-D-Cys)-Ser(Bzl)-Tyr-NH₂:
(T)aeg-c(D-Cys-Pal-D-Trp-Lys-D-Cys)Thr(Bzl)-D-Tyr-NH₂;
(T)aeg-c(D-Cys-Pal-D-Trp-Lys-D-Cys)Thr(Bzl)-Trp-NH₂;
(T)aeg-c(D-Cys-Pal-D-Trp-Lys-D-Pen)Thr(Bzl)-Tyr-NH₂:
(C)aeg-c(D-Cys-Phe-D-Trp-Lys-D-Cys)Thr(Bzl)-Tyr-NH₂;
Ina-c(D-Cys-Phe-D-Trp-Lys-D-Cys)-Thr(Bzl)-Tyr-NH₂;
Mnf-c(D-Cys-Phe-D-Trp-Lys-D-Cys)-Thr(Bzl)-Tyr-NH₂;
Inp-c(D-Cys-Phe-D-Trp-Lys-D-Cys)-Thr(Bzl)-Tyr-NH₂;
Nua-c(D-Cys-Phe-D-Trp-Lys-D-Cys)-Thr(Bzl)-Tyr-NH₂;
(T)aeg-Pal-c(D-Cys-D-Trp-Lys-D-Cys)Thr(Bzl)-Tyr-NH₂;
(T)aeg-Pal-c(D-Cys-D-Trp-Lys-D-Cys)Tyr(Bzl)-Thr-NH₂;
(C)aeg-Phe-c(D-Cys-D-Trp-Lys-D-Cys)Thr(Bzl)-Tyr-NH₂; or
(T)aeg-D-Trp-c(D-Cys-Pal-Lys-D-Cys)Thr(Bzl)-Leu-NH₂;
or a pharmaceutically acceptable salt thereof.

17. A compound according to claim 10, wherein said compound is according to the formula
cyclo(Trp-D-Trp-Lys-Phe(4-O-Bzl)-Phe-(T)aeg);
cyclo(Trp-D-Trp-Lys-Pal-Phe -(T)aeg); or
cyclo(Phe-Phe-D-Trp-Lys-Thr-(T)aeg);
or a pharmaceutically acceptable salt thereof.

18. Use of a compound according to claim 13 or a pharmaceutically acceptable salt thereof in the manufacture of a medicament for eliciting a neuromedin B receptor agonist effect in a subject in need thereof.

19. Use of a compound according to claim 14 or a pharmaceutically acceptable salt thereof in the manufacture of a medicament for eliciting a somatostatin receptor agonist effect in a subject in need thereof.

20. Use of a compound according to claim 15 or a pharmaceutically acceptable salt thereof in the manufacture of a medicament for eliciting a neuromedin B receptor agonist effect in a subject in need thereof.

21. Use of a compound according to claim 16 or a pharmaceutically acceptable salt thereof in the manufacture of a medicament for eliciting a somatostatin receptor agonist effect in a subject in need thereof.

22. Use of a compound according to claim 17 or a pharmaceutically acceptable salt thereof in the manufacture of a medicament for eliciting a somatostatin receptor agonist effect in a subject in need thereof, provided said compound is not
Cycle(Trp-D-Trp-Lys-Phe(4-O-Bzl)-Phe-(T) aeg); or
Cyclo(Trp-D-Trp-Lys-Pal-Phe-(T) aeg).

23. Use of a compound according to claim 14 or a pharmaceutically acceptable salt thereof in the manufacture of a medicament for eliciting a SSTR-1 agonist effect in a subject in need thereof, provided said compound is not
Nal-Tyr-cyclo(Cys-D-Trp-Lys-O-Cys)-Val-Nal-NH₂;
Nal-Tyr-cyclo(Cys-D-Trp-Lys-D-Cys)-Abu-Nal-NH₂;
Dip-Tyr-cyclo(D-Cys-D-Tr₃-Lys-D-Cys)-Abu-Nal-NH₂;
Nal-Tyr-cyclo(D-Cys-D-T-Lys-D-Cys)-Abu-Nal-NH₂;
Dip-Tyr-cyclo(D-Cys-D-T-Lys-D-Cys)-Val-Nal-NH₂;
Nal-Tyr-cyclo(D-Cys-D-Trp-Lys-D-Cys)-Val-Nal-NH₂;
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp Lys-D-Cys)-A6c-Nal-NH₂;
(G(z))aeg-cyclo(D-Cys-D-Trp-Lys-D-Cys)-A5c-Nal-NH₂;
D-Cpa-cyclo(Cys-D-Trp-Lys-D-Cys)-A5c-Nal-NH₂;
Pal-cyclo(D-Cys-D-Trp-Lys-D-Cys)-A5c-Nal-NH₂;
Cpa-cyclo(D-Cys-D-Trp-Lys-D-Cys)-A5c-Nal-NH₂;
Cpa-Pal-cyclo(D-Cys-D-Trp-Lys-D-Cys)-β-Ala-Nal-NH₂;
cyclo(D-Cys-Lys-D-Trp-D-Cyr)-A5c-Nal-NH₂;
Cpa-Pal-cyclo(D-Cys-D-Trp-Lys-D-Cys)-Sar-Nal-NH₂;
Cpa-Pal-cyclo(D-Cys-D-Trp-Lys-D-Cys)-Aic-Nal-NH₂;
Cpa-Pal-cyclo(D-Cys-D-Trp-Lys-D-Cys)-Gaba-Nal-NH₂; or
Cpa-Pal-cyclo(D-Cys-D-Trp-Lys-D-Cys)-Pro-Nal-NH₂.

24. Use of a compound according to claim 16, or a pharmaceutically acceptable salt thereof in the manufacture of a medicament for eliciting a SSTR-1 agonist effect in a subject in need thereof, provided said compound is not
Ac-D-Phe-cyclo(D-Cys-Tyr-D-Trp-Lys-Gys)-Nal-NH₂;
Ac-D-Nal-cyclo(D-Cys-Tyr-D-Trp-Lys-Cys)-Nal-NH₂;
D-Phe-cyclo(D-Cys-Tyr-D-Trp-Lys-Cys)-Nal-NH₂;
Nal-cyclo(D-Cys-Tyr-D-Tr Lys-Cys)-Nal-NH₂;
D-Nal-cyclo(D-Cys-Tyr-D-Tyr-Lys-Cys)-Nal-NH₂;
D-4-NO₂-Phe-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys) - Thr(Bzl)-NH₂;
D-4-NO₂-Phe-cyclo(D-Cys-Pal-D-Trp-Lys-D-Cys) - Thr(Bzl)-Tyr-NH₂:
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-Trp-Lys-Cys)-Thr(Bzl)-Tyr-NH₂;
D-Nal-Cyclo(D-Cys-Pal-D-Tzp-Lys-Cy₅)-Thr(Bzl)-Tyr-NH₂;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-Cys)-Thr(Bzl) Tyr-NH₂;
(C)aeg-cyclo(D-Cys-Pal-D-Trp-Lys-Cys)-Thr(Bzl)-Tyr-NH₂;
Aic-cyclo(D-Cys-Pal-D-Trp-Lys-Cys)-Thr(Bzl)-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(O-Cys-tyr-D- rp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D- rp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NH₂; or
D-Cpa-cyclo(D-Cys-(T)aeg-D-Trp-Lys-Cys)-Thr (Bzl)-Tyr-NH₂;

25. A pharmaceutical composition comprising an effective amount of a compound according to claim 1 or a pharmaceutically acceptable salt thereof and a pharmaceutically acceptable carrier.

26. Use of a compound according to claim 1 in the manufacture of a medicament for the treatment of a disease selected from lung cancer, glioma, anorexia, hypothyroidism, hyperaldosteronism, H. pylori proliferation, acromegaly, restenosis, Crohn's disease, systemic sclerosis, external and internal pancreatic pseudocysts and ascites, VIPoma, nesidoblastosis, hyperinsulinism, gastrinoma, Zollinger-Ellison Syndrome, diarrhea, AIDS related diarrhea, chemotherapy related diarrhea, scleroderma, Irritable Bowel Syndrome, pancreatitis, small bowel obstruction, gastroesophageal reflux, duodenogastric reflux, Cushing's Syndrome, gonadotropinoma, hyperparathyroidism, Graves' Disease, diabetic neuropathy, Paget's disease, polycystic ovary disease, thyroid cancer, hepatome, leukemia, meningioma, cancer cachexia, orthostatic hypotension, postprandial hypotension, panic attacks GH secreting adenomas, Acromegaly, TSH secreting adenomas, prolactin secreting adenomas, insulinoma, glucagonoma, diabetes mellitus, hyperlipidemia, insulin insensitivity, Syndrome X, angiopathy, proliferative retinopathy, dawn phenomenon, Nephropathy, gastric acid secretion, peptic ulcers, enterocutaneous fistula, pancreaticocutaneous fistula, Dumping syndrome, watery diarrhea syndrome, pancreatitis, gastrointestinal hormone secreting tumor, angiogenesis, arthiritis, allograft rejection, graft vessel bleeding, portal hypertension, gastrointestinal bleeding, obesity, and opioid overdose.

## Patentansprüche

1. Verbindung der Formel (I):
(R¹R²)-AA¹-AA²-AA³ -AA^{3b}-AA⁴-AA⁵-AA⁶-AA⁷-AA^{7b}-AA⁸-R⁵ (I)
oder ein pharmazeutisch annehmbares Salz davon, worin
der α-Stickstoff von AA¹, AA², AA³, AA^{3b}, AA⁴, AA⁵, AA⁶, AA⁷, AA^{7b} und AA⁸ jeweils unabhängig voneinander gegebenenfalls substituiert ist mit (C₁₋₄)-Alkyl, (C₃₋₄)-Alkenyl, (C₃₋₄)-Alkinyl oder (C₁₋₆)-Alkyl-C(O)-;
AA¹ nicht vorhanden ist oder das D- oder L-Isomer einer Aminosäure ist, gewählt aus der Gruppe bestehend aus R¹¹, Aac, Aic, Arg, Asn, Asp, Dip, Gln, Glu, Hca, Hyp, Lys, Mac, Macab, Orn, Pro, Ser, Ser(Bzl), Thr, Thr(Bzl), Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, C4c, 5-Iqs, Htqa, 4-Mqc, Thn, α-Chpa, Cit, Nua, Pyp und einer gegebenenfalls substituierten aromatischen α-Aminosäure;
worin die gegebenenfalls substituierte aromatische α-Aminosäure gegebenenfalls substituiert ist mit einem oder mehreren Substituenten, jeweils unabhängig voneinander gewählt aus der Gruppe bestehend aus Halogen, NO₂, OH, CN, (C₁₋₆)-Alkyl, (C₂₋₆)-Alkenyl, (C₂₋₆)-Alkinyl, (C₁₋₆)-Alkoxy, Bzl, O-Bzl und NR⁹R¹⁰;
AA² nicht vorhanden ist oder das D- oder das L-Isomer einer Aminosäure ist, gewählt aus der Gruppe bestehend aus R¹¹, Aic, Arg, Hca, His, Hyp, Pal, F₅-Phe, Phe, Pro, Trp und X⁰-Phe Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua und Pyp;
AA³ das D- oder L-Isomer einer Aminosäure ist, gewählt aus der Gruppe bestehend aus Cys, hCys, Pen, Tpa, Tmpa, Mac, Macab und einer gegebenenfalls substituierten aromatischen α-Aminosäure;
worin die gegebenenfalls substituierte aromatische α-Aminosäure gegebenenfalls substituiert ist mit einem oder mehreren Substituenten, gewählt aus der Gruppe bestehend aus Halogen, NO₂, OH, CN, (C₁₋₄)-Alkyl, (C₂-₄)-Alkenyl, (C₂₋₄)-Alkinyl, (C₁₋₄)-Alkoxy, Bzl, O-Bzl und NR⁹R¹⁰, Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua und Pyp; AA^{3b} nicht vorhanden ist oder das D- oder L-Isomer einer Aminosäure ist, gewählt aus der Gruppe bestehend aus Pal, 4-Pal, His, Arg, Nal, Trp, Bpa, F₅-Phe, Phe, X⁰-Phe, R¹¹, hArg, Bip, Tic, Htic, Dip, Sala, Aala, Thza, Thia, Bal, Fala und Pala;
AA⁴ ein D- oder L-Isomer einer gegebenenfalls substituierten Aminosäure oder einer gegebenenfalls substituierten aromatischen α-Aminosäure ist;
worin die gegebenenfalls substituierte Aminosäure gewählt ist aus der Gruppe bestehend aus Trp, Lys, Orn, hLys, cis-4-Acha, trans-4-Acha, trans-4Amcha, 4-Pip-Gly, N-Met-Trp, β-Met-Trp, His, hHis, hArg, Bip, Tic, Htic, Dip, Sala, Aala, Thza, Thia, Bal, Fala, Pala und 4-Pip-Ala;
worin die Seitenketten-Aminogruppe der gegebenenfalls substituierten Aminosäure gegebenenfalls substituiert ist mit R³ und R⁴; und
worin die gegebenenfalls substituierte aromatische α-Aminosäure gegebenenfalls substituiert ist mit einem oder mehreren Substituenten, jeweils unabhängig voneinander gewählt aus der Gruppe bestehend aus Halogen, NO₂, OH, CN, (C₁₋₄)-Alkyl, (C₂₋₄)-Alkenyl, (C₂₋₄)-Alkinyl, Bzl, 0-Bzl und NR⁹R¹⁰;
AA⁵ ist nicht vorhanden, R¹¹, Aic, A3c, A4c, A5c, A6c, Abu, Aib, β-Ala, Bpa, Cha, Deg, Gaba, Ile, Leu, Nal, Nle, Pro, Sar, Ser, Ser(Bzl), Thr, Thr(Bzl), Trp, Val, Pal, F⁵-Phe, Phe, X⁰-Phe oder ein gegebenenfalls substituiertes D- oder L-Isomer einer Aminosäure, gewählt aus der Gruppe bestehend aus 4-Pip-Gly, 4-Pip-Ala, cis-4-Acha, trans-4-Acha, trans-4-Amcha, hLys, Lys, Orn, hArg, Bip, Tic, Htic, Dip, Sala, Aala, Thza, Thia, Bal, Fala und Pala; worin die Seitenketten-Aminogruppe der gegebenenfalls substituierten Aminosäure gegebenenfalls mono- oder disubstituiert ist mit R³ und R⁴;
AA⁶ nicht vorhanden ist oder das D- oder L-Isomer einer Aminosäure ist, gewählt aus der Gruppe bestehend aus R¹¹, einer gegebenenfalls substituierten aromatischen α-Aminosäure, Cys, hCys, Pen, Tpa, Tmpa, Thr, Thr(Bzl), Ser, Ser(Bzl), hArg, Bip, Tic, Htic, Dip, Sala, Aala, Thza, Thia, Bal, Fala und Pala;
AA⁷ nicht vorhanden ist oder das D- oder L-Isomer einer Aminosäure ist, gewählt aus der Gruppe bestehend aus R¹¹, einer gegebenenfalls substituierten aromatischen α-Aminosäure, A3c, A4c, A5c, A6c, Abu, Aib, Aic, β-Ala, Arg, Cha, Deg, Gaba, Ile, Leu, Nle, Pip, Pro, Sar, Ser, Ser(Bzl), Thr, Thr(Bzl), Val, Tic, Htic, Sala, Aala, Thza, Thia, Bal, Fala, Pala, hArg, Bip, Bpa, Dip, Pal, Sala und X⁰-Phe;
AA^{7b} nicht vorhanden ist oder ein D- oder L-Isomer einer Aminosäure ist, gewählt aus der Gruppe bestehend aus R¹¹, Bpa, Phe, F₅-Phe, X⁰-Phe, Nal, Pro, Ser, Ser(Bzl), Thr, Thr(Bzl), Trp, hArg, Bip, Tic, Htic, Dip, Sala, Aala, Thza, Thia, Bal, Fala und Pala;
AA⁸ nicht vorhanden ist oder das D- oder L-Isomer einer Aminosäure ist, gewählt aus der Gruppe bestehend aus R¹¹, Maa, Maaab, Thr, Thr(Bzl), Ser, Ser(Bzl), Tyr, Phe(4-O-Bzl), F₅-Phe und X⁵-Phe und einer gegebenenfalls substituierten aromatischen α-Aminosäure;
R¹ und R² jeweils unabhängig voneinander H, E-, E(O)₂S-, E(O)C-, EOOC-, R¹³ oder nicht vorhanden sind;
R³ und R⁴ jeweils unabhängig voneinander (C₁₋₁₂)-Alkyl, (C₂₋₁₂)-Alkenyl, (C₂₋₁₂)-Alkinyl, Phenyl, Naphthyl, Phenyl-(C₁₋₆)-alkyl, Phenyl-(C₂₋₆)-alkenyl, Phenyl-(C₂₋₆)-alkinyl, Naphthyl-(C₁₋₆)-alkyl, Naphthyl-(C₂₋₆)-alkenyl, Naphthyl-(C₂₋₆)-alkinyl, (Cyclo-(C₃₋₇)-alkyl)-(C₁₋₆)-alkyl, (Cyclo-(C₃₋₇)-alkyl)-(C₂₋₆)-alkenyl, (Cyclo-(C₃₋₇)-alkyl)-(C₂₋₆)-alkinyl, Heterocyclyl-(C₁₋₄)-alkyl, Heterocyclyl-(C₂₋₄)-alkenyl, Heterocyclyl-(C₂₋₄)-alkinyl, 1-Adamantyl, 2-Adamantyl, 9-Fluorenylmethyl, Dicyclopropylmethyl, Dimethylcyclopropylmethyl oder Benzhydryl;
R⁵ ist -OR⁶, -NR⁷R⁸ oder nicht vorhanden,
worin R⁶, R⁷ und R⁸ jeweils unabhängig voneinander H, (C₁₋₁₂)-Alkyl, (C₂₋₁₂)-Alkenyl, (C₂₋₁₂)-Alkinyl, Phenyl, Naphthyl, Phenyl-(C₁₋₆)-alkyl, Phenyl-(C₂₋₆)-alkenyl, Phenyl-(C₂₋₆)-alkinyl, Naphthyl-(C₁₋₆)-alkyl, Naphthyl-(C₂₋₆)-alkenyl, Naphthyl-(C₂₋₆)-alkinyl, 1-Adamantyl, 2-Adamantyl, 9-Fluorenylmethyl, Dicyclopropylmethyl, Dimethylcyclopropylmethyl oder Benzhydryl ist;
R⁹ und R¹⁰ jeweils, unabhängig voneinander, H, (C₁₋₆)-Alkyl, (C₃₋₄)-Alkenyl, (C₃₋₄)-Alkinyl, 1-Adamantyl oder 2-Adamantyl sind;
R¹¹, unabhängig vom jeweiligen Auftreten, eine D- oder L-Aminosäure der folgenden Formel ist: 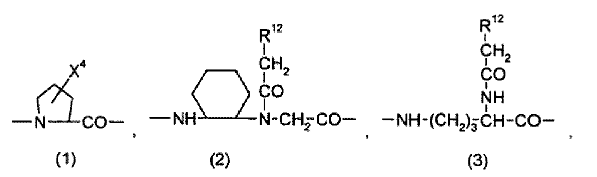 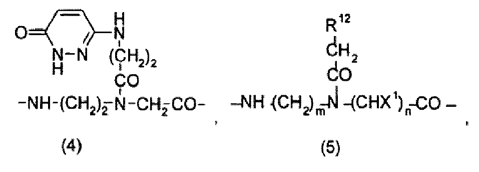 oder 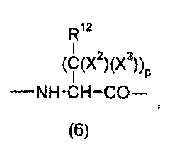 worin m und n jeweils, unabhängig voneinander, 1, 2 oder 3 und p 0, 1 oder 2 ist;
R¹², unabhängig vom jeweiligen Auftreten, ein gegebenenfalls substituierter Rest der folgenden Formel ist: 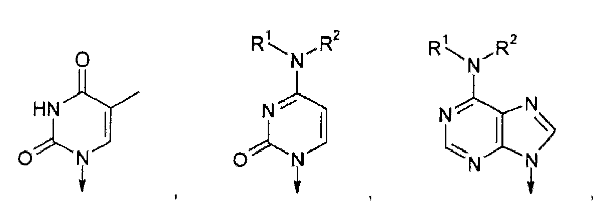 oder 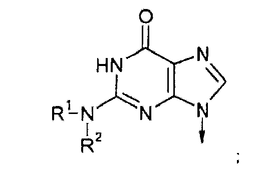 R¹³ ein Rest der Formel 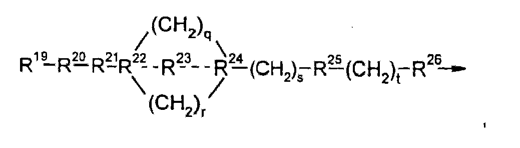 ist, worin q, r, s und t jeweils, unabhängig voneinander, 0, 1, 2, 3, 4 oder 5 ist;
R¹⁹ nicht vorhanden ist oder H, NH₂, OH, (C₁₋₆)-Hydroxyalkyl, N(R²⁷R²⁸), SO₃H ist, oder ein gegebenenfalls substituierter Rest, gewählt aus der Gruppe bestehend aus Heterocyclyl, Phenyl und Naphthyl,
worin der für R¹⁹ definierte, gegebenenfalls substituierte Rest gegebenenfalls substituiert ist mit einem oder mehreren Substituenten, gewählt, unabhängig vom jeweiligen Auftreten, aus der Gruppe bestehend aus Halogen, NO₂, OH, (C₁₋₆)-Alkyl, (C₂₋₆)-Alkenyl, (C₂₋₆)-Alkinyl, (C₁₋₆)-Alkoxy, NH₂, Mono- oder Di-(C₁-₆)-alkylamino, Bzl und O-Bzl;
R²⁰ O oder nicht vorhanden ist;
R²¹ (C₁₋₆)-Alkyl oder nicht vorhanden ist;
R²² N, O, C oder CH ist;
R²³ (C₁₋₆)-Alkyl oder nicht vorhanden ist;
R²⁴ N, CH oder C ist;
R²⁵ NH, O oder nicht vorhanden ist;
R²⁶ SO₂, CO oder CH ist;
R²⁷ und R²⁸ jeweils, unabhängig voneinander, H oder (C₁₋₆)-Alkyl ist;
E, unabhängig vom jeweiligen Auftreten, ein gegebenenfalls substituierter Rest ist, gewählt aus der Gruppe bestehend aus (C₁-₁₂)-Alkyl, (C₂₋₁₂)-Alkenyl, (C₂₋₁₂)-Alkinyl, Phenyl, Naphthyl, Phenyl-(C₁₋₆)-alkyl, Phenyl-(C₂₋₆)-alkenyl, Phenyl-(C₂₋₆)-alkinyl, Naphthyl-(C₁₋₆)-alkyl, Naphthyl-(C₂₋₆)-alkenyl, Naphthyl-(C₂₋₆)-alkinyl, (Cyclo-(C₃₋₇)-alkyl)-(C₁₋₆)-alkyl, (Cyclo-(C₃₋₇)-alkyl)-(C₂₋₆)-alkenyl, (Cyclo-(C₃₋₇)-alkyl)-(C₂-₆)-alkinyl, Heterocyclyl-(C₁-₄)-alkyl, Heterocyclyl-(C₂₋₄)-alkenyl, Heterocyclyl-(C₂₋₆)-alkinyl, 1-Adamantyl, 2-Adamantyl, Dicyclopropylmethyl, Dimethylcyclopropylmethyl, 9-Fluorenylmethyl und Benzhydryl;
worin der für E definierte, gegebenenfalls substituierte Rest gegebenenfalls substituiert ist mit einem oder mehreren Substituenten jeweils unabhängig voneinander gewählt aus der Gruppe bestehend aus Halogen, OH, Bzl, O-Bzl, NO₂, CN, COOH und SH;
X⁰ Halogen, NO₂, OH, (C₁₋₆)-Alkyl, (C₁₋₆)-Alkoxy, Mono- oder Di-(C₁₋₆)-alkylamino, Bzl, O-Bzl, NR⁹R¹⁰ oder CN ist;
X¹ H, (C₁-₆)-Alkyl, (C₂-₆)-Alkenyl, (C₂-₆)-Alkinyl, Indolyl, Imidazolyl, 1-Naphthyl, 3-Pyridyl, gegebenenfalls ringsubstituiertes Benzyl oder ein Rest ist, der der Seitenketten-Gruppe von Arg, Leu, Gln, Lys, Tyr, His, Thr, Trp, Phe, Val, Ala, Lys oder His entspricht;
worin das gegebenenfalls ringsubstituierte Benzyl gegebenenfalls substituiert ist mit einem oder mehreren Substituenten, gewählt aus der Gruppe bestehend aus Halogen, OH, (C₁₋₆)-Alkoxy, Mono- oder Di-(C₁₋₆)-alkylamino, (C₁₋₄)-Alkyl, (C₂₋₄)-Alkenyl, (C₂₋₄)-Alkinyl und NR⁹R¹⁰;
X² und X³ jeweils unabhängig voneinander H, Halogen, OH, =O, =S, (C₁-₁₂)-Alkyl**,** (C₂₋₁₂)-Alkenyl, (C₂₋₁₂)-Alkinyl, Phenyl, Naphthyl, Phenyl-(C₁₋₆)-alkyl, Phenyl-(C₂₋₆)-alkenyl, Phenyl-(C₂₋₆)-alkinyl, Naphthyl-(C₁₋₆)-alkyl, Naphthyl-(C₂₋₆)-alkenyl, Naphthyl-(C₂₋₆)-alkinyl, (Cyclo-(C₃₋₇)-alkyl)-(C₁₋₆)alkyl, (Cyclo-(C₃₋₇)-alkyl)-(C₂₋₆)alkenyl, (Cyclo-(C₃₋₇)-alkyl)-(C₂₋₆)-alkinyl, Heterocyclyl-(C₁₋₄)-alkyl, Heterocyclyl-(C₂-₄)-alkenyl, Heterocyclyl-(C₂₋₄)-alkinyl, 1-Adamantyl, 2-Adamantyl, Dicyclopropylmethyl oder Dimethylcyclopropylmethyl ist;
X⁴ H, OH oder NH₂ ist; und
X⁵ Halogen, NO₂, CH₃, OH, Bzl oder O-Bzl ist;
vorausgesetzt, daß:
mindestens 6 Aminosäurereste vorhanden sind;
wenn AA³ ein D- oder L-Isomer einer Aminosäure, gewählt aus der Gruppe bestehend aus Cys, hCys, Pen, Tpa oder Tmpa ist und AA⁶ ein D- oder L-Isomer einer Aminosäure, gewählt aus der Gruppe bestehend aus Cys, hCys, Pen, Tpa oder Tmpa ist, dann sind AA³ und AA⁶ durch eine Disulfidbindung verknüpft;
wenn AA¹ oder AA³ ein D- oder L-Isomer einer Aminosäure, gewählt aus der Gruppe bestehend aus Mac oder Macab ist, dann ist AA⁸ ein D- oder L-Isomer einer Aminosäure, gewählt aus der Gruppe bestehend aus Maa und Maaab und wenn AA⁸ ein D- oder L-Isomer einer Aminosäure,
gewählt aus der Gruppe bestehend aus Maa und Maaab ist, dann ist AA¹ oder AA³ ein D- oder
L-Isomer von Mac oder Macab und AA¹ oder AA³ ist durch eine Disulfidbindung mit AA⁸ verknüpft;
AA² kann nur dann D- oder L-Hca sein, wenn AA¹ nicht vorhanden ist;
wenn einer der Reste R¹ oder R² E(O)₂S-, E(O)C-, EOOC- oder R¹³ ist, dann ist der andere Rest H;
wenn R⁵ nicht vorhanden ist, dann ist einer der Reste R¹ oder R² ebenfalls nicht vorhanden und
die N-terminale Aminosäure und die C-terminale Aminosäure bilden zusammen eine Amidgruppe;
wenn einer der Reste X² oder X³ C=O oder C=S ist, dann ist der andere Rest nicht vorhanden;
und die Verbindung der Formel (I) weist nicht die Formel auf:
D-Phe-Tyr-cyclo(D-Cys-D-Trp-Lys-Cys)-Abu-Thr-NH₂;
Ac-Phe-Tyr-cyclo(D-Cys-D-Trp-Lys-Cys)-Abu-Thr-NH₂;
L-4-NO₂-Phe-Tyr-cyclo(D-Cys-D-Trp-Lys-Cys)-Abu-Thr-NH₂;
Ac-L-4-NO₂-Phe-Tyr-cyclo(D-Cys-D-Trp-Lys-Cys)-Abu-Thr-NH₂;
Hca-Tyr-cyclo(D-Cys-D-Trp-Lys-Cys)-Abu-Thr-NH₂;
D-Dip-Tyr-cyclo(Cys-D-Trp-Lys-D-Cys)-Val-Nal-NH₂;
D-4-NO₂-Phe-Phe(4-O-Bzl)-cyclo(D-Cys-D-Trp-Lys-Cys)Cha-Nal-NH₂;
oder
D-4-NO₂-Phe-cyclo(D-Cys-Phe(4-O-Bzl)-D-Trp-Lys-Cys)-Val-Tyr-NH₂.

2. Verbindung nach Anspruch 1, worin die Verbindung die Formel (II) aufweist: 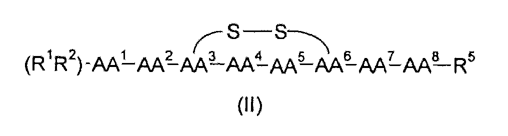 oder ein pharmazeutisch annehmbares Salz davon, worin
AA¹ nicht vorhanden ist oder das D- oder L-Isomer eine Aminosäure ist, gewählt aus der Gruppe bestehend aus R¹¹, Aac, Aic, Arg, Asn, Asp, Dip, Gln, Glu, Hyp, Lys, Mac, Macab, Orn, Pip, Pro, Ser, Ser(Bzl), Thr, Thr(Bzl), Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp und einer gegebenenfalls substituierten aromatischen α-Aminosäure, worin die gegebenenfalls substituierte aromatische α-Aminosäure gegebenenfalls substituiert ist mit einem oder mehreren Substituenten, gewählt aus der Gruppe bestehend aus Halogen, NO₂, OH, CN, (C₁₋₆)-Alkyl, (C₂₋₆)-Alkenyl, (C₂₋₆)-Alkinyl und NR⁹R¹⁰;
AA² nicht vorhanden ist oder das D- oder L-Isomer eine Aminosäure ist, gewählt aus der Gruppe bestehend aus R¹¹, Aic, Arg, Hca, His, Hyp, Pal, F₅-Phe, Phe, Pro, Trp, X⁰-Phe, Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua und Pyp; AA³ das D-oder L-Isomer einer Aminosäure ist, gewählt aus der Gruppe bestehend aus Cys, hCys, Pen, Tpa und Tmpa;
AA⁴ ein D- oder L-Isomer einer Aminosäure ist, gewählt aus der Gruppe bestehend aus Trp, N-Met-Trp, β-Met-Trp, His, hHis, hArg, Bip, Tic, Htic, Dip, Sala, Aala, Thza, Thia, Bal, Fala, Pala und einer gegebenenfalls substituierten aromatischen α-Aminosäure,
worin die gegebenenfalls substituierte aromatische α-Aminosäure gegebenenfalls substituiert ist mit einem oder mehreren Substituenten, jeweils unabhängig voneinander gewählt aus der Gruppe bestehend aus Halogen, NO₂, OH, (C₁₋₄)-Alkyl, (C₂₋₄)-Alkenyl, (C₂₋₄)-Alkinyl, Bzl, O-Bzl und NR⁹R¹⁰;
AA⁵ ein D- oder L-Isomer einer Aminosäure ist, gewählt aus der Gruppe bestehend aus 4-Pip-Gly, 4-Pip-Ala, cis-4-Acha, trans-4-Acha, trans-4-Amcha, hLys, Lys, Orn, hArg, Bip, Tic, Htic, Dip, Sala, Aala, Thza, Thia, Bal, Fala und Pala,
worin die Seitenketten-Aminogruppe der Aminosäure gegebenenfalls mono- oder disubstituiert ist mit R³ und R⁴;
AA⁶ ein D- oder L-Isomer einer Aminosäure ist, gewählt aus der Gruppe bestehend aus Cys, hCys, Pen, Tpa und Tmpa;
AA¹ nicht vorhanden ist oder ein D- oder L-Isomer eine Aminosäure ist, gewählt aus der Gruppe bestehend aus R¹¹, Aic, A3c, A4c, A5c, A6c, Abu, Aib, β-Ala, Arg, Bpa, Cha, Deg, Gaba, His, Ile, Leu, Nal, Nle, Pal, Phe, F₅-Phe, Pro, Sar, Ser, Ser(Bzl), Thr, Thr(Bzl), Trp, N-Me-Trp, Val, N-Me-Val, hArg, Bip, Tic, Htic, Dip, Sala, Aala, Thza, Thia, Bal, Fala, Pala und X⁰-Phe;
AA⁸ nicht vorhanden ist oder das D- oder L-Isomer eine Aminosäure ist, gewählt aus der Gruppe bestehend aus R¹¹, einer gegebenenfalls substituierten aromatischen α-Aminosäure, Maa, Maaab, Ser, Ser(Bzl), Thr, Thr(Bzl), Tyr, Phe(4-O-Bzl), F₅-Phe und X⁵-Phe;
R¹³ ein Rest gemäß der Formel 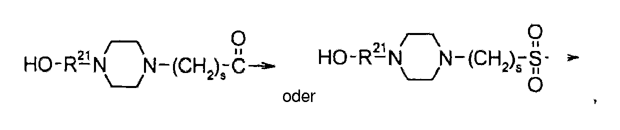 ist, worin R²¹ (C₁₋₄)-Alkyl ist und s 1, 2, 3 oder 4 ist und
X⁰ Halogen, NO₂, CH₃, OH, Bzl, O-Bzl oder CN ist;
vorausgesetzt, daß mindestens eines von AA⁷ oder AA⁸ vorhanden ist.

3. Verbindung nach Anspruch 1, worin die Verbindung die Formel (III) aufweist: 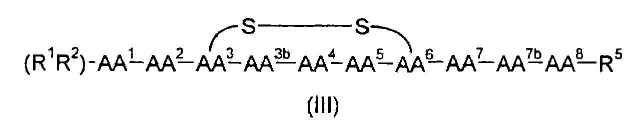 oder ein pharmazeutisch annehmbares Salz davon,
worin
AA¹ nicht vorhanden ist oder das D- oder L-Isomer einer Aminosäure ist, gewählt aus der Gruppe bestehend aus R¹¹, Aac, Aic, Arg, Asn, Asp, Gln, Glu, Hca, His, Hyp, Lys, Mac, Macab, Orn, Pro, Ser, Ser(Bzl), Thr, Thr(Bzl), Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp und einer gegebenenfalls substituierten aromatischen α-Aminosäure,
worin die gegebenenfalls substituierte aromatische α-Aminosäure gegebenenfalls substituiert ist mit einem oder mehreren Substituenten, gewählt aus der Gruppe bestehend aus Halogen, NO₂, OH, CN, (C₁₋₆)-Alkyl, (C₂₋₆)-Alkenyl, (C₂₋₆)-Alkinyl und NR⁹R¹⁰;
AA³ ein D- oder L-Isomer einer Aminosäure ist, gewählt aus der Gruppe bestehend aus Cys, hCys, Pen, Tpa und Tmpa;
AA^{3b} das D- oder L-Isomer einer Aminosäure ist, gewählt aus der Gruppe bestehend aus R¹¹, Arg, Bpa, F₅-Phe, His, Nal, Pal, 4-Pal, Phe, Trp, hArg, Bip, Tic, Htic, Dip, Sala, Aala, Thza, Thia, Bal, Fala, Pala, and X⁵-Phe;
AA⁴ ein D- oder L-Isomer eine Aminosäure ist, gewählt ist aus der Gruppe bestehend aus Trp, N-Met-Trp, β-Met-Trp, His, hHis, hArg, Bip, Tic, Htic, Dip, Sala, Aala, Thza, Thia, Bal, Fala, Pala und einer gegebenenfalls substituierten aromatischen α-Aminosäure;
worin die gegebenenfalls substituierte aromatische α-Aminosäure gegebenenfalls substituiert ist mit einem oder mehreren Substituenten, jeweils unabhängig voneinander gewählt aus der Gruppe bestehend aus Halogen, NO₂, OH, CN, (C₁₋₄)-Alkyl, (C₂₋₄)-Alkenyl, (C₂₋₄)-Alkinyl, Bzl, 0-Bzl und NR⁹R¹⁰;
AA⁵ ein D- oder L-Isomer einer Aminosäure ist, gewählt aus der Gruppe bestehend aus 4-Pip-Gly, 4-Pip-Ala, cis-4-Acha, trans-4-Acha, trans-4-Amcha, hLys, Lys, Orn, hArg, Bip, Tic, Htic, Dip, Sala, Aala, Thza, Thia, Bal, Fala, Pala,
worin die Seitenketten-Aminogruppe der Aminosäure gegebenenfalls mono- oder disubstituiert ist mit R³ und R⁴;
AA⁶ ein D- oder L-Isomer einer Aminosäure ist, gewählt aus der Gruppe bestehend aus Cys, hCys, Pen, Tpa und Tmpa;
AA⁷ nicht vorhanden ist oder ein D- oder L-Isomer einer Aminosäure ist, gewählt aus der Gruppe bestehend aus R¹¹, Aic, A3c, A4c, A5c, A6c, Abu, Aib, β-Ala, Arg, Bpa, Cha, Deg, Gaba, His, Ile, Leu, Nal, Nle, Pal, Phe, F₅-Phe, Pro, Sar, Ser, Ser(Bzl), Thr, Thr(Bzl), Trp, N-Me-Trp, Val, N-Me-Val, hArg, Bip, Tic, Htic, Dip, Sala, Aala, Thza, Thia, Bal, Fala, Pala und X⁰-Phe;
X⁰ Halogen, NO₂, CH₃, OH, CN, Bzl oder O-Bzl ist;
R¹ und R² jeweils unabhängig voneinander H, E-, E(O)₂S-, E(O)C-, EOOC-, R¹³ oder nicht vorhanden ist;
R⁵ -OR⁶ oder -NR⁷R⁸ ist;
R¹³ ein Rest der Fomel 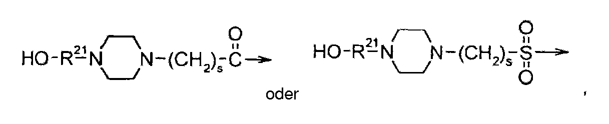 ist, worin R²¹ (C₁₋₄)-Alkyl ist und s 1, 2, 3 oder 4 ist;
vorausgesetzt, daß:
mindestens eines von AA¹ oder AA² vorhanden ist;
wenn AA¹ ein D- oder L-Isomer ist von Pro, Hyp, Arg, Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, σ-Chpa, Cit, Nua, Pyp oder His, dann kann AA² kein D- oder L-Isomer von Pro, Hyp, Arg, Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, I-Iqc, 3-Iqc, C4c, 5-Iqs, Htqa, 4-Mqc, Thn, α-Chpa, Cit, Nua, Pyp oder His sein;
wenn AA⁷ ein D- oder L-Isomer von Thr oder von Ser ist, dann kann AA⁸ kein D- oder L-Isomer von Thr oder von Ser sein;
zumindest eines von AA¹, AA², AA^{3b}, AA⁷, AA^{7b} oder AA⁸ das D- oder L-Isomer von R¹¹ ist; und
wenn eines von X² oder X³ =O oder =S ist, dann ist das andere nicht vorhanden;
oder ein pharmazeutisch annehmbares Salz davon.

4. Verbindung nach Anspruch 1, worin die Verbindung die Formel (IV) aufweist: 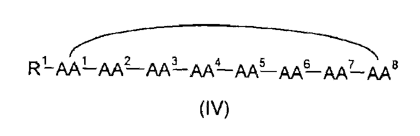 worin
AA¹ nicht vorhanden ist oder das D- oder L-Isomer einer Aminosäure ist, gewählt aus der Gruppe bestehend aus R¹¹, Aic, Hyp, Pro, Ser, Ser(Bzl), Thr, Thr(Bzl), Tic, Htic, Fala und einer gegebenenfalls substituierten aromatischen α-Aminosäure;
worin die gegebenenfalls substituierte aromatische α-Aminosäure gegebenenfalls substituiert ist mit einem oder mehreren Substituenten, jeweils unabhängig voneinander gewählt aus der Gruppe bestehend aus Halogen, NO₂, OH, CN, (C₁₋₆)-Alkyl, (C₂₋₆)-Alkenyl, (C₂₋₆)-Alkinyl, (C₁₋₆)-Alkoxy, Bzl, O-Bzl und NR⁹R¹⁰;
AA² nicht vorhanden ist oder das D- oder L-Isomer einer Aminosäure ist, gewählt aus der Gruppe bestehend aus R¹¹, Arg, F₅-Phe, His, Pal, Phe, Trp, hArg, Pala, Bal, Fala, Sala und X⁰-Phe;
AA³ das D- oder L-Isomer einer gegebenenfalls substituierten aromatischen Aminosäure ist, worin die gegebenenfalls substituierte aromatische α-Aminosäure gegebenenfalls substituiert ist mit einem oder mehreren Substituenten, gewählt aus der Gruppe bestehend aus Halogen, NO₂, OH, CN, (C₁₋₄)-Alkyl, (C₂₋₄)-Alkenyl, (C₂₋₄)-Alkinyl, Bzl, O-Bzl und NR⁹R¹⁰;
AA⁴ ein D- oder L-Isomer einer gegebenenfalls substituierten Aminosäure ist, gewählt aus der Gruppe bestehend aus Trp, N-Met-Trp, β-Me-Trp, Lys, Orn, hLys, cis-4-Acha, trans-4-Acha, trans-4-Amcha, 4-Pip-Gly, 4-Pip-Ala, hArg, Bip, Tic, Htic, Dip, Sala, Aala, Thza, Thia, Bal, Fala und Pala;
worin die Seitenketten-Aminogruppe der gegebenenfalls substituierten Aminosäure gegebenenfalls substituiert ist mit R³ und R⁴;
AA⁵ nicht vorhanden ist oder ein D- oder L-Isomer von R¹¹, A3c, A4c, A5c, A6c, Abu, Aib, Aic, β-Ala, Bpa, Cha, Deg, F₅-Phe, Gaba, Ile, Leu, Nal, Nle, Pal, Phe, Pro, Sar, Ser, Ser(Bzl), Thr, Thr(Bzl), Trp, N-Me-Trp, Val, N-Me-Val, hArg, Bip, Tic, Htic, Dip, Sala, Aala, Thza, Thia, Bal, Fala, Pala oder X⁰-Phe ist;
AA⁶ nicht vorhanden ist, das D- oder L-Isomer von R¹¹, eine aromatische α-Aminosäure, F₅-Phe, Phe, Thr, Thr(Bzl), Ser, Ser(Bzl) oder X⁰-Phe ist;
AA⁷ nicht vorhanden ist, das D- oder L-Isomer von R¹¹ oder das D- oder L-Isomer einer aromatischen α-Aminosäure ist;
AA⁸ ein D- oder L-Isomer von R¹¹ ist;
R¹ H, E-, E(O)₂S-, E(O)C-, EOOC- oder R¹³ ist;
R¹³ ein Rest der Formel 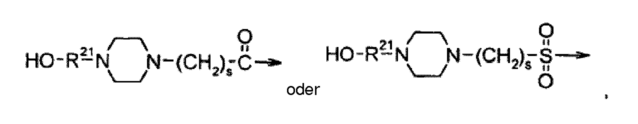 ist, worin R²¹ (C₁₋₄)-Alkyl ist und s 1, 2, 3 oder 4 ist;
X⁰ in der Definition von AA² und AA⁵ Halogen, NO₂, OH, (C₁₋₆)-Alkyl, (C₁₋₆)-Alkoxy, Mono- oder Di-(C₁₋₆)-Alkylamino, Bzl oder O-Bzl ist;
X⁰ in der Definition von AA⁶ Halogen, NO₂, OH, (C₁₋₆)-Alkyl, (C₁₋₆)-Alkoxy, Mono- oder Di-(C₁₋₆)-Alkylamino, Bzl, O-Bzl oder NR⁹R¹⁰ ist;
vorausgesetzt, daß:
mindestens eines von AA¹ oder AA² vorhanden ist;
wenn AA¹ nicht vorhanden ist, dann bilden AA² und AA⁸ zusammen eine Bindung; und
mindestens zwei von AA⁵, AA⁶ und AA⁷ vorhanden sind;
oder ein pharmazeutisch annehmbares Salz davon.

5. Verbindung nach Anspruch 2, worin AA¹ nicht vorhanden ist, Ac-D-Phe oder das D- oder L-Isomer von R¹¹, Pip, Pro oder Ser ist oder von einer aromatischen α-Aminosäure, gewählt aus der Gruppe bestehend aus Cpa, Dip, Nal, Pal und Phe; 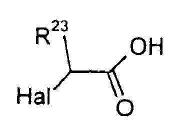 AA² nicht vorhanden ist, Aic, Pal, Phe, F₅-Phe, 4-NO₂-Phe, Trp, Tyr, Phe(4-O-Bzl) oder 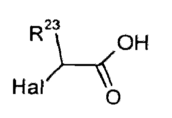 ist;
AA³ das D- oder L-Isomer einer Aminosäure, gewählt aus der Gruppe bestehend aus Pen, Cys, hCys und Tmpa ist;
AA⁴ das D- oder L-Isomer von Trp, His, N-Me-Trp, β-Me-Trp, hTrp oder hHis ist;
AA⁵ Lys, hLys, N-Me-Lys, Orn, cis-4-Acha oder 4-Pip-Ala ist;
AA⁶ das D- oder L-Isomer einer Aminosäure ist, gewählt aus der Gruppe bestehend aus Cys, hCys, Pen und Tmpa;
AA⁷ A3c, A4c, A5c, A6c, Abu, Aic, β-Ala, Gaba, Nle, F₅-Phe, Phe, Pro, Sar, Ser, Thr, Thr(Bzl), Tyr, Val oder nicht vorhanden ist; und
AA⁸ R¹¹, Nal, Thr, Thr(Bzl), Tyr, Phe(4-O-Bzl) oder nicht vorhanden ist;
oder ein pharmazeutisch annehmbares Salz davon.

6. Verbindung nach Anspruch 5, worin
AA¹ nicht vorhanden ist oder das D- oder L-Isomer von R¹¹, Pip oder Pro oder von einer aromatischen α-Aminosäure ist, gewählt aus der Gruppe bestehend aus Cpa, Dip, Nal, Pal, Phe und Ac-Phe;
AA² Tyr, Pal, Phe, 4-NO₂-Phe, Trp oder nicht vorhanden ist;
AA³ ein D- oder L-Isomer von Cys oder Pen ist;
AA⁴ D-Trp ist;
AA⁵ Lys, Orn oder cis-4-Acha ist;
AA⁶ ein D- oder L-Isomer von Cys oder Pen ist;
AA⁷ A3c, A4c, A5c, A6c, Abu, Aic, β-Ala, Gaba, Nle, Phe, Pro, Sar, Thr, Thr(Bzl), Tyr, Val oder nicht vorhanden ist; und
AA⁸ R¹¹, Thr, Tyr, Nal oder nicht vorhanden ist;
oder ein pharmazeutisch annehmbares Salz davon.

7. Verbindung nach Anspruch 3, worin
AA¹ R¹¹, Aic, Hca, Pro, Ser, Ser(Bzl), Trp, Tyr oder ein D- oder L-Isomer einer aromatischen α-Aminosäure, gewählt aus der Gruppe bestehend aus Cpa, Nal, Ac-Nal, Phe, Ac-Phe, 4-NO₂-Phe und Ac-4-NO₂-Phe ist;
AA² Pal, Phe, F₅-Phe, Tyr oder nicht vorhanden ist;
AA³ ein D- oder L-Isomer von Cys, hCys, Pen oder Tmpa ist;
AA^{3b} Pal, 4-Pal, His, Trp, Tyr, Phe(4-O-Bzl), Phe oder R¹¹ ist;
AA⁴ ein D- oder L-Isomer von Trp oder His ist;
AA⁵ Lys, N-Me-Lys, Orn, hLys, cis-4-Acha oder 4-Pip-Ala ist;
AA⁶ ein D- oder L-Isomer von Cys, hCys, Pen oder Tmpa ist;
AA⁷ R¹¹, A4c, A5c, Abu, β-Ala, Gaba, Phe, F₅-Phe, Ser(Bzl), Thr, Thr(Bzl), Phe(4-O-Bzl) oder nicht vorhanden ist;
AA^{7b} R¹¹, Nal, F₅-Phe, X⁰-Phe oder nicht vorhanden ist, worin X⁰ Halogen, NO₂, CH₃, OH, Bzl oder O-Bzl ist; und
AA⁸ R¹¹, Nal, Tyr, Phe(4-O-Bzl) oder nicht vorhanden ist;
oder ein pharmazeutisch annehmbares Salz davon.

8. Verbindung nach Anspruch 7, worin
AA¹ R¹¹, Aic, Hca, Pro, Ser(Bzl) oder ein D- oder L-Isomer einer aromatischen α-Aminosäure, gewählt aus der Gruppe bestehend aus Cpa, Nal, Ac-Nal, Phe, Ac-Phe, 4-NO₂-Phe und Ac-4-NO₂-Phe ist;
AA² Pal, Tyr oder nicht vorhanden ist;
AA³ ein D- oder L-Isomer von Cys oder Pen ist;
AA^{3b} R¹¹, Pal, 4-Pal, Trp, Tyr, Phe(4-O-Bzl) oder Phe ist, worin R¹¹ (T)aeg ist;
AA⁴ D-Trp ist;
AA⁵ Lys, N-Me-Lys, Orn, oder cis-4-Acha ist;
AA⁶ ein D- oder L-Isomer von Cys oder Pen ist;
AA⁷ R¹¹, A5c, Abu, Ser(Bzl), Thr, Thr(Bzl), Phe(4-O-Bzl), Gaba oder nicht vorhanden ist;
AA^{7b} Nal, X⁰-Phe oder nicht vorhanden ist; und
AA⁸ Tyr oder nicht vorhanden ist;
oder ein pharmazeutisch annehmbares Salz davon.

9. Verbindung nach Anspruch 4, worin
AA¹ Aic, Hyp, Cpa, D-Cpa, Nal, Pal, Phe, Pro, R¹¹, Tyr oder nicht vorhanden ist;
AA² Phe, Trp, F₅-Phe, His, Tyr, Phe(4-O-Bzl) oder R¹¹ ist;
AA³ ein D-Isomer von Trp, His oder Pal ist;
AA⁴ Lys, N-Me-Lys, Orn, hLys, cis-4-Acha oder 4-Pip-Ala ist;
AA⁵ Pal, Phe(4-O-Bzl), Thr(Bzl), Thr, Sar, Gaba, β-Ala, A4c, A5c, A6c, Abu, Aic oder nicht vorhanden ist;
AA⁶ Thr, Tyr, Ser, F₅-Phe, Cpa, Nal oder D- oder L-Phe ist;
AA⁷ Nal, Pal oder nicht vorhanden ist; und
AA⁸ R¹¹ ist;
oder ein pharmazeutisch annehmbares Salz davon.

10. Verbindung nach Anspruch 9, worin
AA¹ Cpa, Nal, Pal, Phe, Tyr oder nicht vorhanden ist;
AA² Phe, Tyr, Trp oder R¹¹ ist;
AA³ D-Trp ist;
AA⁴ Lys, N-Me-Lys oder cis-4-Acha ist;
AA⁵ Pal, Phe(4-O-Bzl), Aic, Gaba, A5c oder nicht vorhanden ist;
AA⁶ Thr, Nal oder D- oder L-Phe ist;
AA⁷ nicht vorhanden ist; und
AA⁸ R¹¹ ist;
oder ein pharmazeutisch annehmbares Salz davon.

11. Verbindung nach Anspruch 2, worin R¹ und R⁵ nicht vorhanden sind und die N-terminale Aminosäure und die C-terminale Aminosäure zusammen eine Amidbindung bilden, oder ein pharmazeutisch annehmbares Salz davon.

12. Verbindung nach Anspruch 3, worin R¹ und R⁵ nicht vorhanden sind und die N-terminale Aminosäure und die C-terminale Aminosäure zusammen eine Amidbindung bilden, oder ein pharmazeutisch annehmbares Salz davon.

13. Verbindung nach Anspruch 6, worin die Verbindung die Formel aufweist:
Ac-D-Phe-Tyr-cyclo(D-Cys-D-Trp-Lys-Cys)-Abu-Thr-NH₂;
Nal-Tyr-cyclo(Cys-D-Trp-Lys-D-Cys)-Val-Nal-NH₂;
Nal-Tyr-cyclo(Cys-D-Trp-Lys-D-Cys)-Abu-Nal-NH₂;
D-Dip-Tyr-cyclo(Cys-D-Trp-Lys-D-Cysj-Abu-Nal-NH₂;
Dip-Tyr-cyclo(D-Cys-D-Trp-Lys-D-Cys)-Abu-Nal-NH₂;
Nal-Tyr-cyclo(D-Cys-D-Trp-Lys-D-Cys)-Abu-Nal-NH₂;
Dip-Tyr-cyclo(D-Cys-D-Trp-Lys-D-Cys)-Val-Nal-NH₂;
Nal-Tyr-cyclo(D-Cys-D-Trp-Lys-D-Cys)-Val-Nal-NH₂;
cyclo(D-Phe-Tyr-cyclo(D-Cys-D-Trp-Lys-Cys)-Abu-Thr);
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp-Lys-D-Cys)-A6c-Nal-NH₂;
(G(z))aeg-cyclo(D-Cys-D-Trp-Lys-D-Cys)-A5c-Nal-NH₂:
Pal-cyclo(D-Cys-D-Trp-Lys-D-Cys)-A5c-Nal-NH₂;
Cpa-Pal-cyclo(D-Cys-D-Trp-Lys-D-Cys)-β-Ala-Nal-NH₂;
Cpa-Pal-cyclo(D-Cys-D-Trp-Lys-D-Cys)-Sar-Nal-NH₂;
Cpa-Pal-cyclo(D-Cys-D-Trp-Lys-D-Cys)-Gaba-Nal-NH₂;
Cpa-Pal-cyclo(D-Cys-D-Trp-Lys-D-Cys)-Pro-Nal-NH₂;
Pro-Phe-c(D-Cys-D-Trp-Lys-D-Cys)-Nle-Phe-NH₂;
Pro-Phe-c(D-Cys-D-Trp-Lys-D-Cys)-Thr-Nle-NH₂;
Pro-Phe-c(D-Cys-D-Trp-Lys-D-Cys)-Thr-Phe-NH₂;
Cpa-Phe-c(D-Cys-D-Trp-Lys-D-Cys)-Gaba-NH₂;
Cpa-Phe-c(D-Cys-D-Trp-Lys-D-Cys)-Gaba-Tyr-NH₂;
Pip-Phe-c(D-Cys-D-Trp-Lys-D-Cys)-NH₂;
Pip-Phe-c(Cys-D-Trp-Lys-Cys)-Gaba-NH₂; oder
Pro-Phe-c(D-Cys-D-Trp-Lys-D-Cys)-Thr-NH₂;
oder ein pharmazeutisch annehmbares Salz davon.

14. Verbindung nach Anspruch 6, worin die Verbindung die Formel aufweist:
Phe-cyclo(Cys-D-Trp-Lys-Cys)-Thr-NH₂;
Phe-Tyr-cyclo(D-Cys-D-Trp-Lys-Cys)-Abu-Thr-NH₂;
Ac-D-Phe-Tyr-cyclo(D-Cys-D-Trp-Lys-Cys)-Abu-Thr-NH₂;
Nal-Tyr-cyclo(Cys-D-Trp-Lys-D-Cys)-Val-Nal-NH₂;
Nal-Tyr-cyclo(Cys-D-Trp-Lys-D-Cys)-Abu-Nal-NH₂;
Dip-Tyr-cyclo(D-Cys-D-Trp-Lys-D-Cys)-Abu-Nal-NH₂;
Nal-Tyr-cyclo(D-Cys-D-Trp-Lys-D-Cys)-Abu-Nal-NH₂;
Dip-Tyr-cyclo(D-Cys-D-Trp-Lys-D-Cys)-Val-Nal-NH₂;
Nal-Tyr-cyclo(D-Cys-D-Trp-Lys-D-Cys)-Val-Nal-NH₂;
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp-Lys-D-Cys)-A6c-Nal-NH₂;
(G(z))aeg-cyclo(D-Cys-D-Trp-Lys-D-Cys)-A5c-Nal-NH₂;
D-Cpa-cyclo(Cys-D-Trp-Lys-D-Cys)-A5c-Nal-NH₂;
Pal-cyclo(D-Cys-D-Trp-Lys-D-Cys)-A5c-Nal-NH₂;
Cpa-cyclo(D-Cys-D-Trp-Lys-D-Cys)-A5c-Nal-NH₂;
Cpa-Pal-cyclo(D-Cys-D-Trp-Lys-D-Gys)-ß-Ala-Nal-NH₂;
Cpa-Pal-cyclo(D-Cys-D-Trp-Lys-D-Cys)-Sar-Nal-NH₂;
Cpa-Pal-cyclo(D-Cys-D-Trp-Lys-D-Cys)-Aic-Nal-NH₂;
Cpa-Pal-cyclo(D-Cys-D-Trp-Lys-D-Cys)-Gaba-Nal-NH₂;
Cpa-Pal-cyclo(D-Cys-D-Trp-Lys-D-Cys)-Pro-Nal-NH₂;
(T)aeg-cyclo(D-Gys-D-Trp-Lys-D-Cys)-(A)aeg-NH₂;
Cpa-Pal-cyclo(D-Cys-D-Trp-Lys-D-Cys)-A4c-Nal-NH₂;
Cpa-Pal-cyclo(D-Cys-D-Trp-Lys-D-Cys)-Nal-NH₂;
Pal-cyclo(D-Cys-D-Trp-Lys-D-Cys)-Nal-NH₂;
Pro-Phe-cyclo(Cys-D-Trp-Lys-D-Cys)-Val-NH₂;
Pro-Phe-cyclo(D-Cys-D-Trp-Lys-Cys)-Val-NH₂;
Pip-4-NO2-Phe-cyclo(D-Cys-D-Trp-Lys-D-Cys)-Nle-NH₂;
(G)aeg-Pal-cyclo(D-Cys-D-Trp-Lys-D-Cys)-Thr(Bzl)-(C)aeg-NH₂; oder
(C)aeg-Pal-cyclo(D-Cys-D-Trp-Lys-D-Cys)-Thr(Bzl)-(G)aeg-NH₂;
oder ein pharmazeutisch annehmbares Salz davon.

15. Verbindung nach Anspruch 8, worin die Verbindung die Formel aufweist:
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂:
D-Phe-cyclo(Cys-Tyr-D-Trp-Lys-Cys)-Thr-NH₂;
D-4-NO₂-Phe-cyclo(D-Cys-Tyr-D-Trp-Lys-Cys)-Nal-NH₂;
Ac-D-4-NO₂-Phe-cyclo(D-Cys-Tyr-D-Trp-Lys-Cys)-Nal-NH₂;
D-4-NO₂-Phe-Pal-cyclo(D-Cys-Phe(4-O-Bzl)-D-Trp-Lys-Cys)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys)-Thr-Tyr-NH₂;
D-4-NO₂-Phe-cyclo(D-Cys-Pal-D-Trp-Lys-Cys)-Thr(Bzl)-NH₂:
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Gys-Pal-D-Trp-Lys-Gys)-Thr(Bzl)-Tyr-NH₂;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NH₂;
D-Cpa-cyclo(D-Cys-(T)aeg-D-Trp-Lys-Cys)-Thr(Bzl)-Tyr-NH₂;
(C)aeg-c(D-Cys-Pal-D-Trp-Lys-D-Cys)-Thr(Bzl)-Tyr-NH₂;
D-Cpa-c(D-Cys-Pal-D-Trp-Lys-D-Cys)Thr(Bzl)-Tyr-NH₂:
(T)aeg-c(Pen-Pal-D-Trp-Lys-D-Cys)Thr(Bzl)-Tyr-NH₂;
(T)aeg-c(D-Cys-Trp-D-Trp-Lys-D-Cys)Thr(Bzl)-Tyr-NH₂;
(T)aeg-c(D-Cys-Phe-D-Trp-Lys-D-Cys)Thr(Bzl)-Tyr-NH₂;
(T)aeg-c(D-Cys-Pal-D-Trp-Orn-D-Cys)Thr(Bzl)-Tyr-NH₂;
(T)aeg-c(D-Cys-Pal-D-Trp-hLys-D-Cys)Thr(Bzl)-Tyr-NH₂;
(T)aeg-c(D-Cys-Pal-D-Trp-lamp-D-Cys)Thr(Bzl)-Tyr-NH₂;
(T)aeg-c(D-Cys-Pal-D-Trp-Cha(4-am)-D-Cys)Thr(Bzl)-Tyr-NH₂;
(T)aeg-c(D-Cys-Pal-D-Trp-Lys-D-Cys)-Ser(Bzl)-Tyr-NH₂;
(T)aeg-c(D-Cys-Pal-D-Trp-Lys-D-Cys)Thr(Bzl)-D-Tyr-NH₂;
(T)aeg-c(D-Cys-Pal-D-Trp-Lys-D-Cys)Thr(Bzl)-Trp-NH₂;
(T)aeg-c(D-Cys-Pal-D-Trp-Lys-D-Pen)Thr(Bzl)-Tyr-NH₂;
(C)aeg-c(D-Cys-Phe-D-Trp-Lys-D-Cys)Thr(Bzl)-Tyr-NH₂;
Ina-c(D-Cys-Phe-D-Trp-Lys-D-Cys)-Thr(Bzl)-Tyr-NH₂:
Mnf-c(D-Cys-Phe-D-Trp-Lys-D-Cys)-Thr(Bzl)-Tyr-NH₂;
Inp-c(D-Cys-Phe-D-Trp-Lys-D-Cys)-Thr(Bzl)-Tyr-NH₂;
Nua-c(D-Cys-Phe-D-Trp-Lys-D-Cys)-Thr(Bzl)-Tyr-NH₂;
(T)aeg-Pal-c(D-Cys-D-Trp-Lys-D-Cys)Thr(Bzl)-Tyr-NH₂;
(T)aeg-Pal-c(D-Cys-D-Trp-Lys-D-Cys)Tyr(Bzl)-Thr-NH₂;
(C)aeg-Phe-c(D-Cys-D-Trp-Lys-D-Cys)Thr(Bzl)-Tyr-NH₂; oder
(T)aeg-D-Trp-c(D-Cys-Pal-Lys-D-Cys)Thr(Bzl)-Leu-NH₂;
oder ein pharmazeutisch annehmbares Salz davon.

16. Verbindung nach Anspruch 8, worin die Verbindung die Formel aufweist:
Hca-cyclo(D-Cys-Tyr-D-Trp-Lys-Cys)-Nal-NH₂;
Ac-Nal-cyclo(D-Cys-Tyr-D-Trp-Lys-Cys)-Nal-NH₂;
Ac-D-Phe-cyclo(D-Cys-Tyr-D-Trp-Lys-Cys)-Nal-NH₃;
Ac-D-Nal-cyclo(D-Cys-Tyr-D-Trp-Lys-Cys)-Nal-NH₂;
D-Phe-cyclo(D-Cys-Tyr-D-Trp-Lys-Cys)-Nal-NH₂;
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂;
D-Phe-cyclo(Cys-Tyr-D-Trp-Lys-Cys)-Thr-NH₂;
D-4-NO₂-Phe-cyclo(D-Cys-Tyr-D-Trp-Lys-Cys)-Nal-NH₂;
Ac-D-4-NO₂-Phe-cyclo(D-Cys-Tyr-D-Trp-Lys-Cys)-Nal-NH₂;
D-4-NO₂-Phe-Pal-cyclo(D-Cys-Phe(4-O-Bzl)-D-Trp-Lys-Cys)-Tyr-NH₂;
D-4-NO₂-Phe-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys)-Thr(Bzl)-NH₂;
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Cys-Pal-D-Trp-Lys-Cys)-Thr(Bzl)-Tyr-NH₂;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-Cys)-Thr(Bzl)-Tyr-NH₂;
(C)aeg-cyclo(D-Cys-Pal-D-Trp-Lys-Cys)-Thr(Bzl)-Tyr-NH₂;
Aic-cyclo(D-Cys-Pal-D-Trp-Lys-Cys)-Thr(Bzl)-Tyr-NH₂;
(C(z))aeg-cyclo(D-Cys-Pal-D-Trp-Lys-Cys)-Thr(Bzl)-Tyr-NH₂;
(A(z))aeg-cyclo(D-Cys-Pal-D-Trp-Lys-Cys)-Thr(Bzl)-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NH₂;
D-Cpa-cyclo(D-Cys-(T)aeg-D-Trp-Lys-Cys)-Thr(Bzl)-Tyr-NH₂;
(T)aeg-cyclo(D-Cys-Pal-D-Trp-Lys-D-Cys)-Thr(Bzl)-p-Me-Phe-NH₂;
Ac-(T)aeg-cyclo(D-Cys-Pal-D-Trp-Lys-D-Cys)-Thr(Bzl)-Tyr-NH₂;
(T)aeg-cyclo(D-Cys-Pal-D-Trp-Lys-D-Cys)-Nal-NH₂;
D-Cpa-cyclo(D-Cys-Pal-D-Trp-Lys-D-Cys)-Nal-NH₂;
(A)aeg-cyclo(D-Cys-Pal-D-Trp-Lys-D-Cys)-Thr(Bzl)-Tyr-NH₂; (C)aeg-cyclo(D-Cys-Pal-D-Trp-Lys-D-Cys)-Thr(Bzl)-Tyr-NH₂;
(C)aeg-c(D-Cys-Pal-D-Trp-Lys-D-Cys)-Thr(Bzl)-Tyr-NH₂;
D-Cpa-c(D-Cys-Pal-D-Trp-Lys-D-Cys)Thr(Bzl)-Tyr-NH₂;
(T)aeg-c(Pen-Pal-D-Trp-Lys-D-Cys)Thr(Bzl)-Tyr-NH₂;
(T)aeg-c(D-Cys-Trp-D-Trp-Lys-D-Cys)Thr(Bzl)-Tyr-NH₂;
(T)aeg-c(D-Cys-Phe-D-Trp-Lys-D-Cys)Thr(Bzl)-Tyr-NH₂;
(T)aeg-c(D-Cys-Pal-D-Trp-Orn-D-Cys)Thr(Bzl)-Tyr-NH₂;
(T)aeg-c(D-Cys-Pal-D-Trp-hLys-D-Cys)Thr(Bzl)-Tyr-NH₂;
(T)aeg-c(D-Cys-Pal-D-Trp-lamp-D-Cys)Thr(Bzl)-Tyr-NH₂;
(T)aeg-c(D-Cys-Pal-D-Trp-Cha(4-am)-D-Cys)Thr(Bzl)-Tyr-NH₂;
(T)aeg-c(D-Cys-Pal-D-Trp-Lys-D-Cys)-Ser(Bzl)-Tyr-NH₂;
(T)aeg-c(D-Cys-Pal-D-Trp-Lys-D-Cys)Thr(Bzl)-D-Tyr-NH₂;
(T)aeg-c(D-Cys-Pal-D-Trp-Lys-D-Cys)Thr(Bzl)-Trp-NH₂;
(T)aeg-c(D-Cys-Pal-D-Trp-Lys-D-Pen)Thr(Bzl)-Tyr-NH₂;
(C)aeg-c(D-Cys-Phe-D-Trp-Lys-D-Cys)Thr(Bzl)-Tyr-NH₂;
Ina-c(D-Cys-Phe-D-Trp-Lys-D-Cys)-Thr(Bzl)-Tyr-NH₂;
Mnf-c(D-Cys-Phe-D-Trp-Lys-D-Cys)-Thr(Bzl)-Tyr-NH₂;
Inp-c(D-Cys-Phe-D-Trp-Lys-D-Cys)-Thr(Bzl)-Tyr-NH₂;
Nua-c(D-Cys-Phe-D-Trp-Lys-D-Cys)-Thr(Bzl)-Tyr-NH₂;
(T)aeg-Pal-c(D-Cys-D-Trp-Lys-D-Cys)Thr(Bzl)-Tyr-NH₂;
(T)aeg-Pal-c(D-Cys-D-Trp-Lys-D-Cys)Tyr(Bzl)-Thr-NH₂; oder
(C)aeg-Phe-c(D-Cys-D-Trp-Lys-D-Cys)Thr(Bzl)-Tyr-NH₂
(T)aeg-D-Trp-c(D-Cys-Pal-Lys-D-Cys)Thr(Bzl)-Leu-NH₂;
oder ein pharmazeutisch annehmbares Salz davon.

17. Verbindung nach Anspruch 10, worin die Verbindung die Formel aufweist:
cyclo(Trp-D-Trp-Lys-Phe(4-O-Bzl)-Phe-(T)aeg);
cyclo(Trp-D-Trp-Lys-Pal-Phe -(T)aeg); or
cyclo(Phe-Phe-D-Trp-Lys-Thr-(T)aeg);
oder ein pharmazeutisch annehmbares Salz davon.

18. Verwendung einer Verbindung nach Anspruch 13 oder eines pharmazeutisch annehmbaren Salzes davon in der Herstellung eines Medikamentes zum Hervorrufen eines Neuromedin B Rezeptor-Agonist-Effektes in einem Individuum, das dies benötigt.

19. Verwendung einer Verbindung nach Anspruch 14 oder eines pharmazeutisch annehmbaren Salzes davon in der Herstellung eines Medikaments zum Hervorrufen eines Somatostatin Rezeptor-Agonist-Effektes in einem Individuum, das dies benötigt.

20. Verwendung einer Verbindung nach Anspruch 15 oder eines pharmazeutisch annehmbaren Salzes davon in der Herstellung eines Medikaments zum Hervorrufen eines Neuromedin B Rezeptor-Agonist-Effektes in einem Individuum, das dies benötigt.

21. Verwendung einer Verbindung nach Anspruch 16 oder eines pharmazeutisch annehmbaren Salzes davon in der Herstellung eines Medikaments zum Hervorrufen eines Somatostatin Rezeptor-Agonist-Effektes in einem Individuum, das dies benötigt.

22. Verwendung einer Verbindung nach Anspruch 17 oder eines pharmazeutisch annehmbaren Salzes davon in der Herstellung eines Medikaments zum Hervorrufen eines Somatostatin-Rezeptor-Agonisten-Effektes in einem Individuum, das dies benötigt, vorausgesetzt, daß die Verbindung nicht ist:
Cyclo(Trp-D-Trp-Lys-Phe(4-O-Bzl)-Phe-(T)aeg);
oder
Cyclo(Trp-D-Trp-Lys-Pal-Phe-(T)aeg).

23. Verwendung einer Verbindung nach Anspruch 14 oder eines pharmazeutisch annehmbaren Salzes davon in der Herstellung eines Medikaments zum Hervorrufen eines SSTR-1-Agonisten-Effektes in einem Individuum, das dies benötigt, vorausgesetzt, daß die Verbindung nicht ist:
Nal-Tyr-cyclo(Cys-D-Trp-Lys-D-Cys)-Val-Nal-NH₂;
Nal-Tyr-cyclo(Cys-D-Trp-Lys-D-Cys)-Abu-Nal-NH₂;
Dip-Tyr-cyclo(D-Cys-D-Trp-Lys-D-Cys)-Abu-Nal-NH₂;
Nal-Tyr-cyclo(D-Cys-D-Trp-Lys-D-Cys)-Abu-Nal-NH₂;
Dip-Tyr-cyclo(D-Cys-D-Trp-Lys-D-Cys)-Val-Nal-NH₂;
Nal-Tyr-cyclo(D-Cys-D-Trp-Lys-D-Cys)-Val-Nal-NH₂;
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp-Lys-D-Cys)-A6c-Nal-NH₂;
(G(z))aeg-cyclo(D-Cys-D-Trp-Lys-D-Cys)-A5c-Nal-NH₂;
D-Cpa-cyclo(Cys-D-Trp-Lys-D-Cys)-A5c-Nal-NH₂; Pal-cyclo(D-Cys-D-Trp-Lys-D-Cys)-A5c-Nal-NH₂;
Cpa-cyclo(D-Cys-D-Trp-Lys-D-Cys)-A5c-Nal-NH₂; Cpa-Pal-cyclo(D-Cys-D-Trp-Lys-D-Cys)-β-Ala-Nal-NH₂;
cyclo(D-Cys-D-Trp-Lys-D-Cys)-A5c-Nal-NH₂;
Cpa-Pal-cyclo(D-Cys-D-Trp-Lys-D-Cys)-Sar-Nal-NH₂;
Cpa-Pal-cyclo(D-Cys-D-Trp-Lys-D-Cys)-Aic-Nal-NH₂;
Cpa-Pal-cyclo(D-Cys-D-Trp-Lys-D-Cys)-Gaba-Nal-NH₂; oder
Cpa-Pal-cyclo(D-Cys-D-Trp-Lys-D-Cys)-Pro-Nal-NH₂.

24. Verwendung einer Verbindung nach Anspruch 16 oder eines pharmazeutisch annehmbaren Salzes davon in der Herstellung eines Medikaments zum Hervorrufen eines SSTR-1-Agonisten-Effektes in einem Individuum, das dies benötigt, vorausgesetzt, daß die Verbindung nicht ist:
Ac-D-Phe-cyclo(D-Cys-Tyr-D-Trp-Lys-Cys)-Nal-NH₂;
Ac-D-Nal-cyclo(D-Cys-Tyr-D-Trp-Lys-Cys)-Nal-NH₂;
D-Phe-cyclo(D-Cys-Tyr-D-Trp-Lys-Cys)-Nal-NH₂;
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂;
D-4-NO₂-Phe-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys)-Thr(Bzl)-NH₂;
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Cys-Pal-D-Trp-Lys-Cys)-Thr(Bzl)-Tyr-NH₂;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-Cys)-Thr(Bzl)-Tyr-NH₂;
(C)aeg-cyclo(D-Cys-Pal-D-Trp-Lys-Cys)-Thr(Bzl)-Tyr-NH₂;
Aic-cyclo(D-Cys-Pal-D-Trp-Lys-Cys)-Thr(Bzl)-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NH₂; oder
D-Cpa-cyclo(D-Cys-(T)aeg-D-Trp-Lys-Cys)-Thr(Bzl)-Tyr-NH₂.

25. Pharmazeutische Zusammensetzung, umfassend eine wirksame Menge einer Verbindung nach Anspruch 1 oder eines pharmazeutisch annehmbaren Salzes davon und einen pharmazeutisch annehmbaren Träger.

26. Verwendung einer Verbindung nach Anspruch 1 in der Herstellung eines Medikaments zur Behandlung einer Krankheit, gewählt aus Lungenkrebs, Gliom, Anorexie, Hypothyreose, Hyperaldosteronismus, H. pylori Proliferation, Akromegalie, Restenose, Morbus Crohn, systemische Sklerose, externe und interne Pankreas-Pseudo-Zysten und -Asziten, VIPom, Nesidoblastosis, Hyperinsulinismus, Gastrinom, Zollinger-Ellison-Syndrom, Diarrhoe, mit AIDS verbundene Diarrhoe, mit Chemotherapie verbundene Diarrhoe, Sklerodermie, Reizdarmsyndrom, Pankreatitis, Dünndarmobstruktion, gastroösophagealer Reflux, duodenogastrischer Reflux, Cushing-Syndrom, Gonadotropinom, Hyperparathyreoidismus, Graves-Erkrankung, diabetische Neuropathie, Paget-Erkrankung, polyzystische Ovarienerkrankung, Schilddrüsenkrebs, Hepatom, Leukämie, Meningiom, Krebs-Kachexie, orthostatische Hypotonie, postprandiale Hypotonie, panische Attacken, GH sekretierende Adenome, Akromegalie, TSH sekretierende Adenome, Prolactin sekretierende Adenome, Insulinom, Glucagonom, Diabetes mellitus, Hyperlipidämie, Insulinresistenz, Syndrom X, Angiopathie, proliferative Retinopathie, Dawn-Phänomen, Nephropathie, MagensäureSekretion, Magengeschwüre, enterokutane Fisteln, pancreaticocutane Fisteln, Dumping-Syndrom, wäßriger Durchfall, Pankreatitis, gastrointestinale Hormon sezernierende Tumore, Angiogenese, Arthritis, Transplantatabstoßung, Transplantat-Gefäßblutungen, portale Hypertension, gastrointestinale Blutung, Fettleibigkeit und Opioidüberdosierung.

## Revendications

1. Composé de formule (I) :
(R¹R²)-AA¹-AA²-AA³-AA^{3b}-AA⁴-AA⁵-AA⁶-AA⁷-AA^{7b}-AA⁸-R⁵ (I)
ou un sel pharmaceutiquement acceptable de celui-ci,
où
l'azote α de AA¹, AA², AA³, AA^{3b}, AA⁴ AA⁵, AA⁶, AA⁷ AA^{7b} et AA⁸ est chacun, indépendamment, éventuellement substitué par un groupe alkyle en C₁ à C₄, alcényle en C₃ à C₄, alcynyle en C₃ à C₄ ou alkyl en C₁ à C₆-C(O)- ;
AA¹ est absent ou représente l'isomère D ou L d'un acide aminé choisi dans le groupe constitué de R¹, Aac, Aic, Arg, Asn, Asp, Dip, Gln, Glu, Hca, Hyp, Lys, Mac, Macab, Orn, Pro, Ser, Ser(Bzl), Thr, Thr(Bzl), Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, C4c, 5-Iqs, Htqa, 4-Mqc, Thn, α-Chpa, Cit, Nua, Pyp et d'un d'acide α-aminé aromatique éventuellement substitué ;
où ledit acide α-aminé aromatique éventuellement substitué est éventuellement substitué par un ou plusieurs substituants choisis chacun indépendamment dans le groupe constitué d'un atome d'halogène, d'un groupe NO₂, OH, CN, alkyle en C₁ à C₆, alcényle en C₂ à C₆, alcynyle en C₂ à C₆, alcoxy en C₁ à C₆, Bzl, O-Bzl, et NR⁹R¹⁰ ;
AA² est absent ou représente l'isomère D ou L d'un acide aminé choisi dans le groupe constitué de R¹¹, Aic, Arg, Hca, His, Hyp, Pal, F₅-Phe, Phe, Pro, Trp, et X⁰Phe Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, 1-Iqc, 3-Iqc, C4c, 5-Iqs, Htqa, 4-Mqc, Thn, α-Chpa, Cit, Nua, et Pyp ;
AA³ est absent ou représente l'isomère D ou L d'un acide aminé choisi dans le groupe constitué de Cys, hCys, Pen, Tpa, Tmpa, Mac, Macab, et d'un acide α-aminé aromatique éventuellement substitué ;
où ledit acide α-aminé aromatique éventuellement substitué est éventuellement substitué par un ou plusieurs substituants choisis chacun indépendamment dans le groupe constitué d'un atome d'halogène, d'un groupe NO₂, OH, CN, alkyle en C₁ à C₄, alcényle en C₂ à C₄, alcynyle en C₂ à C₄, alcoxy en C₁ à C₄, Bzl, O-Bzl, NR⁹R¹⁰, Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, 1-Iqc, 3-Iqc, C4c, 5-Iqs, Htqa, 4-Mqc, Thn, α-Chpa, Cit, Nua, et Pyp ; AA^{3b} est absent ou représente l'isomère D ou L d'un acide aminé choisi dans le groupe constitué de Pal, 4-Pal, His, Arg, Nal, Trp, Bpa, F₅-Phe, Phe, X⁰-Phe, R¹¹, hArg, Bip, Tic, Htic, Dip, Sala, Aala, Thza, Thia, Bal, Fala, et Pala ;
AA⁴ représente un isomère D ou L d'un acide aminé éventuellement substitué ou d'un acide α-aminé aromatique éventuellement substitué ;
où ledit acide aminé éventuellement substitué est choisi dans le groupe constitué de Trp, Lys, Orn, hLys, *cis*-4-Acha, *trans*-4-Acha, *trans-*4-Amcha, 4-Pip-Gly, N-Met-Trp, β-Met-Trp, His, hHis, hArg, Bip, Tic, Htic, Dip, Sala, Aala, Thza, Thia, Bal, Fala, Pala, et 4-Pip-Ala ;
où le groupe amino de la chaîne latérale dudit acide aminé éventuellement substitué est éventuellement substitué par R³ et R⁴ ; et
où ledit acide α-aminé aromatique éventuellement substitué est éventuellement substitué par un ou plusieurs substituants choisis chacun indépendamment dans le groupe constitué d'un atome d'halogène, d'un groupe NO₂, OH, CN, alkyle en C₁ à C₄, alcényle en C₂ à C₄, alcynyle en C₂ à C₄, Bzl, O-Bzl, et NR⁹R¹⁰ ;
AA⁵ est absent ou représente R¹¹, Aic, A3c, A4c, A5c, A6c, Abu, Aib, β-Ala, Bpa, Cha, Deg, Gaba, Ile, Leu, Nal, Nle, Pro, Sar, Ser, Ser(Bzl), Thr, Thr(Bzl), Trp, Val, Pal, F₅-Phe, Phe, X⁰-Phe, ou un isomère D ou L éventuellement substitué d'un acide aminé choisi dans le groupe constitué de 4-Pip-Gly, 4-Pip-Ala, *cis*-4-Acha, *trans*-4-Acha, *trans*-4-Amcha, hLys, Lys, Orn, hArg, Bip, Tic, Htic, Dip, Sala, Aala, Thza, Thia, Bal, Fala, et Pala ; où le groupe amino de la chaîne latérale dudit acide aminé éventuellement substitué est éventuellement mono- ou di-substitué par R³ et R⁴ ;
AA⁶ est absent ou représente l'isomère D ou L d'un acide aminé choisi dans le groupe constitué de R¹¹, un acide α-aminé aromatique éventuellement substitué, Cys, hCys, Pen, Tpa, Tmpa, Thr, Thr(Bzl), Ser, Ser(Bzl), hArg, Bip, Tic, Htic, Dip, Sala, Aala, Thza, Thia, Bal, Fala, et Pala ;
AA⁷ est absent ou représente l'isomère D ou L d'un acide aminé choisi dans le groupe constitué de R¹¹, un acide α-aminé aromatique éventuellement substitué, A3c, A4c, A5c, A6c, Abu, Aib, Aic, β-Ala, Arg, Cha, Deg, Gaba, Ile, Leu, Nle, Pip, Pro, Sar, Ser, Ser(Bzl), Thr, Thr(Bzl), Val, Tic, Htic, Sala, Aala, Thza, Thia, Bal, Fala, Pala, hArg, Bip, Bpa, Dip, Pal, Sala, et X⁰-Phe ;
AA^{7b} est absent ou représente l'isomère D ou L d'un acide aminé choisi dans le groupe constitué de R¹¹, Bpa, Phe, F₅-Phe, X⁰-Phe, Nal, Pro, Ser, Ser(Bzl), Thr, Thr(Bzl), Trp, hArg, Bip, Tic, Htic, Dip, Sala, Aala, Thza, Thia, Bal, Fala, et Pala ;
AA⁸ est absent ou représente l'isomère D ou L d'un acide aminé choisi dans le groupe constitué de R¹¹, Maa, Maaab, Thr, Thr(Bzl), Ser, Ser(Bzl), Tyr, Phe(4-O-Bzl), F₅-Phe, et X⁵-Phe, et d'un acide α-aminé aromatique éventuellement substitué ;
R¹ et R² représentent chacun indépendamment H, E-, E(O)₂S-, E(O)C-, EOOC-, R¹³, ou sont absents ;
R³ et R⁴ représentent chacun indépendamment un groupe alkyle en C₁ à C₁₂, alcényle en C₂ à C₁₂, alcynyle en C₂ à C₁₂, phényle, naphtyle, phényl-alkyle en C₁ à C₆, phényl-alcényle en C₂ à C₆, phényl-alcynyle en C₂ à C₆, naphtyl-alkyle en C₁ à C₆, naphtyl-alcényle en C₂ à C₆, naphtyl-alcynyle en C₂ à C₆, cycloalkyl en C₃ à C₇-alkyle en C₁ à C₆, cycloalkyl en C₃ à C₇-alcényle en C₂ à C₆, cycloalkyl en C₃ à C₇-alcynyle en C₂ à C₆, hétérocyclyl-alkyle en C₁ à C₄, hétérocyclyl-alcényle en C₂ à C₄, hétérocyclyl-alcynyle en C₂ à C₄, 1-adamantyle, 2-adamantyle, 9-fluorénylméthyle, dicyclopropylméthyle, diméthylcyclopropylméthyle, ou benzhydryle ;
R⁵ représente -OR⁶, -NR⁷R⁸, ou est absent,
où chaque R⁶, R⁷ et R⁸ représente indépendamment H, un groupe alkyle en C₁ à C₁₂, alcényle en C₂ à C₁₂, alcynyle en C₂ à C₁₂, phényle, naphtyle, phényl-alkyle en C₁ à C₆, phényl-alcényle en C₂ à C₆, phényl-alcynyle en C₂ à C₆, naphtyl-alkyle en C₁ à C₆, naphtyl-alcényle en C₂ à C₆, naphtyl-alcynyle en C₂ à C₆, 1-adamantyle, 2-adamantyle, 9-fluorénylméthyle, dicyclopropylméthyle, diméthylcyclopropylméthyle, ou benzhydryle ;
R⁹ et R¹⁰ représentent chacun indépendamment H, un groupe alkyle en C₁ à C₆, alcényle en C₃ à C₄, alcynyle en C₃ à C₄, 1-adamantyle ou 2-adamantyle ;
R¹¹ représente, indépendamment pour chaque apparition, un acide D-ou L-aminé de formule : 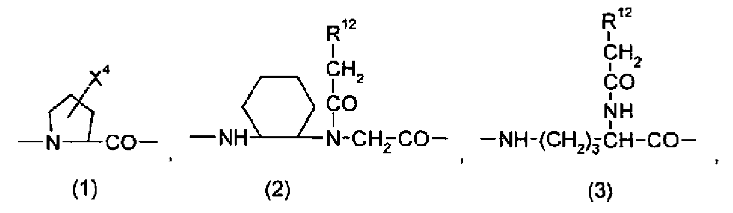 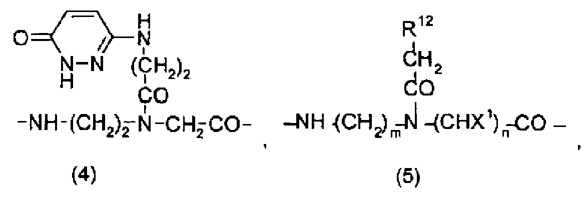 or 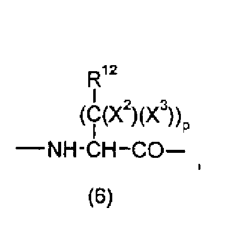 où m et n valent chacun indépendamment 1, 2 ou 3 et p vaut 0, 1 ou 2 ;
R¹² représente, indépendamment pour chaque apparition, un radical éventuellement substitué de formule : 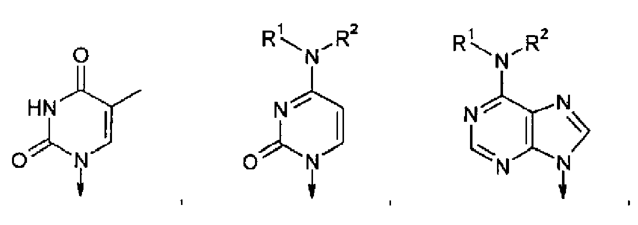 ou 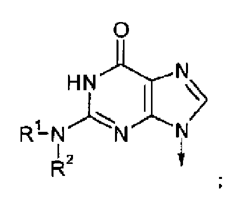 R¹³ représente un radical de formule 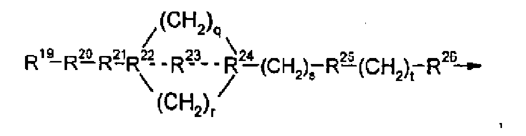 où chaque q, r, s et t valent chacun indépendamment 0, 1, 2, 3, 4 ou 5 ;
R¹⁹ est absent ou représente H, un groupe NH₂, OH, hydroxyalkyle en C₁ à C₆, N(R²⁷R²⁸), SO₃H, ou un radical éventuellement substitué choisi dans le groupe constitué des radicaux hétérocyclyle, phényle et naphtyle,
où le radical éventuellement substitué défini pour R¹⁹ est éventuellement substitué par un ou plusieurs substituants choisis, indépendamment pour chaque apparition, dans le groupe constitué d'un atome d'halogène, un groupe NO₂, OH, alkyle en C₁ à C₆, alcényle en C₂ à C₆, alcynyle en C₂ à C₆, alcoxy en C₁ à C₆, NH₂, mono- ou di-alkyl en C₁ à C₆-amino, Bzl et O-Bzl ;
R²⁰ représente O ou est absent ;
R²¹ représente un groupe alkyle en C₁ à C₆ ou est absent ;
R²² représente N, O, C ou CH ;
R²³ représente un groupe alkyle en C₁ à C₆ ou est absent ;
R²⁴ représente N, CH ou C ;
R²⁵ représente NH, O ou est absent ;
R²⁶ représente SO₂, CO ou CH ;
R²⁷ et R²⁸ représentent chacun indépendamment H ou un groupe alkyle en C₁ à C₆ ;
E représente, indépendamment pour chaque apparition, un radical éventuellement substitué choisi dans le groupe constitué des radicaux alkyle en C₁ à C₁₂, alcényle en C₂ à C₁₂, alcynyle en C₂ à C₁₂, phényle, naphtyle, phényl-alkyle en C₁ à C₆, phényl-alcényle en C₂ à C₆, phényl-alcynyle en C₂ à C₆, naphtyl-alkyle en C₁ à C₆, naphtyl-alcényle en C₂ à C₆, naphtyl-alcynyle en C₂ à C₆, cycloalkyl en C₃ à C₇-alkyle en C₁ à C₆, cycloalkyl en C₃ à C₇-alcényle en C₂ à C₆, cycloalkyl en C₃ à C₇-alcynyle en C₂ à C₆, hétérocyclyl-alkyle en C₁ à C₄, hétérocyclyl-alcényle en C₂ à C₄, hétérocyclyl-alcynyle en C₂ à C₄, 1-adamantyle, 2-adamantyle, dicyclopropylméthyle, diméthylcyclopropylméthyle, 9-fluorénylméthyle, et benzhydryle ;
où le radical éventuellement substitué défini pour E est éventuellement substitué par un ou plusieurs substituants choisis chacun indépendamment dans le groupe constitué d'un atome d'halogène, OH, Bzl, O-Bzl, NO₂, CN, COOH, et SH ;
X⁰ représente un atome d'halogène, un groupe NO₂, OH, alkyle en C₁ à C₆, alcoxy en C₁ à C₆, mono- or di-alkyl en C₁ à C₆-amino, Bzl, O-Bzl, NR⁹R¹⁰, ou CN ;
X¹ représente H, un groupe alkyle en C₁ à C₆, alcényle en C₂ à C₆, alcynyle en C₂ à C₆, indolyle, imidazolyle, 1-naphtyle, 3-pyridyle, benzyle à cycle éventuellement substitué, ou un radical qui correspond au groupe de la chaîne latérale de Arg, Leu, Gln, Lys, Tyr, His, Thr, Trp, Phe, Val, Ala, Lys, ou His ;
où ledit groupe benzyle à cycle éventuellement substitué est éventuellement substitué par un ou plusieurs substituants choisis dans le groupe constitué d'un atome d'halogène, d'un groupe OH, alcoxy en C₁ à C₆, mono- ou di-alkyl en C₁ à C₆-amino, alkyle en C₁ à C₄, alcényle en C₂ à C₄, alcynyle en C₂ à C₄, et NR⁹R¹⁰ ;
X² et X³ représentent chacun indépendamment H, un atome d'halogène, un groupe OH, =O, =S, alkyle en C₁ à C₁₂, alcényle en C₂ à C₁₂, alcynyle en C₂ à C₁₂, phényle, naphtyle, phényl-alkyle en C₁ à C₆, phényl-alcényle en C₂ à C₆, phényl-alcynyle en C₂ à C₆, naphtyl-alkyle en C₁ à C₆, naphtyl-alcényle en C₂ à C₆, naphtyl-alcynyle en C₂ à C₆, cycloalkyl en C₃ à C₇-alkyle en C₁ à C₆, cycloalkyl en C₃ à C₇-alcényle en C₂ à C₆, cycloalkyl en C₃ à C₇-alcynyle en C₂ à C₆, hétérocyclyl-alkyle en C₁ à C₄, hétérocyclyl-alcényle en C₂ à C₄, hétérocyclyl-alcynyle en C₂ à C₄, 1-adamantyle, 2-adamantyle, dicyclopropylméthyle ou diméthylcyclopropylméthyle ;
X⁴ représente H, OH ou NH₂ ; et
X⁵ représente un atome d'halogène, NO₂, CH₃, OH, Bzl ou O-Bzl ;
à condition que :
au moins six résidus d'acides aminés soient présents ;
lorsque AA³ représente un isomère D ou L d'un acide aminé choisi dans le groupe constitué de Cys, hCys, Pen, Tpa, ou Tmpa, et AA⁶ représente un isomère D ou L d'un acide aminé choisi dans le groupe constitué de Cys, hCys, Pen, Tpa, ou Tmpa, alors AA³ et AA⁶ sont reliés par une liaison disulfure ;
lorsque AA¹ ou AA³ représente un isomère D ou L d'un acide aminé choisi dans le groupe constitué de Mac ou Macab, alors AA⁸ est un isomère D ou L d'un acide aminé choisi dans le groupe constitué de Maa et Maaab, et lorsque AA⁸ représente un isomère D ou L d'un acide aminé choisi dans le groupe constitué de Maa et Maaab, alors AA¹ ou AA³ représente un isomère D ou L de Mac ou de Macab, et AA¹ ou AA³ est relié par une liaison disulfure avec AA⁸ ;
AA² puisse représenter D- ou L-Hca uniquement lorsque AA¹ est absent ;
lorsque l'un de R¹ ou R² représente E(O)₂S-, E(O)C-, EOOC-, ou R¹³, l'autre représente H ;
lorsque R⁵ est absent, alors l'un de R¹ ou R² est également absent, et l'acide aminé N-terminal et l'acide aminé C-terminal forment ensemble une liaison amide ;
lorsque l'un de X² ou X³ représente C=O ou C=S, l'autre est absent ; et
ledit composé de formule (I) n'est pas de formule :
D-Phe-Tyr-cyclo(D-Cys-D-Trp-Lys-Cys)-Abu-Thr-NH₂ ;
Ac-Phe-Tyr-cyclo(D-Cys-D-Trp-Lys-Cys)-Abu-Thr-NH₂ ;
L-4-NO₂-Phe-Tyr-cyclo(D-Cys-D-Trp-Lys-Cys)-Abu-Thr-NH₂ ;
Ac-L-4-NO₂-Phe-Tyr-cyclo(D-Cys-D-Trp-Lys-Cys)-Abu-Thr-NH₂ ;
Hca-Tyr-cyclo(D-Cys-D-Trp-Lys-Cys)-Abu-Thr-NH₂ ;
D-Dip-Tyr-cyclo(Cys-D-Trp-Lys-D-Cys)-Val-Nal-NH₂ ;
D-4-NO₂-Phe-Phe(4-O-Bzl)-cyclo(D-Cys-D-Trp-Lys-Cys)Cha-Nal-NH₂ ; ou
D-4-NO₂-Phe-cyclo(D-Cys-Phe(4-O-Bzl)-D-Trp-Lys-Cys)-Val-Tyr-NH₂.

2. Composé selon la revendication 1, où ledit composé est de formule (II) : 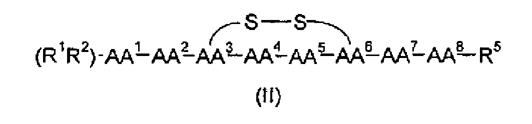 ou un sel pharmaceutiquement acceptable de celui-ci,
où
AA¹ est absent ou représente l'isomère D ou L d'un acide aminé choisi dans le groupe constitué de R¹¹, Aac, Aic, Arg, Asn, Asp, Dip, Gln, Glu, Hyp, Lys, Mac, Macab, Orn, Pip, Pro, Ser, Ser(Bzl), Thr, Thr(Bzl), Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, 1-Iqc, 3-lqc, C4c, 5-Iqs, Htqa, 4-Mqc, Thn, α-Chpa, Cit, Nua, Pyp et d'un d'acide α-aminé aromatique éventuellement substitué ;
où ledit acide α-aminé aromatique éventuellement substitué est éventuellement substitué par un ou plusieurs substituants choisis chacun indépendamment dans le groupe constitué d'un atome d'halogène, d'un groupe NO₂, OH, CN, alkyle en C₁ à C₆, alcényle en C₂ à C₆, alcynyle en C₂ à C₆, et N R⁹R¹⁰ ;
AA² est absent ou représente l'isomère D ou L d'un acide aminé choisi dans le groupe constitué de R¹¹, Aic, Arg, Hca, His, Hyp, Pal, F₅-Phe, Phe, Pro, Trp, X⁰-Phe, Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, 1-Iqc, 3-Iqc, C4c, 5-Iqs, Htqa, 4-Mqc, Thn, α-Chpa, Cit, Nua, et Pyp ; AA³ représente l'isomère D ou L d'un acide aminé choisi dans le groupe constitué de Cys, hCys, Pen, Tpa et Tmpa ;
AA⁴ représente un isomère D ou L d'un acide aminé choisi dans le groupe constitué de Trp, N-Met-Trp, β-Met-Trp, His, hHis, hArg, Bip, Tic, Htic, Dip, Sala, Aala, Thza, Thia, Bal, Fala, Pala, et d'un d'acide α-aminé aromatique éventuellement substitué ;
où ledit acide α-aminé aromatique éventuellement substitué est éventuellement substitué par un ou plusieurs substituants choisis chacun indépendamment dans le groupe constitué d'un atome d'halogène, d'un groupe NO₂, OH, alkyle en C₁ à C₄, alcényle en C₂ à C₄, alcynyle en C₂ à C₄, Bzl, O-Bzl et NR⁹R¹⁰.
AA⁵ représente un isomère D ou L d'un acide aminé choisi dans le groupe constitué de 4-Pip-Gly, 4-Pip-Ala, cis-4-Acha, trans-4-Acha, trans-4-Amcha, hLys, Lys, Orn, hArg, Bip, Tic, Htic, Dip, Sala, Aala, Thza, Thia, Bal, Fala, et Pala,
où le groupe amino de la chaîne latérale dudit acide aminé éventuellement substitué est éventuellement mono- ou di-substitué par R³ et R⁴ ;
AA⁶ représente un isomère D ou L d'un acide aminé choisi dans le groupe constitué de Cys, hCys, Pen, Tpa, et Tmpa ;
AA⁷ est absent ou représente un isomère D ou L d'un acide aminé choisi dans le groupe constitué de R¹¹, Aic, A3c, A4c, A5c, A6c, Abu, Aib, β-Ala, Arg, Bpa, Cha, Deg, Gaba, His, Ile, Leu, Nal, Nle, Pal, Phe, F₅-Phe, Pro, Sar, Ser, Ser(Bzl), Thr, Thr(Bzl), Trp, N-Me-Trp, Val, N-Me-Val, hArg, Bip, Tic, Htic, Dip, Sala, Aala, Thza, Thia, Bal, Fala, Pala, et X³-Phe ;
AA⁸ est absent ou représente un isomère D ou L d'un acide aminé choisi dans le groupe constitué de R¹¹, un acide α-aminé aromatique éventuellement substitué, Maa, Maaab, Ser, Ser(Bzl), Thr, Thr(Bzl), Tyr, Phe(4-O-Bzl), F₅-Phe, et X⁵-Phe ;
R¹³ représente un radical selon la formule 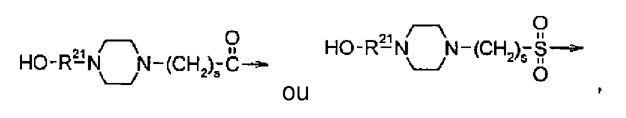 où R²¹ représente un groupe alkyle en C₁ à C₄ et s vaut 1, 2, 3 ou 4 ; et
X⁰ représente un atome d'halogène, NO₂, CH₃, OH, Bzl, O-Bzl ou CN ;
à condition qu'au moins l'un de AA⁷ ou AA⁸ soit présent.

3. Composé selon la revendication 1, où ledit composé est de formule (III) : 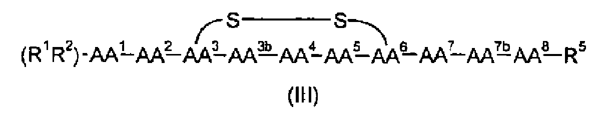 ou un sel pharmaceutiquement acceptable de celui-ci,
où
AA¹ est absent ou représente l'isomère D ou L d'un acide aminé choisi dans le groupe constitué de R¹¹, Aac, Aic, Arg, Asn, Asp, Gln, Glu, Hca, His, Hyp, Lys, Mac, Macab, Orn, Pro, Ser, Ser(Bzl), Thr, Thr(Bzl), Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, 1-Iqc, 3-Iqc, C4c, 5-Iqs, Htqa, 4-Mqc, Thn, α-Chpa, Cit, Nua, Pyp et d'un d'acide α-aminé aromatique éventuellement substitué ;
où ledit acide α-aminé aromatique éventuellement substitué est éventuellement substitué par un ou plusieurs substituants choisis chacun indépendamment dans le groupe constitué d'un atome d'halogène, d'un groupe NO₂, OH, CN, alkyle en C₁ à C₆, alcényle en C₂ à C₆, alcynyle en C₂ à C₆, et NR⁹R¹⁰ ;
AA³ représente un isomère D ou L d'un acide aminé choisi dans le groupe constitué de Cys, hCys, Pen, Tpa, et Tmpa ;
AA^{3b} représente l'isomère D ou L d'un acide aminé choisi dans le groupe constitué de R¹¹, Arg, Bpa, F₅-Phe, His, Nal, Pal, 4-Pal, Phe, Trp, hArg, Bip, Tic, Htic, Dip, Sala, Aala, Thza, Thia, Bal, Fala, Pala, et X⁵-Phe ;
AA⁴ représente un isomère D ou L d'un acide aminé choisi dans le groupe constitué de Trp, N-Met-Trp, β-Met-Trp, His, hHis, hArg, Bip, Tic, Htic, Dip, Sala, Aala, Thza, Thia, Bal, Fala, Pala, et d'un d'acide α-aminé aromatique éventuellement substitué ;
où ledit acide α-aminé aromatique éventuellement substitué est éventuellement substitué par un ou plusieurs substituants choisis chacun indépendamment dans le groupe constitué d'un atome d'halogène, d'un groupe NO₂, OH, CN, alkyle en C₁ à C₄, alcényle en C₂ à C₄, alcynyle en C₂ à C₄, Bzl, O-Bzl et NR⁹R¹⁰ ;
AA⁵ représente un isomère D ou L d'un acide aminé choisi dans le groupe constitué de 4-Pip-Gly, 4-Pip-Ala, cis-4-Acha, trans-4-Acha, trans-4-Amcha, hLys, Lys et Orn, et, hArg, Bip, Tic, Htic, Dip, Sala, Aala, Thza, Thia, Bal, Fala, Pala,
où le groupe amino de la chaîne latérale dudit acide aminé est éventuellement mono- ou di-substitué par R³ et R⁴ ;
AA⁶ représente un isomère D ou L d'un acide aminé choisi dans le groupe constitué de Cys, hCys, Pen, Tpa, et Tmpa ;
AA⁷ est absent ou représente un isomère D ou L d'un acide aminé choisi dans le groupe constitué de R¹¹, Aic, A3c, A4c, A5c, A6c, Abu, Aib, β-Ala, Arg, Bpa, Cha, Deg, Gaba, His, Ile, Leu, Nal, Nle, Pal, Phe, F₅-Phe, Pro, Sar, Ser, Ser(Bzl), Thr, Thr(Bzl), Trp, N-Me-Trp, Val, N-Me-Val, hArg, Bip, Tic, Htic, Dip, Sala, Aala, Thza, Thia, Bal, Fala, Pala, et X⁰-Phe ;
X⁰ représente un atome d'halogène, NO₂, CH₃, OH, CN, Bzl ou O-Bzl ;
R¹ et R² représentent chacun indépendamment H, E-, E(O)₂S-, E(O)C-, EOOC-, R¹³, ou sont absents ;
R⁵ représente -OR⁶ ou -NR⁷R⁸ ;
R¹³ représente un radical de formule 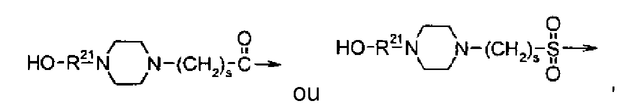 où R²¹ représente un groupe alkyle en C₁ à C₄ et s vaut 1, 2, 3 ou 4 ;
à condition que :
au moins l'un de AA¹ ou AA² soit présent ;
lorsque AA¹ représente un isomère D ou L de Pro, Hyp, Arg, Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, 1-Iqc, 3-Iqc, C4c, 5-Iqs, Htqa, 4-Mqc, Thn, α-Chpa, Cit, Nua, Pyp ou His, AA² ne puisse pas représenter un isomère D ou L de Pro, Hyp, Arg, Pip, hArg, Bip, Bpa, Tic, Cmp, Inc, Inp, Nip, Ppc, Htic, Thi, Tra, Cmpi, Tpr, lia, Alla, Aba, Gba, Car, Ipa, laa, Inip, Apa, Mim, Thnc, Sala, Aala, Thza, Thia, Bal, Fala, Pala, Dap, Agly, Pgly, Ina, Dipa, Mnf, Inic, 1-Iqc, 3-Iqc, C4c, 5-Iqs, Htqa, 4-Mqc, Thn, α-Chpa, Cit, Nua, Pyp ou His ;
lorsque AA⁷ représente un isomère D ou L de Thr ou de Ser, AA⁸ ne puisse pas représenter un isomère D ou L de Thr ou de Ser ;
au moins l'un de AA¹, AA², AA^{3b}, AA⁷ AA^{7b} ou AA⁸ représente l'isomère D ou L de R¹¹ ; et
lorsque l'un de X² ou X³ représente =O ou =S, l'autre soit absent ;
ou un sel pharmaceutiquement acceptable de celui-ci.

4. Composé selon la revendication 1, où ledit composé est de formule (IV) : 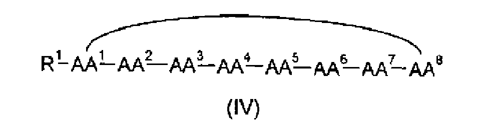 où
AA¹ est absent ou représente l'isomère D ou L d'un acide aminé choisi dans le groupe constitué de R¹¹, Aic, Hyp, Pro, Ser, Ser(Bzl), Thr, Thr(Bzl), Tic, Htic, Fala et d'un d'acide α-aminé aromatique éventuellement substitué ;
où ledit acide α-aminé aromatique éventuellement substitué est éventuellement substitué par un ou plusieurs substituants choisis chacun indépendamment dans le groupe constitué d'un atome d'halogène, d'un groupe NO₂, OH, CN, alkyle en C₁ à C₆, alcényle en C₂ à C₆, alcynyle en C₂ à C₆, alcoxy en C₁ à C₆, Bzl, O-Bzl et NR⁹R¹⁰ ;
AA² est absent ou représente l'isomère D ou L d'un acide aminé choisi dans le groupe constitué de R¹¹, Arg, F₅-Phe, His, Pal, Phe, Trp, hArg, Pala, Bal, Fala, Sala et X⁰-Phe ;
AA³ représente l'isomère D ou L d'un acide α-aminé aromatique éventuellement substitué, où ledit acide α-aminé aromatique éventuellement substitué est éventuellement substitué par un ou plusieurs substituants choisis chacun indépendamment dans le groupe constitué d'un atome d'halogène, d'un groupe NO₂, OH, CN, alkyle en C₁ à C₄, alcényle en C₂ à C₄, alcynyle en C₂ à C₄, Bzl, O-Bzl et NR⁹R¹⁰ ;
AA⁴ représente un isomère D ou L d'un acide aminé éventuellement substitué choisi dans le groupe constitué de Trp, N-Met-Trp, β-Me-Trp, Lys, Orn, hLys, *cis*-4-Acha, *trans*-4-Acha, *trans*-4-Amcha, 4-Pip-Gly, 4-Pip-Ala, hArg, Bip, Tic, Htic, Dip, Sala, Aala, Thza, Thia, Bal, Fala, et Pala ;
où le groupe amino de la chaîne latérale dudit acide aminé éventuellement substitué est éventuellement substitué par R³ et R⁴ ;
AA⁵ est absent ou représente un isomère D ou L de R¹¹, A3c, A4c, A5c, A6c, Abu, Aib, Aic, β-Ala, Bpa, Cha, Deg, F₅-Phe, Gaba, Ile, Leu, Nal, Nle, Pal, Phe, Pro, Sar, Ser, Ser(Bzl), Thr, Thr(Bzl), Trp, N-Me-Trp, Val, N-Me-Val, hArg, Bip, Tic, Htic, Dip, Sala, Aala, Thza, Thia, Bal, Fala, Pala, ou X⁰-Phe ;
AA⁶ est absent ou représente l'isomère D ou L de R¹¹, un acide α-aminé aromatique, F₅-Phe, Phe, Thr, Thr(Bzl), Ser, Ser(Bzl), ou X⁰-Phe ;
AA⁷ est absent ou représente l'isomère D ou L de R¹¹ ou l'isomère D ou L d'un acide α-aminé aromatique ;
AA⁸ représente un isomère D ou L de R¹¹ ;
R¹ représente H, E-, E(O)₂S-, E(O)C-, EOOC-, ou R¹³ ;
R¹³ représente un radical de formule 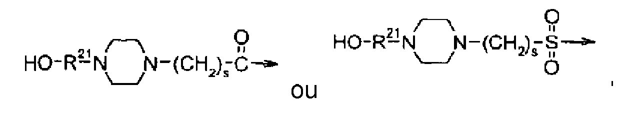 où R²¹ représente un groupe alkyle en C₁ à C₄ et s vaut 1, 2, 3 ou 4 ; X⁰ dans la définition de AA² et AA⁵ représente un atome d'halogène, un groupe NO₂, OH, alkyle en C₁ à C₆, alcoxy en C₁ à C₆, mono- ou di-alkyl en C₁ à C₆-amino, Bzl ou O-Bzl ;
X⁰ dans la définition de AA⁶ représente un atome d'halogène, un groupe NO₂, OH, alkyle en C₁ à C₆, alcoxy en C₁ à C₆, mono- ou di-alkyl en C₁ à C₆-amino, Bzl, O-Bzl ou NR⁹R¹⁰ ;
à condition que :
au moins l'un de AA¹ ou AA² soit présent ;
lorsque AA¹ est absent, AA² et AA⁸ forment ensemble une liaison ; et
au moins deux de AA⁵, AA⁶ et AA⁷ soient présents ;
ou un sel pharmaceutiquement acceptable de celui-ci.

5. Composé selon la revendication 2, où
AA¹ est absent ou représente Ac-D-Phe, ou l'isomère D ou L de R¹¹, Pip, Pro, ou Ser, ou d'un acide α-aminé aromatique choisi dans le groupe constitué de Cpa, Dip, Nal, Pal, et Phe ; 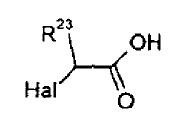 AA² est absent ou représente Aic, Pal, Phe, F₅-Phe, 4-NO₂-Phe, Trp, Tyr, Phe(4-O-Bzl) 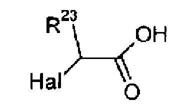 AA³ représente l'isomère D ou L d'un acide aminé choisi dans le groupe constitué de Pen, Cys, hCys et Tmpa ;
AA⁴ représente l'isomère D ou L de Trp, His, N-Me-Trp, β-Me-Trp, hTrp, ou hHis ;
AA⁵ représente Lys, hLys, N-Me-Lys, Orn, cis-4-Acha ou 4-Pip-Ala ;
AA⁶ représente l'isomère D ou L d'un acide aminé choisi dans le groupe constitué de Cys, hCys, Pen et Tmpa ;
AA⁷ représente A3c, A4c, A5c, A6c, Abu, Aic, β-Ala, Gaba, Nle, F₅-Phe, Phe, Pro, Sar, Ser, Thr, Thr(Bzl), Tyr, Val ou est absent ; et
AA⁸ représente R¹¹, Nal, Thr, Thr(Bzl), Tyr, Phe(4-O-Bzl) ou est absent ;
ou un sel pharmaceutiquement acceptable de celui-ci.

6. Composé selon la revendication 5, où
AA¹ est absent ou représente l'isomère D ou L de R¹¹, Pip ou Pro, ou d'un acide α-aminé aromatique choisi dans le groupe constitué de Cpa, Dip, Nal, Pal, Phe et Ac-Phe ;
AA² représente Tyr, Pal, Phe, 4-NO₂-Phe, Trp ou est absent ;
AA³ représente l'isomère D ou L de Cys ou Pen ;
AA⁴ représente D-Trp ;
AA⁵ représente Lys, Orn ou *cis*-4-Acha ;
AA⁶ représente un isomère D ou L de Cys ou Pen ;
AA⁷ représente A3c, A4c, A5c, A6c, Abu, Aic, β-Ala, Gaba, Nle, Phe, Pro, Sar, Thr, Thr(Bzl), Tyr, Val ou est absent ; et
AA⁸ représente R¹¹, Thr, Tyr, Nal ou est absent ;
ou un sel pharmaceutiquement acceptable de celui-ci.

7. Composé selon la revendication 3, où
AA¹ représente R¹¹, Aic, Hca, Pro, Ser, Ser(Bzl), Trp, Tyr, ou un isomère D ou L d'un acide α-aminé aromatique choisi dans le groupe constitué de Cpa, Nal, Ac-Nal, Phe, Ac-Phe, 4-NO₂-Phe, et Ac-4-NO₂-Phe ;
AA² représente Pal, Phe, F₅-Phe, Tyr, ou est absent ;
AA³ représente un isomère D ou L de Cys, hCys, Pen ou Tmpa ; AA^{3b} représente Pal, 4-Pal, His, Trp, Tyr, Phe(4-O-Bzl), Phe, ou R¹¹ ; AA⁴ représente un isomère D ou L de Trp ou His ;
AA⁵ représente Lys, N-Me-Lys, Orn, hLys, *cis*-4-Acha, ou 4-Pip-Ala ;
AA⁶ représente un isomère D ou L de Cys, hCys, Pen ou Tmpa ;
AA⁷ représente R¹¹, A4c, A5c, Abu, β-Ala, Gaba, Phe, F₅-Phe, Ser(Bzl), Thr, Thr(Bzl), Phe(4-O-Bzl) ou est absent ;
AA^{7b} représente R¹¹, Nal, F₅-Phe, X⁰-Phe ou est absent, ou X⁰ représente un atome d'halogène, un groupe NO₂, CH₃, OH, Bzl ou O-Bzl ; et
AA⁸ représente R¹¹, Nal, Tyr, Phe(4-O-Bzl) ou est absent ;
ou un sel pharmaceutiquement acceptable de celui-ci.

8. Composé selon la revendication 7, où
AA¹ représente R¹¹, Aic, Hca, Pro, Ser(Bzl), ou un isomère D ou L d'un acide α-aminé aromatique choisi dans le groupe constitué de Cpa, Nal, Ac-Nal, Phe, Ac-Phe, 4-NO₂-Phe, et Ac-4-NO₂-Phe ;
AA² représente Pal, Tyr, ou est absent ;
AA³ représente un isomère D ou L de Cys ou Pen ;
AA^{3b} représente R¹¹, Pal, 4-Pal, Trp, Tyr, Phe(4-O-Bzl) ou Phe, où R¹¹ représente (T)aeg ;
AA⁴ représente D-Trp ;
AA⁵ représente Lys, N-Me-Lys, Orn ou *cis*-4-Acha ;
AA⁶ représente un isomère D ou L de Cys ou Pen ;
AA⁷ représente R¹¹, A5c, Abu, Ser(Bzl), Thr, Thr(Bzl), Phe(4-O-Bzl), Gaba, ou est absent ;
AA^{7b} représente Nal, X⁰-Phe ou est absent ; et
AA⁸ représente Tyr ou est absent ;
ou un sel pharmaceutiquement acceptable de celui-ci.

9. Composé selon la revendication 4, où
AA¹ représente Aic, Hyp, Cpa, D-Cpa, Nal, Pal, Phe, Pro, R¹¹, Tyr ou est absent ;
AA² représente Phe, Trp, F₅-Phe, His, Tyr, Phe(4-O-Bzl) ou R¹¹ ;
AA³ représente un isomère D de Trp, His ou Pal ;
AA⁴ représente Lys, N-Me-Lys, Orn, hLys, cis-4-Acha, ou 4-Pip-Ala ;
AA⁵ représente Pal, Phe(4-O-Bzl), Thr(Bzl), Thr, Sar, Gaba, β-Ala, A4c, A5c, A6c, Abu, Aic ou est absent ;
AA⁶ représente Thr, Tyr, Ser, F₅-Phe, Cpa, Nal, ou D- ou L-Phe ; AA⁷ représente Nal, Pal ou est absent ; et
AA⁸ représente R¹¹ ;
ou un sel pharmaceutiquement acceptable de celui-ci.

10. Composé selon la revendication 9, où
AA¹ représente Cpa, Nal, Pal, Phe, Tyr ou est absent ;
AA² représente Phe, Tyr, Trp ou R¹¹ ;
AA³ représente D-Trp ;
AA⁴ représente Lys, N-Me-Lys ou *cis*-4-Acha ;
AA⁵ représente Pal, Phe(4-O-Bzl), Aic, Gaba, A5c ou est absent ; AA⁶ représente Thr, Nal, ou D- ou L-Phe ;
AA⁷ est absent ; et
AA⁸ représente R¹¹ ;
ou un sel pharmaceutiquement acceptable de celui-ci.

11. Composé selon la revendication 2, où R¹ et R⁵ sont absents et l'acide aminé N-terminal et l'acide aminé C-terminal forment ensemble une liaison amide ; ou un sel pharmaceutiquement acceptable de celui-ci.

12. Composé selon la revendication 3, où R¹ et R⁵ sont absents et l'acide aminé N-terminal et l'acide aminé C-terminal forment ensemble une liaison amide ; ou un sel pharmaceutiquement acceptable de celui-ci.

13. Composé selon la revendication 6, où ledit composé est de formule :
Ac-D-Phe-Tyr-cyclo(D-Cys-D-Trp-Lys-Cys)-Abu-Thr-NH₂;
Nal-Tyr-cyclo(Cys-D-Trp-Lys-D-Cys)-Val-Nal-NH₂;
Nal-Tyr-cyclo(Cys-D-Trp-Lys-D-Cys)-Abu-Nal-NH₂;
D-Dip-Tyr-cyclo(Cys-D-Trp-Lys-D-Cys)-Abu-Nal-NH₂;
Dip-Tyr-cyclo(D-Cys-D-Trp-Lys-D-Cys)-Abu-Nal-NH₂;
Nal-Tyr-cyclo(D-Cys-D-Trp-Lys-D-Cys)-Abu-Nal-NH₂:
Dip-Tyr-cyclo(D-Cys-D-Trp-Lys-D-Cys)-Val-Nal-NH₂;
Nal-Tyr-cyclo(D-Cys-D-Trp-Lys-D-Cys)-Val-Nal-N H₂;
cyclo(D-Phe-Tyr-cyclo(D-Cys-D-Trp-Lys-Cys)-Abu-Thr);
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp-Lys-D-Cys)-A6c-Nal-NH₂;
(G(z))aeg-cyclo(D-Cys-D-Trp-Lys-D-Cys)-A5c-Nal-NH₂;
Pal-cyclo(D-Cys-D-Trp-Lys-D-Cys)-A5c-Nal-NH₂;
Cpa-Pal-cyclo(D-Cys-D-Trp-Lys-D-Cys)-β-Ala-Nal-NH₂;
Cpa-Pal-cyclo(D-Cys-D-Trp-Lys-D-Cys)-Sar-Nal-NH₂:
Cpa-Pal-cyclo(D-Cys-D-Trp-Lys-D-Cys)-Gaba-Nal-NH₂;
Cpa-Pal-cyclo(D-Cys-D-Trp-Lys-D-Cys)-Pro-Nal-NH₂;
Pro-Phe-c(D-Cys-D-Trp-Lys-D-Cys)-Nle-Phe-NH₂;
Pro-Phe-c(D-Cys-D-Trp-Lys-D-Cys)-Thr-Nle-NH₂;
Pro-Phe-c(D-Cys-D-Trp-Lys-D-Cys)-Thr-Phe-NH₂;
Cpa-Phe-c(D-Cys-D-Trp-Lys-D-Cys)-Gaba-NH₂;
Cpa-Phe-c(D-Cys-D-Trp-Lys-D-Cys)-Gaba-Tyr-NH₂;
Pip-Phe-c(D-Cys-D-Trp-Lys-D-Cys)-NH₂;
Pip-Phe-c(Cys-D-Trp-Lys-Cys)-Gaba-NH₂; ou
Pro-Phe-c(D-Cys-D-Trp-Lys-D-Cys)-Thr-NH₂;
ou un sel pharmaceutiquement acceptable de celui-ci.

14. Composé selon la revendication 6, où ledit composé est selon la formule :
Phe-cyclo(Cys-D-Trp-Lys-Cys)-Thr-NH₂;
Phe-Tyr-cyclo(D-Cys-D-Trp-Lys-Cys)-Abu-Thr-NH₂;
Ac-D-Phe-Tyr-cyclo(D-Cys-D-Trp-Lys-Cys)-Abu-Thr-NH₂;
Nal-Tyr-cyclo(Cys-D-Trp-Lys-D-Cys)-Val-Nal-NH₂;
Nal-Tyr-cyclo(Cys-D-Trp-Lys-D-Cys)-Abu-Nal-NH₂;
Dip-Tyr-cyclo(D-Cys-D-Trp-Lys-D-Cys)-Abu-Nal-NH₂;
Nal-Tyr-cyclo(D-Cys-D-Trp-Lys-D-Cys)-Abu-Nal-NH₂;
Dip-Tyr-cyclo(D-Cys-D-Trp-Lys-D-Cys)-Val-Nal-NH₂;
Nal-Tyr-cyclo(D-Cys-D-Trp-Lys-D-Cys)-Val-Nal-NH₂;
Cpa-Pal-cyclo(D-Cys-D-Trp-Lys-D-Cys)-A3c-Nal-NH₂;
Cpa-Pal-cyclo(D-Cys-D-Trp-Lys-D-Cys)-A5c-Nal-NH₂;
Cpa-Pal-cyclo(D-Cys-D-Trp-Lys-D-Cys)-A6c-Nal-NH₂;
(G(z))aeg-cyclo(D-Cys-D-Trp-Lys-D-Cys)-A5c-Nal-NH₂;
D-Cpa-cyclo(Cys-D-Trp-Lys-D-Cys)-A5c-Nal-NH₂;
Pal-cyclo(D-Cys-D-Trp-Lys-D-Cys)-A5c-Nal-NH₂;
Cpa-cyclo(D-Cys-D-Trp-Lys-D-Cys)-A5c-Nal-NH₂;
Cpa-Pal-cyclo(D-Cys-D-Trp-Lys-D-Cys)-β-Ala-Nal-NH₂:
Cpa-Pal-cyclo(D-Cys-D-Trp-Lys-D-Cys)-Sar-Nal-NH₂;
Cpa-Pal-cyclo(D-Cys-D-Trp-Lys-D-Cys)-Aic-Nal-NH₂;
Cpa-Pal-cyclo(D-Cys-D-Trp-Lys-D-Cys)-Gaba-Nal-NH₂;
Cpa-Pal-cyclo(D-Cys-D-Trp-Lys-D-Cys)-Pro-Nal-NH₂;
(T)aeg-cyclo(D-Cys-O-Trp-Lys-D-Cys)-(A)aeg-NH₂;
Cpa-Pal-cyclo(D-Cys-D-Trp-Lys-D-Cys)-A4c-Nal-NH₂;
Cpa-Pal-cyclo(D-Cys-D-Trp-Lys-D-Cys)-Nal-NH₂;
Pal-cyclo(D-Cys-D-Trp-Lys-D-Cys)-Nal-NH₂;
Pro-Phe-cyclo(Cys-D-Trp-Lys-D-Cys)-Val-NH₂;
Pro-Phe-cyclo(D-Cys-D-Trp-Lys-Cys)-Val-NH₂;
Pip-4-NO2-Phe-cyclo(D-Cys-D-Trp-Lys-D-Cys)-Nie-NH₂;
(G)aeg-Pal-cyclo(D-Cys-D-Trp-Lys-D-Cys)-Thr(Bzl)-(C)aeg-NH₂; ou
(C)aeg-Pal-cyclo(D-Cys-D-Trp-Lys-D-Cys)-Thr(Bzl)-(G)aeg-NH₂;
ou un sel pharmaceutiquement acceptable de celui-ci.

15. Composé selon la revendication 8, où ledit composé est selon la formule
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂;
D-Phe-cyclo(Cys-Tyr-D-Trp-Lys-Cys)-Thr-NH₂;
D-4-NO₂-Phe-cyclo(D-Cys-Tyr-D-Trp-Lys-Cys)-Nal-NH₂;
Ac-D-4-NO₂-Phe-cyclo(D-Cys-Tyr-D-Trp-Lys-Cys)-Nal-NH₂;
D-4-NO₂-Phe-Pal-cyclo(D-Cys-Phe(4-O-Bzl)-D-Trp-Lys-Cys)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys)-Thr-Tyr-NH₂;
D-4-NO₂-Phe-cyclo(D-Cys-Pal-D-Trp-Lys-Cys)-Thr(Bzl)-NH₂;
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Cys-Pal-D-Trp-Lys-Cys)-Thr(Bzl)-Tyr-NH₂;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bz)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzi)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(O-Cys-Pal-D-Trp-Lys-Cys)-Abu-Tyr-NH₂:
D-Cpa-cyclo(D-Cys-(T)aeg-D-Trp-Lys-Cys)-Thr(Bzl)-Tyr-NH₂;
(C)aeg-c(D-Cys-Pal-D-Trp-Lys-D-Cys)-Thr(Bzl)-Tyr-NH₂;
D-Cpa-c(D-Cys-Pal-D-Trp-Lys-D-Cys)Thr(Bzl)-Tyr-NH₂;
(T)aeg-c(Pen-Pal-D-Trp-Lys-D-Cys)Thr(Bzl)-Tyr-NH₂;
(T)aeg-c(D-Cys-Trp-D-Trp-Lys-D-Cys)Thr(Bzl)-Tyr-NH₂;
(T)aeg-c(D-Cys-Phe-D-Trp-Lys-D-Cys)Thr(Bzl)-Tyr-NH₂;
(T)aeg-c(D-Cys-Pal-D-Trp-Orn-D-Cys)Thr(Bzl)-Tyr-NH₂;
(T)aeg-c(D-Cys-Pal-D-Trp-hLys-D-Cys)Thr(Bzl)-Tyr-NH₂;
(T)aeg-c(D-Cys-Pal-D-Trp-lamp-D-Cys)Thr(Bzl)-Tyr-NH₂;
(T)aeg-c(D-Cys-Pal-D-Trp-Cha(4-am)-D-Cys)Thr(Bzl)-Tyr-NH₂;
(T)aeg-c(O-Cys-Pal-D-Trp-Lys-D-Cys)-Ser(Bzl)-Tyr-NH₂:
(T)aeg-c(D-Cys-Pal-D-Trp-Lys-D-Cys)Thr(Bzl)-D-Tyr-NH₂;
(T)aeg-c(D-Cys-Pal-D-Trp-Lys-D-Cys)Thr(Bzl)-Trp-NH₂;
(T)aeg-c(D-Cys-Pal-D-Trp-Lys-D-Pen)Thr(Bzl)-Tyr-NH₂;
(C)aeg-c(D-Cys-Phe-D-Trp-Lys-D-Cys)Thr(Bzl)-Tyr-NH₂;
Ina-c(D-Cys-Phe-D-Trp-Lys-D-Cys)-Thr(Bzl)-Tyr-NH₂;
Mnf-c(D-Cys-Phe-D-Trp-Lys-D-Cys)-Thr(Bzl)-Tyr-NH₂,
Inp-c(D-Cys-Phe-D-Trp-Lys-D-Cys)-Thr(Bzl)-Tyr-NH₂;
Nua-c(D-Cys-Phe-D-Trp-Lys-D-Cys)-Thr(Bzl)-Tyr-NH₂;
(T)aeg-Pal-c(D-Cys-D-Trp-Lys-D-Cys)Thr(Bzl)-Tyr-NH₂;
(T)aeg-Pal-c(D-Cys-D-Trp-Lys-D-Cys)Tyr(Bzl)-Thr-NH₂;
(C)aeg-Phe-c(D-Cys-D-Trp-Lys-D-Cys)Thr(Bzl)-Tyr-NH₂; ou
(T)aeg-D-Trp-c(D-Cys-Pal-Lys-D-C-ys)Thr(Bzl)-Leu-NH₂;
ou un sel pharmaceutiquement acceptable de celui-ci.

16. Composé selon la revendication 8, où ledit composé est selon la formule
Hca-cyclo(D-Cys-Tyr-D-Trp-Lys-Cys)-Nal-NH₂;
Ac-Nal-cyclo(D-Cys-Tyr-D-Trp-Lys-Cys)-Nal-NH₂;
Ac-D-Phe-cyclo(D-Cys-Tyr-D-Trp-Lys-Cys)-Nal-NH₂;
Ac-D-Nal-cyclo(D-Cys-Tyr-D-Trp-Lys-Cys)-Nal-NH₂;
D-Phe-cyclo(D-Cys-Tyr-D-Trp-Lys-Cys)-Nal-NH₂;
Nal-cyclo(D-Cys-Tyr-D-Trp-Lys-Cys)-Nal-NH₂;
D-Nal-cyclo(D-Cys-Tyr-D-Trp-Lys-Cys)-Nal-NH₂;
D-Phe-cyclo(Cys-Tyr-O-Trp-Lys-Cys)-Thr-NH₂;
D-4-NO₂-Phe-cyclo(D-Cys-Tyr-D-Trp-Lys-Cys)-Nal-NH₂;
Ac-D-4-NO₂-Phe-cyclo(D-Cys-Tyr-D-Trp-Lys-Cys)-Nal-NH₂;
D-4-NO₂-Phe-Pal-cyclo(D-Cys-Phe(4-O-Bzl)-D-Trp-Lys-Cys)-Tyr-NH₂;
D-4-NO₂-Phe-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Tyr-NH₂;
D-4-NO₂-Phe-cyclo(D-Cys-Pal-D-Trp-Lys-Cys)-Thr(Bzl)-NH₂;
D-4-NO₂-Phe-cyclo(D-Cys-Pal-D-Trp-Lys-D-Cys)-Thr(Bzl)-Tyr-NH₂;
D-4-NO₂-Phe-cyclo(D-Cys-Tyr-D-Trp-Lys-Cys)-Thr(Bzl)-Tyr-NH₂;
4-NO₂-Phe-cyclo(D-Cys-Pal-D-Trp-Lys-Cys)-Thr(Bzl)-Tyr-NH₂;
D-Nal-cyclo(D-Cys-Pal-D-Trp-Lys-Cys)-Thr(Bzl)-Tyr-NH₃;
Pro-cyclo(D-Cys-Pal-D-Trp-Lys-Cys)-Thr(Bzl)-Tyr-NH₂;
Cpa-cyclo(D-Cys-Pal-D-Trp-Lys-Cys)-Thr(Bzl)-Nal-NH₂;
Ser(Bzl)-cyclo(D-Cys-Pal-D-Trp-Lys-Cys)-Thr-Tyr-NH₂;
(T)aeg-cyclo(D-Cys-Pal-D-Trp-Lys-Cys)-Thr(Bzl)-Tyr-NH₂;
(C)aeg-cyclo(D-Cys-Pal-D-Trp-Lys-Cys)-Thr(Bzl)-Tyr-NH₂;
Aic-cyclo(D-Cys-Pal-D-Trp-Lys-Cys)-Thr(Bzl)-Tyr-NH₂;
(C(z))aeg-cyclo(D-Cys-Pal-D-Trp-lys-Cys)-Thr(Bzl)-Tyr-NH₂;
(A(z))aeg-cyclo(D-Cys-Pal-D-Trp-Lys-Cys)-Thr(Bzl)-Tyr-NH₂;
(T)aeg-cyclo(D-Cys-Pal-D-Trp-Lys-D-Cys)-Thr(Bzl)-Tyr-NH₂;
(A)aeg-cyclo(D-Cys-Pal-D-Trp-Lys-Cys)-Thr(Bzl)-Tyr-NH₂;
(G)aeg-cyclo(D-Cys-Pal-D-Trp-Lys-Cys)-Thr(Bzl)-Tyr-NH₂;
(T)aeg-cyclo(D-Cys-4-Pal-D-Trp-Lys-Cys)-Thr(Bzl)-Tyr-NH₂;
(T)aeg-cyclo(D-Cys-Tyr-D-Trp-Lys-Cys)-Thr(Bzl)-Tyr-NH₂;
(T)aeg-cyclo(D-Cys-Phe-D-Trp-Lys-Cys)-Thr(Bzl)-Tyr-NH₂;
(T)aeg-cyclo(D-Cys-(T)aeg-D-Trp-Lys-Cys)-Thr(Bzl)-Tyr-NH₂;
(T)aeg-cyclo(D-Cys-Pal-D-Trp-Lys-Cys)-Ser(Bzl)-Tyr-NH₂;
(T)aeg-cyclo(D-Cys-Pal-D-Trp-Lys-Cys)-Phe(4-O-Bzl)-Tyr-NH₂;
(T)aeg-cyclo(D-Cys-Pal-D-Trp-Lys-Cys)-A5c-Tyr-NH₂;
(T)aeg-cyclo(D-Cys-Pal-D-Trp-Lys-Cys)-Abu-Tyr-NH₂;
D-Cpa-cyclo(D-Cys-(T)aeg-D-Trp-Lys-Cys)-Thr(Bzl)-Tyr-NH₂;
(T)aeg-cyclo(D-Cys-Pal-D-Trp-Lys-D-Cys)-Thr(Bzl)-p-Me-Phe-NH₂;
Ac-(T)aeg-cyclo(D-Cys-Pal-D-Trp-Lys-D-Cys)-Thr(Bzl)-Tyr-NH₂;
(T)aeg-cyclo(D-Cys-Pal-D-Trp-Lys-D-Cys)-Nal-NH₂;
D-Cpa-cyclo(D-Cys-Pal-D-Trp-Lys-D-Cys)-Nal-NH₂;
(A)aeg-cyclo(D-Cys-Pal-D-Trp-Lys-D-Cys)-Thr(Bzl)-Tyr-NH₂; (C)aeg-cyclo(D-Cys-Pal-D-Trp-Lys-D-Cys)-Thr(Bzl)-Tyr-NH₂;
(C)aeg-c(D-Cys-Pal-D-Trp-Lys-D-Cys)-Thr(Bzl)-Tyr-NH₂;
D-Cpa-c(D-Cys-Pal-D-Trp-Lys-D-Cys)Thr(Bzl)-Tyr-NH₂;
(T)aeg-c(Pen-Pal-D-Trp-Lys-D-Cys)Thr(Bzl)-Tyr-NH₂;
(T)aeg-c(D-Cys-Trp-D-Trp-Lys-D-Cys)Thr(Bzl)-Tyr-NH₂;
(T)aeg-c(D-Cys-Phe-D-Trp-Lys-D-Cys)Thr(Bzl)-Tyr-NH₂;
(T)aeg-c(D-Cys-Pal-D-Trp-Orn-D-Cys)Thr(Bzl)-Tyr-NH₂;
(T)aeg-c(D-Cys-Pal-D-Trp-hLys-D-Cys)Thr(Bzl)-Tyr-NH₂;
(T)aeg-c(D-Cys-Pal-D-Trp-lamp-D-Cys)Thr(Bzl)-Tyr-NH₂;
(T)aeg-c(D-Cys-Pal-D-Trp-Cha(4-am)-D-Cys)Thr(Bzl)-Tyr-NH₂;
(T)aeg-c(D-Cys-Pal-D-Trp-Lys-D-Cys)-Ser(Bzl)-Tyr-NH₂; (T)aeg-c(D-Cys-Pal-D-Trp-Lys-D-Cys)Thr(Bzl)-D-Tyr-NH₂; (T)aeg-c(D-Cys-Pal-D-Trp-Lys-D-Cys)Thr(Bzl)-Trp-NH₂; (T)aeg-c(D-Cys-Pal-D-Trp-Lys-D-Pen)Thr(Bzl)-Tyr-NH₂; (C)aeg-c(D-Cys-Phe-D-Trp-Lys-D-Cys)Thr(Bzl)-Tyr-NH₂; Ina-c(D-Cys-Phe-D-Trp-Lys-D-Cys)-Thr(Bzl)-Tyr-NH₂; Mnf-c(D-Cys-Phe-D-Trp-Lys-D-Cys)-Thr(Bzl)-Tyr-NH₂; Inp-c(D-Cys-Phe-D-Trp-Lys-D-Cys)-Thr(Bzl)-Tyr-NH₂; Nua-c(D-Cys-Phe-D-Trp-Lys-D-Cys)-Thr(Bzl)-Tyr-NH₂; (T)aeg-Pal-c(D-Cys-D-Trp-Lys-D-Cys)Thr(Bzl)-Tyr-NH₂; (T)aeg-Pal-c(D-Cys-D-Trp-Lys-D-Cys)Tyr(Bzl)-Thr-NH₂; (C)aeg-Phe-c(D-Cys-D-Trp-Lys-D-Cys)Thr(Bzl)-Tyr-NH₂; ou (T)aeg-D-Trp-c(D-Cys-Pal-Lys-D-Cys)Thr(Bzl)-Leu-NH₂;
ou un sel pharmaceutiquement acceptable de celui-ci.

17. Composé selon la revendication 10, où ledit composé est selon la formule
cyclo(Trp-D-Trp-Lys-Phe(4-O-Bzl)-Phe-(T)aeg) ;
cyclo(Trp-D-Trp-Lys-Pal-Phe-(T)aeg) ; ou
cyclo(Phe-Phe-D-Trp-Lys-Thr-(T)aeg) ;
ou un sel pharmaceutiquement acceptable de celui-ci.

18. Utilisation d'un composé selon la revendication 13 ou d'un sel pharmaceutiquement acceptable de celui-ci dans la fabrication d'un médicament destiné au déclenchement d'un effet agoniste du récepteur de la neuromédine B chez un sujet en ayant besoin.

19. Utilisation d'un composé selon la revendication 14 ou d'un sel pharmaceutiquement acceptable de celui-ci dans la fabrication d'un médicament destiné au déclenchement d'un effet agoniste du récepteur de la somatostatine chez un sujet en ayant besoin.

20. Utilisation d'un composé selon la revendication 15 ou d'un sel pharmaceutiquement acceptable de celui-ci dans la fabrication d'un médicament destiné au déclenchement d'un effet agoniste du récepteur neuromédine B chez un sujet en ayant besoin.

21. Utilisation d'un composé selon la revendication 16 ou d'un sel pharmaceutiquement acceptable de celui-ci dans la fabrication d'un médicament destiné au déclenchement d'un effet agoniste du récepteur de la somatostatine chez un sujet en ayant besoin.

22. Utilisation d'un composé selon la revendication 17 ou d'un sel pharmaceutiquement acceptable de celui-ci dans la fabrication d'un médicament destiné au déclenchement d'un effet agoniste du récepteur de la somatostatine chez un sujet en ayant besoin, à condition que ledit composé ne soit pas
Cyc1o(Trp-D-Trp-Lys-Phe(4-O-Bzl)-Phe-(T)aeg) ; ou
Cyclo(Trp-D-Trp-Lys-Pal-Phe-(T)aeg).

23. Utilisation d'un composé selon la revendication 14 ou d'un sel pharmaceutiquement acceptable de celui-ci dans la fabrication d'un médicament destiné au déclenchement d'un effet agoniste de SSTR-1 chez un sujet en ayant besoin, à condition que ledit composé ne soit pas
Nal-Tyr-cyclo(Cys-D-Trp-Lys-D-Cys)-Val-Nal-NH₂ ;
Nal-Tyr-cyclo(Cys-D-Trp-Lys-D-Cys)-Abu-Nal-NH₂ ;
Dip-Tyr-cyclo(D-Cys-D-Trp-Lys-D-Cys)-Abu-Nal-NH₂ ;
Nal-Tyr-cyclo(D-Cys-D-Trp-Lys-D-Cys)-Abu-Nal-NH₂ ;
Dip-Tyr-cyclo(D-Cys-D-Trp-Lys-D-Cys)-Val-Nal-NH₂ ;
Nal-Tyr-cyclo(D-Cys-D-Trp-Lys-D-Cys)-Val-Nal-NH₂ ;
Cpa-Pal-cyclo(D-Cys-D-Trp-Lys-D-Cys)-A3c-Nal-NH₂ ;
Cpa-Pal-cyclo(D-Cys-D-Trp-Lys-D-Cys)-A5c-Nal-NH₂ ;
Cpa-Pal-cyclo(D-Cys-D-Trp-Lys-D-Cys)-A6c-Nal-NH₂ ;
(G(z))aeg-cyclo(D-Cys-D-Trp-Lys-D-Cys)-A5c-Nal-NH₂ ;
D-Cpa-cyclo(Cys-D-Trp-Lys-D-Cys)-A5c-Nal-NH₂ ;
Pal-cyclo(D-Cys-D-Trp-Lys-D-Cys)-A5c-Nal-NH₂ ;
Cpa-cyclo(D-Cys-D-Trp-Lys-D-Cys)-A5c-Nal-NH₂ ;
Cpa-Pal-cyclo(D-Cys-D-Trp-Lys-D-Cys)-β-Ala-Nal-NH₂ ;
cyclo(D-Cys-Lys-D-Trp-D-Cys)-A5c-Nal-NH₂ ;
Cpa-Pal-cyclo(D-Cys-D-Trp-Lys-D-Cys)-Sar-Nal-NH₂ ;
Cpa-Pal-cyclo(D-Cys-D-Trp-Lys-D-Cys)-Aic-Nal-NH₂ ;
Cpa-Pal-cyclo(D-Cys-D-Trp-Lys-D-Cys)-Gaba-Nal-NH₂ ; ou
Cpa-Pal-cyclo(D-Cys-D-Trp-Lys-D-Cys)-Pro-Nal-NH₂.

24. Utilisation d'un composé selon la revendication 16 ou d'un sel pharmaceutiquement acceptable de celui-ci dans la fabrication d'un médicament destiné au déclenchement d'un effet agoniste de SSTR-1 chez un sujet en ayant besoin, à condition que ledit composé ne soit pas
Ac-D-Phe-cyclo(D-Cys-Tyr-D-Trp-Lys-Cys)-Nal-NH₂ ;
Ac-D-Nal-cyc1o(D-Cys-Tyr-D-Trp-Lys-Cys)-Nal-NH₂;
D-Phe-cyclo(D-Cys-Tyr-D-Trp-Lys-Cys)-Nal-NH₂ ;
Nal-cyclo(D-Cys-Tyr-D-Trp-Lys-Cys)-Nal-NH₂ ;
D-Nal-cyclo(D-Cys-Tyr-D-Trp-Lys-Cys)-Nal-NH₂ ;
D-4-NO₂-Phe-cyclo(D-Cys-Pal-D-Trp-Lys-Cys)-Thr(Bzl)-Tyr-NH₂ ;
Cpa-cyclo(D-Cys-Pal-D-Trp-Lys-Cys)-Thr(Bzl)-Tyr-NH₂ ;
D-4-NO₂-Phe-cyclo(D-Cys-Pal-D-Trp-Lys-Cys)-Thr(Bzl)-NH₂ ;
D-4-NO₂-Phe-cyclo(D-Cys-Pal-D-Trp-Lys-D-Cys)-Thr(Bzl)-Tyr-NH₂ ;
D-4-NO₂-Phe-cyclo(D-Cys-Tyr-D-Trp-Lys-Cys)-Thr(Bzl)-Tyr-NH₂ ;
4-NO₂-Phe-cyclo(D-Cys-Pal-D-Trp-Lys-Cys)-Thr(Bzl)-Tyr-NH₂ ;
D-Nal-cyclo(D-Cys-Pal-D-Trp-Lys-Cys)-Thr(Bzl)-Tyr-NH₂ ;
Pro-cyclo(D-Cys-Pal-D-Trp-Lys-Cys)-Thr(Bzl)-Tyr-NH₂ ;
Cpa-cyclo(D-Cys-Pal-D-Trp-Lys-Cys)-Thr(Bzl)-Nal-NH₂ ;
Ser(Bzl)-cyclo(D-Cys-Pal-D-Trp-Lys-Cys)-Thr-Tyr-NH₂ ;
(T)aeg-cyclo(D-Cys-Pal-D-Trp-Lys-Cys)-Thr(Bzl)-Tyr-NH₂ ;
(C)aeg-cyclo(D-Cys-Pal-D-Trp-Lys-Cys)-Thr(Bzl)-Tyr-NH₂ ;
Aic-cyclo(D-Cys-Pal-D-Trp-Lys-Cys)-Thr(Bzl)-Tyr-NH₂ ;
(T)aeg-cyclo(D-Cys-Pal-D-Trp-Lys-D-Cys)-Thr(Bzl)-Tyr-NH₂ ;
(A)aeg-cyclo(D-Cys-Pal-D-Trp-Lys-Cys)-Thr(Bzl)-Tyr-NH₂ ;
(G)aeg-cyclo(D-Cys-Pal-D-Trp-Lys-Cys)-Thr(Bzl)-Tyr-NH₂ ;
(T)aeg-cyclo(D-Cys-4-Pal-D-Trp-Lys-Cys)-Thr(Bzl)-Tyr-NH₂ ;
(T)aeg-cyclo(D-Cys-Tyr-D-Trp-Lys-Cys)-Thr(Bzl)-Tyr-NH₂ ;
(T)aeg-cyclo(D-Cys-Phe-D-Trp-Lys-Cys)-Thr(Bzl)-Tyr-NH₂ ;
(T)aeg-cyclo(D-Cys-(T)aeg-D-Trp-Lys-Cys)-Thr(Bzl)-Tyr-NH₂ ;
(T)aeg-cyclo(D-Cys-Pal-D-Trp-Lys-Cys)-Ser(Bzl)-Tyr-NH₂ ;
(T)aeg-cyclo(D-Cys-Pal-D-Trp-Lys-Cys)-Phe(4-O-Bzl)-Tyr-NH₂ ;
(T)aeg-cyclo(D-Cys-Pal-D-Trp-Lys-Cys)-A5c-Tyr-NH₂ ;
(T)aeg-cyclo(D-Cys-Pal-D-Trp-Lys-Cys)-Abu-Tyr-NH₂ ; ou
D-Cpa-cyclo(D-Cys-(T)aeg-D-Trp-Lys-Cys)-Thr(Bzl)-Tyr-NH₂.

25. Composition pharmaceutique comprenant une quantité efficace d'un composé selon la revendication 1 ou d'un sel pharmaceutiquement acceptable de celui-ci et un support pharmaceutiquement acceptable.

26. Utilisation d'un composé selon la revendication 1 dans la fabrication d'un médicament destiné au traitement d'une maladie choisie parmi un cancer du poumon, un gliome, l'anorexie, l'hyperthyroïdie, l'hyperaldostéronisme, la prolifération de *H. pylori*, l'acromégalie, une resténose, la maladie de Crohn, la sclérose systémique, des pseudokystes pancréatiques externes et internes et l'ascite, un VIPome, la nésidioblastose, l'hyperinsulinisme, un gastrinome, le syndrome de Zollinger-Ellison, la diarrhée, la diarrhée associée au SIDA, la diarrhée associée à une chimiothérapie, une sclérodermie, le syndrome de l'intestin irritable, une pancréatite, une obstruction de l'intestin grêle, un reflux gastro-oesophagien, un reflux duodéno-gastrique, le syndrome de Cushing, un gonadotropinome, l'hyperparathyroïdie, la maladie de Basedow, une neuropathie diabétique, la maladie de Paget, la polykystose ovarienne, un cancer de la thyroïde, un hépatome, une leucémie, un méningiome, la cachexie due à un cancer, l'hypotension orthostatique, l'hypotension postprandiale, les crises de panique, les adénomes sécrétant de la GH, l'acromégalie, les adénomes sécrétant de la TSH, les adénomes sécrétant de la prolactine, un insulinome, un glucagonome, le diabète sucré, une hyperlipidémie, une insensibilité à l'insuline, le syndrome X, une angiopathie, une rétinopathie proliférative, l'hyperglycémie matinale, une néphropathie, la sécrétion d'acide gastrique, les ulcères peptiques, les fistules entérocutanées, les fistules pancréatico-cutanées, le syndrome de chasse, le syndrome des diarrhées liquides, une pancréatite, une tumeur sécrétant de l'hormone gastro-intestinale, une angiogenèse, une arthrite, le rejet d'allogreffe, les saignements de vaisseaux greffés, l'hypertension portale, les saignements gastro-intestinaux, l'obésité et un surdosage d'opioïde.
